# EUROPEAN PATENT APPLICATION

(11) **EP 4 206 183 A1**
(43) Date of publication of application: **05.07.2023**
(21) Application number: 21306963.6
(22) Date of filing: 31.12.2021
(51) Int. Cl.: C07C 235/20, C07D 335/16, C07D 295/108, C07F 9/00, C08F 265/00

(54) **POLYHYDROXYLATED PHOTOINITIATORS**

(71) Applicant: ARKEMA FRANCE, 92700 Colombes (FR)
(72) Inventor: JEPSON, David, WETHERBY, LS22 7NS (GB); SQUIRES, Kelly, WETHERBY, LS22 7NS (GB); SEHNAL, Petr, WETHERBY, LS22 7NS (GB); PLENDERLEITH, Richard, WETHERBY, LS22 7NS (GB); DEMOULIN, Kevin, 60550 VERNEUIL EN HALATTE (FR); CICERON, Philippe, 60550 VERNEUIL EN HALATTE (FR); MELEC, Pierre, 60550 VERNEUIL EN HALATTE (FR)
(74) Representative: Arkema Patent

(57) **Abstract**

The present invention relates to novel photoinitiators having hydroxyl groups and/or (meth)acrylate groups. The invention also relates to compositions comprising these photoinitiators as well as their use for photopolymerizing ethylenically unsaturated compounds.

## Description

### Field of the Invention

The present invention relates to novel photoinitiators which have hydroxyl groups and/or (meth)acrylate groups. The invention also relates to compositions comprising these photoinitiators as well as their use for photopolymerizing ethylenically unsaturated compounds, in particular in water-based curable compositions.

### Background of the Invention

Radiation curable compositions containing ethylenically unsaturated compounds can be polymerised by exposure to radiation, such as ultraviolet (UV) light. For rapid and effective curing, a photoinitiator is often used. The photoinitiator forms radical species upon irradiation with photons and initiates free-radical polymerisation of unsaturated groups, leading to hardening (curing) of the material.

Free radical photoinitiators can adopt two different modes of action, and are classified by mode of action as Norrish Type I and Norrish Type II Photoinitiators. Norrish Type I photoinitiators cleave upon exposure to radiation, producing radical species which are capable of initiating the polymerisation of unsaturated compounds. Norrish Type II photoinitiators are compounds which do not fragment upon exposure to radiation and so will not typically initiate radical-chain polymerisation unless a co-initiator such as an amine synergist is present. Upon exposure to radiation, interaction between the Type II photoinitiator and the co-initiator leads to the generation of radical species which can initiate the polymerisation of UV-curable resins.

The most common Type-II photoinitiators are diaryl ketones such as benzophenones (e.g. SpeedCure^{®} BP available from Arkema) or thioxanthones such as 2-isopropylthioxanthone (SpeedCure^{®} 2-ITX available from Arkema) or diethylthioxanthone (SpeedCure^{®} DETX available from Arkema).

One well recognized problem with the use of UV curable systems for coating and adhesive applications is the fate of the photo-by products created by the curing process. In the case of typical α-cleavage type photoinitiators, the production of benzaldehyde (and often related compounds) is often a significant concern from both a toxicity and product odor standpoint. Such concerns become especially important when the use of radiation curable materials is considered for applications that involve skin or food contact. Various effective approaches have been taken to reduce the odor and extractable by-product content of UV curable materials. One approach has been the use of co-polymerizable or polymeric photoinitiators which are chemically incorporated into the cured polymeric matrix as opposed to remaining in the irradiated material as a small molecule (see (a) Fouassier, J. P.; Rabek, J. F., Eds. Radiation Curing in Science and Technology, 1993, Elsevier Appl. Sci., vol. 2, 283-321. (b) Fouassier, J. P. Photoinitiation, Photopolymerization and Photocuring, Fundamentals and Applications, 1995, Hanser Publishers, 71-73.).

The use of polymerizable or polymeric H-abstraction type photoinitiators, in principle, presents the possibility of creating a system with zero extractable components related to the photoinitiation system. Various groups have presented systems based upon poly(vinyl benzophenone) and its copolymers or polymers derived from acrylated benzophenone derivatives (see (a) David, C.; Demarteu, W.; Geuskens, G. Polymer, 1969, 10, 21-27. (b) Carlini, C.; Ciardelli, F.; Donati, D.; Gurzoni, F. Polymer, 1983, 24, 599-606.). The direct use of acrylated benzophenones has also been disclosed (U.S. Pat. No. 3,429,852). Unfortunately, these Type II systems often suffer from issues related to photoefficiency relative to analogous small molecule photoinitation systems. Thus, it is often most desirable to use polymeric photoinitiators as opposed to polymerizable photoinitiators.

The increasing demand for water-based polymerizable systems has triggered the development of water-soluble or water-dispersible photoinitiators. The leading product on the market, SpeedCure 2959, has a limited water solubility of 0.5 wt%, which often falls below the required formulation amount. Quaternary or tertiary salts of photoinitiators displaying good water-solubility have been tested, but the resulting coating has an increased hydrophilic nature (hygroscopic) leading to swelling. The salts slow radical polymerisation, lead to yellowing of the coating and the higher cost of these materials is unattractive to the industry.

Accordingly, there continues to be a need in the art for improved H-abstraction photoinitiators useful in the manufacture of radiation curable adhesives and coating formulations that do not suffer from the above-identified drawbacks and still produce low odor products with fewer (or no) inherent extractable photochemical by-products. The present development fulfils this need.

The photoinitiators of the present invention have multiple hydroxyl groups which advantageously enhances their solubility in water-based system and polar solvents. The increased water solubility enables increased photoinitiator concentrations, higher cure speeds and much better ease of formulation. Further, the hydroxyl groups provide suitable functionalization points as they can be easily (meth)acrylated by esterification with (meth)acrylic acid or by transesterification with a (C1-C6)alkyl (meth)acrylate, hence reducing the migration potential of the resulting polymerizable photoinitiator.

The curable compositions comprising the photoinitiators of the invention may present one or more of the following advantages:
- usable in sensitive applications (food and beverage packaging, food and beverage processing, pharmaceutical packaging, nail polish, dental products, textiles in contact with a human body, medical devices, water pipes) due to their low migration properties,
- low viscosity,
- high UV reactivity,
- low yellowing,
- weak odor or no odor,
- good mechanical properties after curing (e.g. hardness, scratch resistance, solvent resistance).

### Summary of the Invention

A first aspect of the invention is a compound according to the following formula (I): 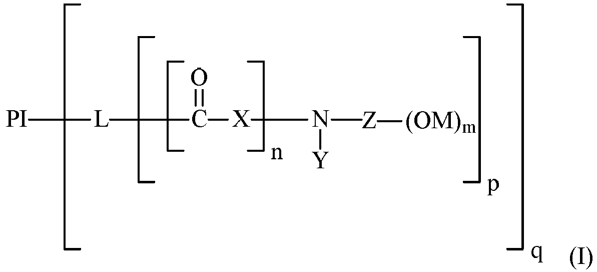 wherein PI, L, M, X, Y, Z, m, n, p, and q are as defined herein.

Another aspect of the invention is a photoinitiator composition comprising at least one compound of formula (I) according to the invention and at least one compound according to one of the following formula (XXXVII) or (XXXVIII) 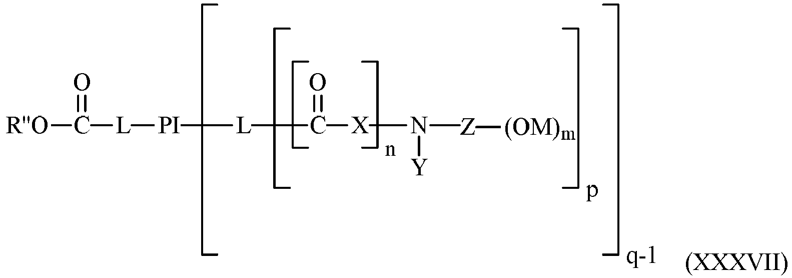 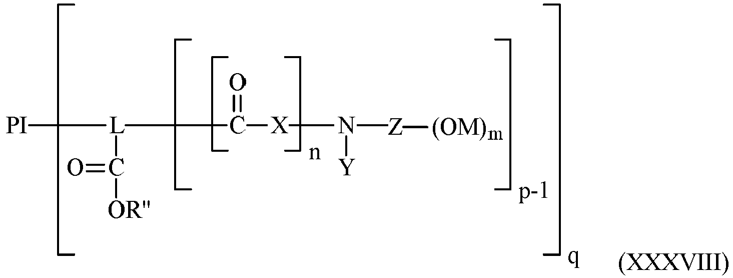 wherein PI, L, M, X, Y, Z, R", m, n, p and q are as defined herein.

Another aspect of the invention is a photoinitiator composition comprising a mixture of at least two distinct compounds of formula (I) according to the invention.

Another aspect of the invention is a photoinitiator composition comprising a compound of formula (I) according to the invention and an amine synergist other than a compound of formula (I), preferably an amine synergist bearing a tertiary amine group.

The invention also aims to provide a process for preparing a compound of formula (I) according to the invention, wherein at least part of the M groups are (meth)acryloyl, the process comprising reacting a compound of formula (I) wherein all of the M groups are H with a (meth)acrylic acid or a (C1-C6)alkyl (meth)acrylate in the presence of a catalyst.

The invention also aims to provide a process for photopolymerizing one or more ethylenically unsaturated compounds, the process comprising contacting one or more ethylenically unsaturated compounds with a compound of formula (I) according to the invention or a photoinitiator composition according to the invention, and irradiating the mixture, in particular with visible, near-UV and/or UV light.

Another aspect of the invention is a curable composition comprising:
a) a compound of formula (I) according to the invention or a photoinitiator composition according to the invention; and
b) an ethylenically unsaturated compound.

Another aspect of the invention is a curable composition comprising:
a) a compound of formula (I) according to the invention or a photoinitiator composition according to the invention; and
c) an unsaturated polymer dispersion or emulsion.

Another aspect of the invention is a process for the preparation of a cured product, comprising curing the curable composition according to the invention, in particular by exposing the curable composition to radiation such as UV, near-UV and/or visible radiation.

Another aspect of the invention is a process of inkjet printing comprising jetting the curable composition according to the invention onto a substrate.

Another aspect of the invention is substrate on which the curable composition according to the invention has been applied and cured, in particular the substrate is a food and beverage packaging, a pharmaceutical packaging, a textile, a nail, a tooth, a medical device, a food and beverage processing equipment, a water pipe.

Another aspect of the invention is a use of a compound of formula (I) according to the invention or a photoinitiator composition according to the invention, in a photopolymerization reaction.

Another aspect of the invention is a use of a compound of formula (I) according to the invention or a photoinitiator composition according to the invention, as a photoinitiator or a photoinitiating system, in particular as a photoinitiator or a photoinitiating system in a radiation curable composition, in particular in a UV or LED-curable composition.

Another aspect of the invention is a use of a compound of formula (I) according to the invention wherein at least part of the M groups are (meth)acryloyl, to reduce the amount of extractibles from a cured layer of a curable composition.

### Detailed Description

### Definitions

In the present application, the term "comprise(s) a/an" means "comprise(s) one or more".

Unless mentioned otherwise, the % by weight in a compound or a composition are expressed based on the weight of the compound, respectively of the composition.

The term « (meth)acryloyloxy » means a group of formula -O-C(=O)-CH(R)=CH₂ wherein R is H or Me. The term « (meth)acryloyloxy » encompasses both an acryloyloxy group wherein R is H and a methacryloyloxy group wherein R is Me.

The term « (meth)acryloyl » means a group of formula -C(=O)-CH(R)=CH₂ wherein R is H or Me. The term « (meth)acryloyl » encompasses both an acryloyl group wherein R is H and a methacryloyl group wherein R is Me.

The term "photoreactive compound, group or linker" means a compound, group or linker that participates in, and/or initiates a photochemical reaction upon irradiation with electromagnetic radiation. A photoreactive compound, group or linker typically comprises a photoinitiator moiety and optionally one or more synergist moieties.

The term "photoinitiator moiety" means a moiety comprising a group capable of absorbing light and generating a reactive species useful to initiate a polymerisation reaction.

The term "synergist moiety" means a moiety comprising a hydrogen-donor group capable of reacting with an activated photoinitiator moiety by hydrogen abstraction to generate a free-radical species.

The term « aryl » means an optionally substituted polyunsaturated aromatic group. The aryl may contain a single ring (i.e. phenyl) or more than one ring wherein at least one ring is aromatic. When the aryl comprises more than one ring, the rings may be fused, linked via a covalent bond (for example biphenyl). The aromatic ring may optionally comprise one to two additional fused rings (i.e. cycloalkyl, heterocycloalkyl or heteroaryl). The term "aryl" also encompasses partially hydrogenated derivatives of the carbocyclic system is described above. Examples include phenyl, naphtyl, biphenyl, phenanthrenyl and naphthacenyl.

The term « alkyl » means a monovalent saturated acyclic hydrocarbon group of formula - CₙH₂ₙ₊₁ wherein n is 1 to 20. An alkyl may be linear or branched. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term « heteroatom-containing alkyl » means an alkyl interrupted by one or more heteroatoms independently selected from O, N or S.

The term « halogen » means an atom selected from Cl, Br, F and I.

The term « cycloalkyl » means a monovalent saturated alicyclic hydrocarbon group comprising a cycle. Examples of cycloalkyl groups include cyclopentyl, cyclohexyl and isobornyl.

The term « heterocycloalkyl » means a cycloalkyl having at least one ring atom that is a heteroatom selected from O, N or S.

The term « alkoxy » means a group of formula -O-alkyl, wherein the alkyl is as defined above.

The term « aryloxy » means a group of formula -O-aryl, wherein the aryl is as defined above.

The term « thioalkyl » means a group of formula -S-alkyl, wherein the alkyl is as defined above.

The term « thioaryl » means a group of formula -S-aryl, wherein the aryl is as defined above.

The term « alkenyl » means a monovalent alicyclic hydrocarbon group comprising at least one C=C double bond. An alkenyl may be linear or branched.

The term « alkynyl » means a monovalent alicyclic hydrocarbon group comprising at least one C=C triple bond. An alkynyl may be linear or branched.

The term « aralkyl » means an aryl substituted by an alkyl group. An example of an aralkyl group is tolyl.

The term « alkaryl » means an alkyl substituted by an aryl group. An example of an alkaryl group is benzyl (-CH₂-Phenyl).

The term « aralkyloxy » means a group of formula -O-aralkyl, wherein the aralkyl is as defined above.

The term « alkaryloxy » means a group of formula -O-alkaryloxy, wherein the alkaryl is as defined above.

The term « heteroaryl » means an aryl having at least one ring atom that is a heteroatom selected from O, N or S.

The term « alkylamino » means an alkyl substituted by at least one amino group.

The term « alkylthiol » means an alkyl substituted by at least one thiol group.

The term « hydroxyalkyl » means an alkyl substituted by at least one hydroxy group.

The term « haloalkyl » means an alkyl substituted by at least one halogen.

The term « alkylene » or « alkanediyl » means a linker derived from an alkane of formula CₘH₂ₘ₊₂ by removing one hydrogen atom at each point of attachment of the linker. An alkylene may be divalent, trivalent, tetravalent or have even higher valencies.

The term "alkoxylated" means a compound, group or linker containing one or more oxyalkylene moieties, in particular one or more oxyalkylene selected from oxyethylene (-O-CH₂-CH₂-), oxypropylene (-O-CH₂-CH(CH₃)- or -O-CH(CH₃)-CH₂-), oxybutylene (-O-CH₂-CH₂-CH₂-CH₂-) and mixtures thereof. For example, an alkoxylated compound, group or linker may contain from 1 to 30 oxyalkylene moieties.

The term « linker » means a plurivalent group. A linker may connect at least two moieties of a compound together, in particular 2 to 16 moieties of a compound together. For example, a linker that connects two moieties of a compound together is referred to as a divalent linker, a linker that connects three moieties of a compound together is referred to as a trivalent linker, etc....

The term « aliphatic compound, group or linker » means a non-aromatic acyclic compound, group or linker. It may be linear or branched, saturated or unsaturated. It may be substituted by one or more groups, for example selected from alkyl, hydroxyl, halogen (Br, Cl, I), isocyanate, carbonyl, amine, carboxylic acid, -C(=O)-OR', -C(=O)-O-C(=O)-R', each R' being independently a C1-C6 alkyl. It may comprise one or more bonds selected from ether, ester, amide, urethane, urea and mixtures thereof.

The term « acyclic compound, group or linker » means a compound, group or linker that does not comprise any rings

The term « cycloaliphatic compound, group or linker » means a non-aromatic cyclic compound, group or linker. It may be substituted by one or more groups as defined for the term « aliphatic ». It may comprise one or more bonds as defined for the term « aliphatic ».

The term « aromatic compound, group or linker » means a compound, group or linker comprising an aromatic ring, which means that respects Hückel's aromaticity rule, in particular a compound comprising a phenyl group. It may be substituted by one or more groups as defined for the term « aliphatic ». It may comprise one or more bonds as defined for the term « aliphatic ».

The term « saturated compound, group or linker » means a compound, group or linker that does not comprise any double or triple carbon-carbon bonds.

The term « unsaturated compound, group or linker » means a compound, group or linker that comprises a double or triple carbon-carbon bond, in particular a double carbon-carbon bond.

The term « polyol » means a compound comprising at least two hydroxyl groups.

The term « polyether polyol » or « polyether linker » means a polyol, respectively a linker, comprising at least two ether bonds.

The term « polyester polyol » or « polyester linker » means a polyol, respectively a linker, comprising at least two ester bonds.

The term « polycarbonate polyol » or « polycarbonate linker » means a polyol, respectively a linker, comprising at least two carbonate bonds.

The term « polyurethane linker » means a linker comprising at least two urethane bonds.

The term « polyorganosiloxane polyol » or « polyorganosiloxane linker » means a polyol, respectively a linker, comprising at least two organosiloxane bonds. The organosiloxane may, for example be a dimethylsiloxane bond.

The term « polycaprolactone polyol » or « polycaprolactone linker » means a polyol, respectively a linker, comprising at least two units derived from the ring-opening polymerization of ε-caprolactone, in particular at least two -[(CH₂)₅-C(=O)O]- units.

The term « polybutadiene polyol » or « polybutadiene linker » means a polyol, respectively a linker, comprising at least two units derived from the polymerization of butadiene, in particular at least two units selected from -CH₂-CH=CH-CH₂- and CH₂-CH(CH=CH₂)-.

The term « amino group » means a -NRₐ₁R_{b1} group, wherein Rₐ₁ and R_{b1} are independently H or an optionally substituted alkyl. A « primary amino group » means a -NRₐ₁R_{b1} group, wherein Rₐ₁ and R_{b1} are H. A « secondary amino group » means a -NRₐ₁R_{b1} group, wherein Rₐ₁ is H and R_{b1} is an optionally substituted alkyl.

The term « carboxylic acid » means a -COOH group.

The term « isocyanate group » means a -N=C=O group.

The term « ester bond » means a -C(=O)-O- or -O-C(=O)- bond.

The term « ether bond » means a -O- bond.

The term « organosiloxane bond » means a -Si(R_{c1})₂-O- bond, wherein R_{c1} is an organic group, in particular an organic group selected from alkyl, alkoxy and aryl. The term « dimethylsiloxane bond » means a -Si(CH₃)₂-O- bond

The term « carbonate bond » means a -O-C(=O)-O- bond.

The term « urethane or carbamate bond » means a -NH-C(=O)-O- or -O-C(=O)-NH- bond.

The term « polyisocyanate » means a compound comprising at least two isocyanate groups.

The term « optionally substituted compound, group or linker » means a compound, group or linker optionally substituted by one or more groups selected from halogen, alkyl, cycloalkyl, heterocycloalkyl, aryl, heteroaryl, alkoxy, aryloxy, aralkyl, alkaryl, aralkyloxy, alkaryloxy, haloalkyl, -OH, -SH, hydroxyalkyl, thioalkyl, thioaryl, alkylthiol, amino, alkylamino, isocyanate, nitrile, oxo (=O), -C(=O)-R', -O-C(=O)-R', -C(=O)-OR', -C(=O)-N(R')₂, -NR'-C(=O)-R', -C(=O)-O-C(=O)-R' and -SO₂-N(R')₂, each R' being independently H or an optionally substituted group selected from alkyl, aryl and alkylaryl.

### Compound of formula (I)

The compound of the invention corresponds to the following formula (I): 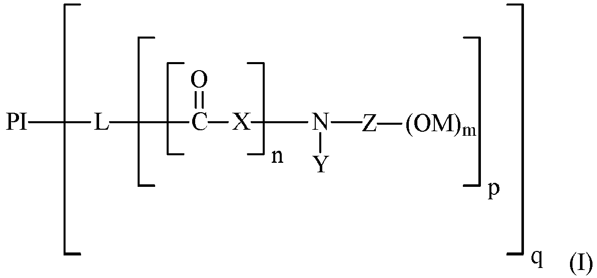
PI is a photoinitiator moiety;
each L is independently selected from a direct bond or a linker comprising 1 to 20 carbon atoms;
each X is independently selected from a direct bond, a -P(=O)(R')- bond and a linker comprising 1 to 6, in particular 1 to 4, more particularly 1 or 2, carbon atoms;
R' is aryl, alkyl or alkoxy;
each Y is independently H, alkyl or -Z'-(OM')_{m'};
each Z and Z' is independently a linker comprising 1 to 6, in particular 2 to 5, more particularly 2 to 4, carbon atoms;
each M and M' is independently H or (meth)acryloyl;
each m and m' is independently 1, 2 or 3, in particular 1;
each n is independently 0 or 1, in particular 1;
each p is independently 1, 2 or 3; in particular 1 or 2;
q is 1, 2 or 3, in particular 1 or 2.

The -L-[C(=O)-X]ₙ- moieties and the -Z- moieties may be devoid of ester bonds. Indeed, the presence of ester bonds in said moieties would not be compatible with a transesterification reaction to functionalize the compounds of formula (I) with (meth)acrylate groups.

The compound of formula (I) bears a photoinitiator moiety PI. In one embodiment, the compound of formula (I) bears a single photoinitiator moiety PI.

PI may be a photoinitiator moiety comprising an optionally substituted aryl selected from benzophenone, thioxanthone, anthraquinone, xanthone, acridone and phenyl.

An optionally substituted phenyl should be understood as being distinct from an aryl comprising a benzophenone, thioxanthone, anthraquinone, xanthone or acridone moiety. Accordingly, when PI comprises an optionally substituted phenyl, the phenyl and its optional substituents do not form or are not part of a benzophenone, thioxanthone, anthraquinone, xanthone or acridone moiety. When PI comprises an optionally substituted phenyl, at least one -C(=O)- group is directly bonded to said phenyl. Said at least one -C(=O)- group may be present as a substituent on said phenyl group and/or it may be included in at least one of the -L-[[C(=O)-X]ₙ-NY-Z-(OM)ₘ]ₚ arms borne by the phenyl group (i.e. at least one of said arms has L = a direct bond and n = 1).

In particular, PI corresponds to one of the following formulae (II) to (IX) 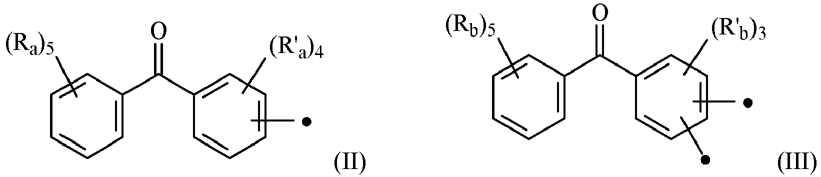 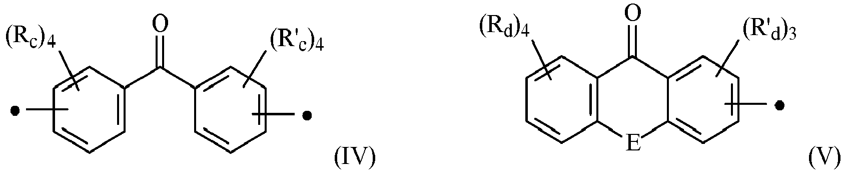 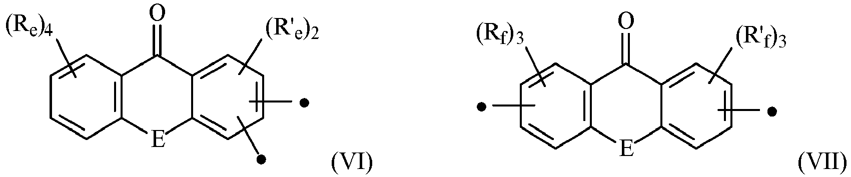 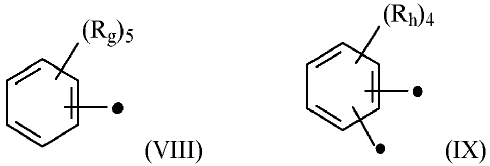 wherein
E is S, O, C(=O) or NR₁, in particular S;
R₁ is H, an optionally substituted alkyl, an optionally substituted aryl;
each symbol ● represents a point of attachment to a linker L;
each Rₐ, R'ₐ, R_{b}, R'_{b}, R_{c}, R'_{c}, R_{d}, R'_{d}, Re, R'ₑ, R_{f}, R'_{f}, R_{g} and Rₕ is independently selected from H, F, Cl, Br, I, -OR₂, -SR₂, -N(R₂)₂, -NO2, -CN, -C(=O)R₂, -O-C(=O)R₂, -C(=O)OR₂, -C(=O)N(R₂)₂, -NR₂-C(=O)-R₂, -SO₂-N(R₂)₂, -C(=O)-P(=O)R₃R₄, or an optionally substituted group selected from alkyl, heteroatom-containing alkyl, cycloalkyl, heterocycloalkyl, alkenyl, alkynyl, aryl, aralkyl, alkaryl and heteroaryl;
two adjacent Ra, R'a, Rb, R'b, R_{c}, R'_{c}, R_{d}, R'_{d}, Rₑ, R'ₑ, R_{f}, R'_{f}, R_{g} or Rₕ groups may form, with the carbon atoms to which they are attached, a 5 to 8 membered ring;
each R₂ is independently H or an optionally substituted group selected from alkyl, cycloalkyl, heterocycloalkyl, alkenyl, alkynyl, aryl, aralkyl, alkaryl, heteroaryl and heteroatom-containing alkyl;
R₃ and R₄ are independently an optionally substituted group selected from aryl, alkyl, alkoxy or amino.

PI may be a photoinitiator moiety comprising an optionally substituted benzophenone. In particular, PI may be a benzophenone-containing moiety according to one of formula (II), (III) or (IV) as defined above. More particularly, PI may be a benzophenone-containing moiety according to one of formula (II), (III) or (IV) as defined above wherein Rₐ, R'ₐ, R_{b}, R'_{b}, R_{c} and R'_{c} are independently H, F, Cl, Br, I, -OR₂, -SR₂, -N(R₂)₂, -C(=O)R₂, or an optionally substituted group selected from alkyl, heteroatom-containing alkyl, and aryl, and two adjacent Rₐ, R'ₐ, R_{b}, R'_{b}, R_{c}, or R'_{c} groups may form, with the carbon atoms to which they are attached, a 5 to 8 membered ring; in particular Rₐ, R'ₐ, R_{b}, R'_{b}, R_{c} and R'_{c} are independently H or -C(=O)R₂.

PI may be a photoinitiator moiety comprising an optionally substituted thioxanthone. In particular, PI may be a thioxanthone-containing moiety according to one of formula (V), (VI) or (VII) as defined above wherein X = S. More particularly, PI may be a thioxanthone-containing moiety according to one of formula (V), (VI) or (VII) as defined above wherein X = S and wherein R_{d}, R'_{d}, Rₑ, R'ₑ, R_{f} and R'_{f} are independently H, F, Cl, Br, I, -OR₂, -SR₂, -N(R₂)₂, -C(=O)R₂, or an optionally substituted group selected from alkyl, heteroatom-containing alkyl, and aryl, and two adjacent R_{d}, R'_{d}, Rₑ, R'ₑ, R_{f} or R'_{f} groups may form, with the carbon atoms to which they are attached, a 5 to 8 membered ring; in particular R_{d}, R'_{d}, Rₑ, R'ₑ, R_{f} and R'_{f} are independently H or -C(=O)R₂.

PI may be a photoinitiator moiety comprising an optionally substituted anthraquinone. In particular, PI may be an anthraquinone-containing moiety according to one of formula (V), (VI) or (VII) as defined above wherein X = -C(=O)-. More particularly, PI may be an anthraquinone-containing moiety according to one of formula (V), (VI) or (VII) as defined above wherein X = -C(=O)- and wherein R_{d}, R'_{d}, Rₑ, R'ₑ, R_{f} and R'_{f} are independently H, F, Cl, Br, I, -OR₂, -SR₂, -N(R₂)₂, -C(=O)R₂, or an optionally substituted group selected from alkyl, heteroatom-containing alkyl, and aryl, and two adjacent R_{d}, R'_{d}, Rₑ, R'ₑ, R_{f} or R'_{f} groups may form, with the carbon atoms to which they are attached, a 5 to 8 membered ring; in particular R_{d}, R'_{d}, Rₑ, R'ₑ, R_{f} and R'_{f} are independently H or -C(=O)R₂.

PI may be a photoinitiator moiety comprising an optionally substituted xanthone. In particular, PI may be a xanthone-containing moiety according to one of formula (V), (VI) or (VII) as defined above wherein X = O. More particularly, PI may be a xanthone-containing moiety according to one of formula (V), (VI) or (VII) as defined above wherein X = O and wherein R_{d}, R'_{d}, Rₑ, R'ₑ, R_{f} and R'_{f} are independently H, F, Cl, Br, I, -OR₂, -SR₂, -N(R₂)₂, -C(=O)R₂, or an optionally substituted group selected from alkyl, heteroatom-containing alkyl, and aryl, and two adjacent R_{d}, R'_{d}, Rₑ, R'ₑ, R_{f} or R'_{f} groups may form, with the carbon atoms to which they are attached, a 5 to 8 membered ring; in particular R_{d}, R'_{d}, Rₑ, R'ₑ, R_{f} and R'_{f} are independently H or -C(=O)R₂.

PI may be a photoinitiator moiety comprising an optionally substituted acridone. In particular, PI may be an acridone-containing moiety according to one of formula (V), (VI) or (VII) as defined above wherein X = NRi. More particularly, PI may be an acridone-containing moiety according to one of formula (V), (VI) or (VII) as defined above wherein X = NRi and wherein R_{d}, R'_{d}, Rₑ, R'ₑ, R_{f} and R'_{f} are independently H, F, Cl, Br, I, -OR₂, -SR₂, -N(R₂)₂, -C(=O)R₂, or an optionally substituted group selected from alkyl, heteroatom-containing alkyl, and aryl, and two adjacent R_{d}, R'_{d}, Rₑ, R'ₑ, R_{f} or R'_{f} groups may form, with the carbon atoms to which they are attached, a 5 to 8 membered ring; in particular R_{d}, R'_{d}, Rₑ, R'ₑ, R_{f} and R'_{f} are independently H or -C(=O)R₂.

PI may be a photoinitiator moiety comprising an optionally substituted phenyl. In particular, PI may be a phenyl-containing moiety according to one of formula (VIII) or (IX) as defined above. More particularly, PI may be a phenyl-containing moiety according to one of formula (VIII) or (IX) as defined above wherein R_{g} and Rₕ are independently H, F, Cl, Br, I, -OR₂, -SR₂, -N(R₂)₂, -C(=O)R₂, or an optionally substituted group selected from alkyl, heteroatom-containing alkyl, and aryl, and two adjacent R_{g} or Rₕ groups may form, with the carbon atoms to which they are attached, a 5 to 8 membered ring; in particular R_{g} and Rₕ are independently H, -C(=O)R₂ or alkyl.

When PI corresponds to formula (VIII) or (IX), at least one -C(=O)- group is directly bonded to the phenyl group of formula (VIII) or (IX). Said -C(=O)- may be directly bonded to the phenyl group of formula (VIII) or (IX) at a symbol ● and/or at a substituent R_{g} or Rₕ. In other words, at least one of the -L-[[C(=O)-X]ₙ-NY-Z-(OM)ₘ]ₚ arms borne by the phenyl moiety has L = a direct bond and n = 1 and/or at least one of R_{g} and Rₕ is -C(=O)R₂ wherein R₂ is as defined above.

In particular, PI may correspond to formula (VIII) or (IX) as defined above. Such compounds, may be an acetophenone-based photoinitiator or a phosphine oxide based photoinitiator. More particularly, when PI corresponds to formula (VIII) or (IX) as defined above, the compound of formula (I) may comprise a moiety having the following formula (X) or (XI) directly bonded to the phenyl group of PI: 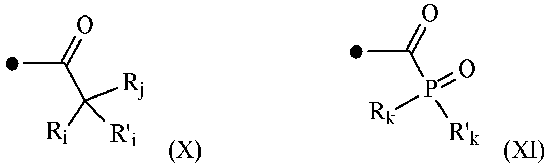 wherein
Rᵢ and R'ᵢ are alkyl or Rᵢ and R'ᵢ may, together with the carbon atom to which they are attached, form a 5 to 8 membered ring
Rⱼ is -OH or -NRₗR'ₗ;
Rₖ and R'ₖ are independently selected from aryl, alkyl, alkoxy and -N(Y)-Z-(OM)ₘ;
Rₗ and R'ₗ are independently selected from alkyl and -Z-(OM)ₘ or Rₗ and R'ₗ may, together with the nitrogen atom to which they are attached, form a 5 to 8 membered ring;
each Y is independently H, alkyl or -Z'-(OM')_{m'};
each Z and Z' is independently a linker comprising 1 to 6, in particular 2 to 5, more particularly 2 to 4, carbon atoms;
each M and M' is independently H or (meth)acryloyl;
each m and m' is independently 1, 2 or 3, in particular 1;
the symbol ● represents a point of attachment to the phenyl group of PI.

The moiety of formula (X) or (XI) as defined above may be directly bonded to the phenyl group of formula (VIII) or (IX) at a symbol ● and/or at a substituent R_{g} or Rₕ.

When a moiety of formula (X) is directly bonded to the phenyl group of formula (VIII) or (IX) at a symbol ●, then said moiety of formula (X) has Rⱼ is -NRₗR'ₗ and at least one of Rₗ and R'ₗ is -Z-(OM)ₘ.

When a moiety of formula (X) is directly bonded to the phenyl group of formula (VIII) or (IX) at a substituent R_{g}, then said moiety of formula (X) has Rⱼ is -OH or -NRₗR'ₗ and Rₗ and R'ₗ are independently selected from alkyl or Rₗ and R'ₗ may, together with the nitrogen atom to which they are attached, form a 5 to 8 membered ring.

When a moiety of formula (XI) is directly bonded to the phenyl group of formula (VIII) or (IX) at a symbol ●, then said moiety of formula (XI) has Rₖ is selected from aryl, alkyl and alkoxy and R'ₖ is -N(Y)-Z-(OM)ₘ.

When a moiety of formula (XI) is directly bonded to the phenyl group of formula (VIII) or (IX) at a substituent Rₕ, then said moiety of formula (XI) has Rₖ and R'ₖ are independently selected from aryl, alkyl, alkoxy.

When PI corresponds to formula (VIII) or (IX) as defined above, at least one of the -L-[[C(=O)-X]ₙ-NY-Z-(OM)ₘ]ₚ arms borne by the phenyl moiety may be a moiety of formula (X) [i.e. L = a direct bond, n = 1 and X = -CRᵢR'ᵢ-] or formula (XI) [i.e. L = a direct bond, n = 1 and X = -P(=O)(Rₖ)-) ; and/or at least one of R_{g} and Rₕ is a moiety of formula (X) or (XI).

In a preferred embodiment, the compound of formula (I) according to the invention may be according to one of formulae (XII) to (XXV): 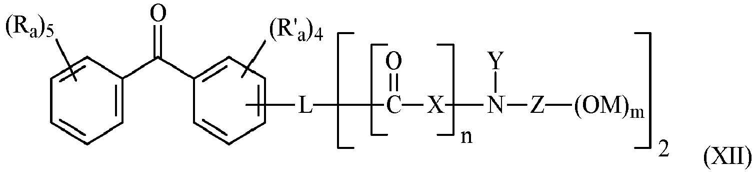 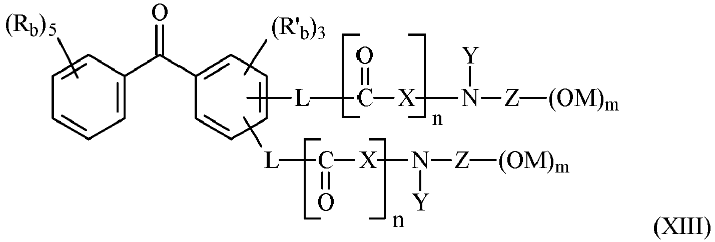 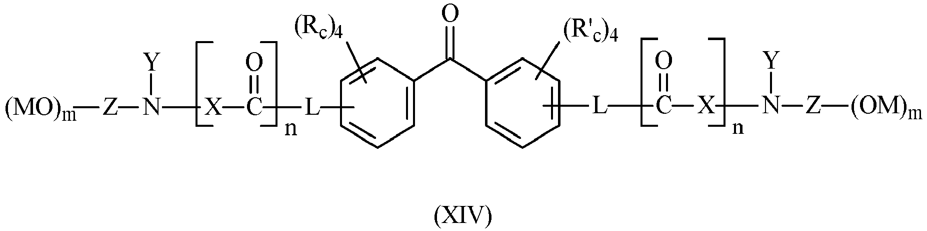 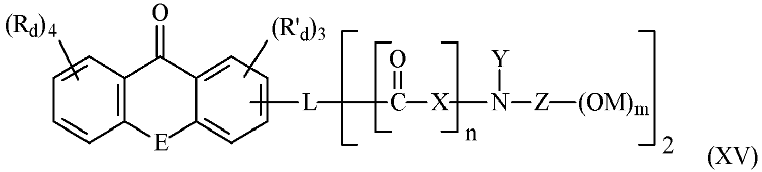 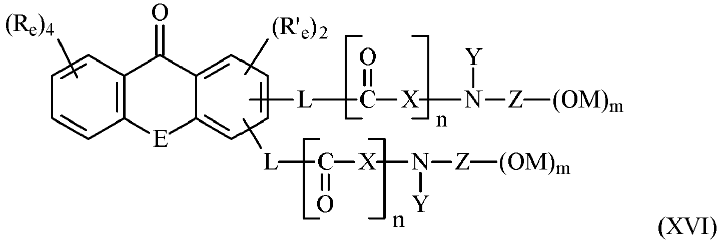 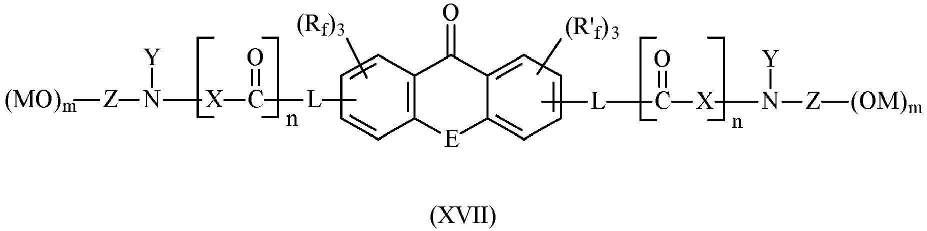 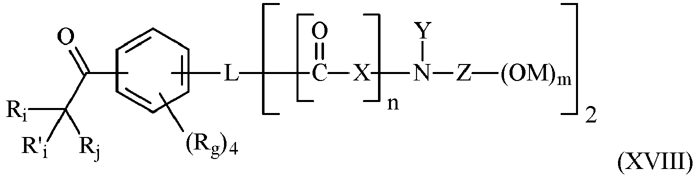 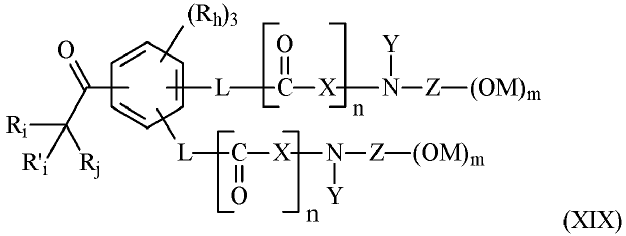 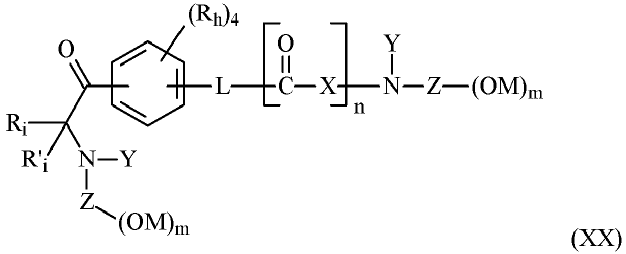 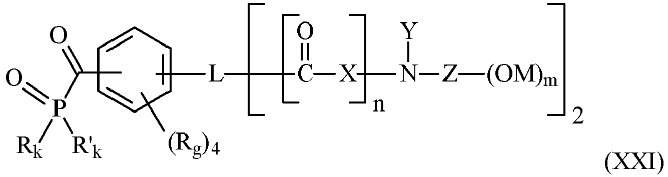 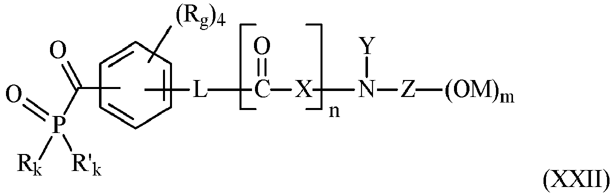 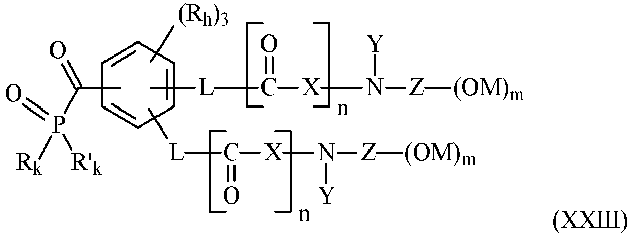 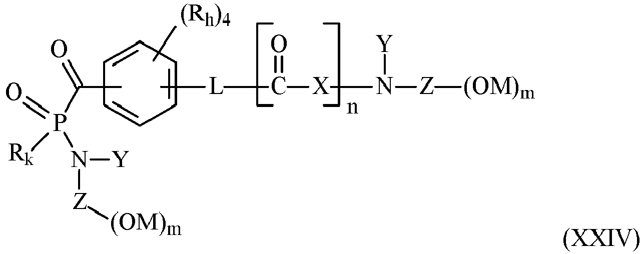 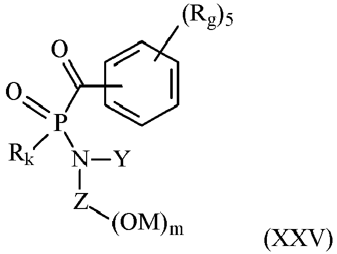 wherein
L, M, X, Y, Z, m and n are as defined above for the compound of formula (I);
Rₐ, R'ₐ, R_{b}, R'_{b}, R_{c}, R'_{c}, R_{d}, R'_{d}, Rₑ, R'ₑ, R_{f}, R'_{f}, R_{g} and Rₕ are as defined above for the PI moieties of formula (II) to (IX);
Rᵢ and R'ᵢ are as defined above for the moiety of formula (X);
Rⱼ is -OH or -NRₗR'ₗ;
Rₖ and R'ₖ are independently selected from aryl, alkyl and alkoxy;
Rₗ and R'ₗ are independently selected from alkyl or Rₗ and R'ₗ may, together with the nitrogen atom to which they are attached, form a 5 to 8 membered ring.

The compound of formula (I) may be a benzophenone-based photoinitiator, in other words a compound comprising an optionally substituted benzophenone moiety. In particular, the compound of formula (I) may be a benzophenone-based photoinitiator according to one of formula (XII), (XIII) or (XIV) as defined above.

The compound of formula (I) may be a thioxanthone-based photoinitiator, in other words a compound comprising an optionally substituted thioxanthone moiety. In particular, the compound of formula (I) may be a thioxanthone-based photoinitiator according to one of formula (XV), (XVI) or (XVII) as defined above, wherein E is S.

The compound of formula (I) may be an anthraquinone-based photoinitiator, in other words a compound comprising an optionally substituted anthraquinone moiety. In particular, the compound of formula (I) may be an anthraquinone-based photoinitiator according to one of formula (XV), (XVI) or (XVII) as defined above, wherein E is -C(=O)-.

The compound of formula (I) may be a xanthone-based photoinitiator, in other words a compound comprising an optionally substituted xanthone moiety. In particular, the compound of formula (I) may be a xanthone-based photoinitiator according to one of formula (XV), (XVI) or (XVII) as defined above, wherein E is O.

The compound of formula (I) may be an acridone-based photoinitiator, in other words a compound comprising an optionally substituted acridone moiety. In particular, the compound of formula (I) may be an acridone-based photoinitiator according to one of formula (XV), (XVI) or (XVII) as defined above, wherein E is NRₗ.

The compound of formula (I) may be an acetophenone-based photoinitiator, in other words a compound comprising a *-C(=O)-Ph moiety wherein Ph is an optionally substituted phenyl and the symbol * represents a point of attachment to a carbon atom. In particular, the compound of formula (I) may be an acetophenone-based photoinitiator according to one of formula (XVIII), (XIX) or (XX) as defined above.

The compound of formula (I) may be an acylphosphine oxide-based photoinitiator, in other words a compound comprising a -P(=O)-C(=O)-Ph moiety wherein Ph is an optionally substituted phenyl. In particular, the compound of formula (I) may be an acylphosphine oxide-based photoinitiator according to one of formula (XXI), (XXII) (XXIII) or (XXIV) as defined above.

In the compounds of formula (I) and (XII) to (XXV), PI is connected to a linker L. Each L is independently selected from a direct bond or a linker comprising 1 to 20 carbon atoms. In particular, the linker comprising 1 to 20 carbon atoms may be a divalent or trivalent linker. In particular, the linker comprising 1 to 20 carbon atoms may further optionally comprise one or more heteroatoms, such as O or S. More particularly, the linker comprising 1 to 20 carbon atoms may be:
- an acyclic linker which may be saturated or unsaturated, in particular an acyclic linker having from 1 to 6, from 1 to 4 or from 1 to 2 carbon atoms; or
- a cyclic linker which may be aromatic or non-aromatic, monocyclic or polycyclic, in particular a cyclic linker having from 6 to 20, from 7 to 15 or from 7 to 13 carbon atoms.

In a preferred embodiment, each L is independently selected from direct bond, C1-C6 alkylene, C1-C6 oxyalkylene, C1-C6 alkenylene, C1-C6 thioalkylene, C1-C6 ketoalkylene, C1-C6 ketoalkenylene, C7-C13 ketoarylene, C6-C13 ketocycloalkylene;
more particularly each L is independently selected from a direct bond, an alkylene of formula (XXVI) or (XXVII), an oxyalkylene of formula (XXVIII), a thioalkylene of formula (XXIX), a ketoalkylene of formula (XXX), a ketoalkenylene of formula (XXXI), a ketoarylene of formula (XXXII), a ketocycloalkylene of formula (XXXIII) 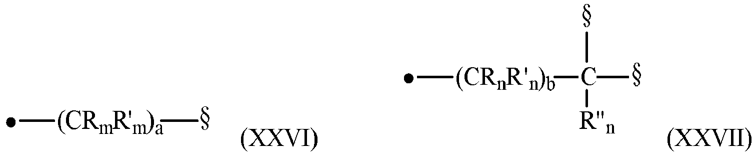 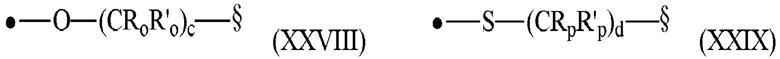 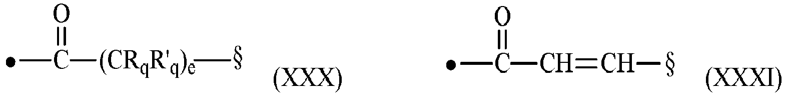 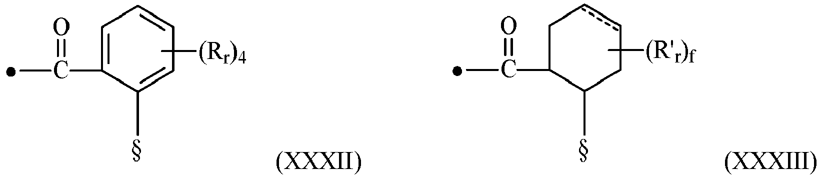 wherein
each Rₘ, R'ₘ, Rₙ, R'ₙ, R"ₙ, Rₒ, R'ₒ, Rₚ, R'ₚ, R_{q} and R'_{q} is independently H or alkyl, in particular H;
each Rᵣ and R'ᵣ is independently selected from H, F, Cl, Br, I, -OR₂, -SR₂, -N(R₂)₂, -NO₂,
-CN, -C(=O)R₂, -O-C(=O)R₂, -C(=O)OR₂, -C(=O)N(R₂)₂, -NR₂-C(=O)-R₂, -SO₂-N(R₂)₂,
-C(=O)-P(=O)R₃R₄, or an optionally substituted group selected from alkyl, heteroatom-containing alkyl, cycloalkyl, heterocycloalkyl, alkenyl, alkynyl, aryl, aralkyl, alkaryl and heteroaryl;
two adjacent Rᵣ or R'ᵣ groups may form, with the carbon atoms to which they are attached, a 5 to 8 membered ring;
each R₂ is independently H or an optionally substituted group selected from alkyl, cycloalkyl, heterocycloalkyl, alkenyl, alkynyl, aryl, aralkyl, alkaryl, heteroaryl and heteroatom-containing alkyl;
R₃ and R₄ are independently an optionally substituted group selected from aryl, alkyl, alkoxy or amino
a is 1, 2, 3, 4, 5 or 6, in particular 1 or 2;
b is 1, 2, 3, 4 or 5, in particular 1;
c is 1, 2, 3, 4, 5 or 6, in particular 1 or 2;
d is 1, 2, 3, 4, 5 or 6, in particular 1 or 2;
e is 1, 2, 3, 4 or 5, in particular 1;
f is 6 or 8;
the partially hashed bond 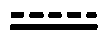 represents either a single C-C bond or a double C=C bond; the symbol ● represents a point of attachment to the PI moiety;
the symbol § represents a point of attachment to a -C(=O)-X-N(Y)-Z-(OM)ₘ moiety.

More particularly, each L may independently be selected from direct bond, an alkylene of formula (XXVI) or (XXVII) and an oxyalkylene of formula (XXVIII) as defined above.

In one embodiment, q is 1, p is 2 and L is an alkylene of formula (XXVII), in particular an alkylene of formula (XXVII) wherein
Rₙ, R'ₙ and R"ₙ are H;
a is 1.

In another embodiment, q is 2, p is 1 and each L is independently selected from direct bond and oxyalkylene of formula (XXVIII), in particular direct bond and oxyalkylene of formula (XXVIII) wherein
Rₒ, and R'ₒ are H;
c is 1.

When q is 2 and p is 1, at least one L may be an oxyalkylene of formula (XXVIII). For example, one L may be direct bond and the other L may be an oxyalkylene of formula (XXVIII) or both L may be an oxyalkylene of formula (XXVIII).

When L is a direct bond, it is directly connected to a -C(=O)-X- moiety (i.e. n is 1). When L is other than a direct bond, it is preferably directly connected to a -C(=O)-X- moiety (i.e. n is 1).

In the compounds of formula (I) and (XII) to (XXV), when n is 1, each X is independently selected from a direct bond, a -P(=O)(R')- bond and a linker comprising 1 to 6, in particular 1 to 4, more particularly 1 to 3, carbon atoms. R' is aryl, alkyl or alkoxy. In particular, the linker comprising 1 to 6 carbon atoms may be an acyclic linker which may be saturated or unsaturated, preferably saturated.

In a preferred embodiment, each X is independently selected from direct bond, C1-C6 alkylene and -P(=O)(R')- where R' is aryl, alkyl or alkoxy;
in particular each X is independently selected from a direct bond, an alkylene of formula -(CRₛR'ₛ)g- and -P(=O)(R')- where R' is phenyl;
wherein each Rₛ and R'ₛ is independently H or alkyl, in particular alkyl;
g is 1, 2, 3, 4, 5 or 6, in particular 1.

In particular, in a moiety of formula -L-[C(=O)-X]ₙ-, when X is a -P(=O)(R')- bond, then L is a direct bond.

In particular, in a moiety of formula -L-[C(=O)-X]ₙ-, when X is a direct bond then L is not a direct bond (in other words, n is 1).

When X is a -P(=O)(R')- bond, the compound of formula (I) may comprise a single -N(Y)-Z-(OM)ₘ moieties where Y is other than H. When X is a -P(=O)(R')- bond and the compound of formula (I) comprises a single -N(Y)-Z-(OM)ₘ moieties where Y is other than H, then m may be 2 or 3, in particular 3. Preferably, when X is a -P(=O)(R')- bond, the compound of formula (I) may comprise at least one -N(Y)-Z-(OM)ₘ moiety where Y is -Z'-(OM')_{m'}. In such a case, m and m' are preferably 1 or 2, preferably 1. Alternatively, when X is a -P(=O)(R')- bond and the compound of formula (I) is a phosphine-oxide based photoinitiator, it may comprise at least one -N(Y)-Z-(OM)ₘ moiety where Y is H. In such a case, m is preferably 3.

When X is other than a -P(=O)(R')- bond, the compound of formula (I) may comprise at least two -N(Y)-Z-(OM)ₘ moieties where Y is other than H, preferably the compound of formula (I) may comprise at least two -N(Y)-Z-(OM)ₘ moieties where Y is -Z'-(OM')ₘ'. When X is other than a -P(=O)(R')- bond and the compound of formula (I) comprises at least two -N(Y)-Z-(OM)ₘ moieties where Y is -Z'-(OM')ₘ', then m may be 1 or 2, preferably 1. When X is other than a -P(=O)(R')- bond and the compound of formula (I) comprises at least two -N(Y)-Z-(OM)ₘ moieties where Y is alkyl, then m may be 2 or 3, in particular 3.

In the compounds of formula (I) and (XII) to (XXV), each Y is independently H, alkyl or -Z'-(OM')_{m'}. Z' and m' are as defined below.

In a preferred embodiment, each Y is independently alkyl or -Z'-(OM')ₘ', in particular methyl, ethyl or -Z'-(OM')ₘ', more particularly -Z'-(OM')_{m'}. All of the Y groups may be -Z'-(OM')_{m'}. Alternatively, all of the Y groups may be alkyl. Alternatively part of the Y groups may be -Z'-(OM')ₘ' and part of the Y groups may be alkyl.

When Y is H, the following embodiments are preferred:
- at least one of m, p or q is 2 or 3; or
- L is a direct bond, n is 1 and X is a -P(=O)(R')- bond.

When Y is alkyl, at least one of m, p or q is preferably 2 or 3, more preferably 3.

In the compounds of formula (I) and (XII) to (XXV), each Z and Z' is independently a linker comprising 1 to 6, in particular 2 to 5, more particularly 2 to 4, carbon atoms. In particular, the linker comprising 1 to 6 carbon atoms may be an acyclic linker which may be saturated or unsaturated, preferably saturated.

In a preferred embodiment, each Z and Z' is independently an alkylene; in particular each Z and Z' is independently an alkylene of formula (XXXIV), (XXXV) or (XXXVI), even more particularly an alkylene of formula (XXXIV) 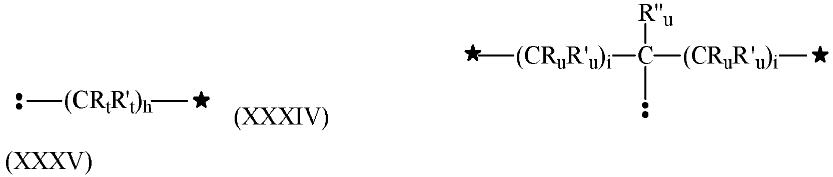 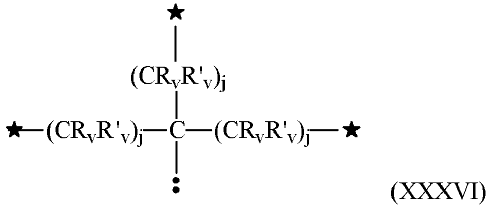
wherein each Rₜ, R'ₜ, Rᵤ, R'ᵤ, Rᵥ, R'ᵥ is independently H or alkyl, in particular H;
R"ᵤ is H or alkyl, in particular alkyl;
h is 1, 2, 3, 4, 5 or 6, in particular 2;
each i is independently 1, 2 or 3, in particular 1;
each j is independently 1, 2 or 3, in particular 1;
the symbol 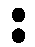 represents a point of attachment to the nitrogen atom;
each symbol 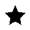 represents a point of attachment to an -OM or -OM' group.

The compounds of formula (I) and (XII) to (XXV), may have a total number of -OM and -OM' groups of at least 2, in particular at least 3, more particularly at least 4. As used herein, the total number of OM and OM' groups in the compound corresponds to the sum of all the OM and OM' groups in the compound. If the compound has a total number of OM and OM' groups that is less than 4, it preferably comprises a -P(=O)(R')- bond. Thioxanthone-based photoinitiators, benzophenone-based photoinitiators and acetophenone-based photoinitiators may have a total number of OM and OM' groups that is equal to 3 or 4. Phosphine oxide-based photoinitiators may have a total number of OM and OM' groups that is equal to 2, 3 or 4.

In one embodiment, all of the M and M' groups may be H. In another embodiment, all of the M and M' groups are (meth)acryloyl. In yet another embodiment, part of the M and M' groups are H and part of the M and M' groups are (meth)acryloyl.

In a preferred embodiment, at least one of the -L-[[C(=O)-X]ₙ-NY-Z-(OM)ₘ]ₚ arms borne by the photoinitiator moiety PI of the compound of formula (I) comprises an amide bond, an amine bond, or an aminophosphine bond. An amide bond may be formed when L is other than direct bond, n is 1 and X is bond. Preferably, the amide bond is a substituted amide bond, i.e. Y is not H. An amine bond may be formed when L is bond, n is 1 and X is alkylene. Preferably, the amine bond is a tertiary amine bond, i.e. Y is not H. An aminophosphine bond may be formed when L is bond, n is 1 and X is -P(=O)(R')-.

In a preferred embodiment, the compound of formula (I) may be a benzophenone-based photoinitiator according to formula (XII) wherein:
L is an alkylene of formula (XXVII);
n is 1;
X is a direct bond;
Y is -Z'-(OM')_{m'};
Z and Z' are independently an alkylene of formula (XXXIV);
M and M' are independently H or (meth)acryloyl;
m and m' are l;
each Ra and R'a is independently as defined above for the PI moiety of formula (II), preferably Ra and R'a are independently H or -C(=O)R₂.

In a preferred embodiment, the compound of formula (I) may be a benzophenone-based photoinitiator according to formula (XIII) or (XIV) wherein:
each L is independently a direct bond or an oxyalkylene of formula (XXVIII), preferably at least one of L is an oxyalkylene of formula (XXVIII) and the other L is a direct bond or an oxyalkylene of formula (XXVIII);
n is 1;
X is a direct bond;
Y is -Z'-(OM')_{m'};
Z and Z' are independently an alkylene of formula (XXXIV);;
M and M' are independently H or (meth)acryloyl;
m and m' are 1;
each R_{b}, R'_{b}, R_{c} and R'_{c} is independently as defined above for the PI moiety of formula (III) or (IV), preferably R_{b} R'_{b}, R_{c} and R'_{c} are independently H or -C(=O)R₂.

In a preferred embodiment, the compound of formula (I) may be a thioxanthone-based photoinitiator according to formula (XV) wherein:
E is S;
L is an alkylene of formula (XXVII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each R_{d} and R'_{d} is independently as defined above for the PI moiety of formula (V), preferably R_{d} and R'_{d} are independently H or -C(=O)R₂.

In a preferred embodiment, the compound of formula (I) may be a thioxanthone-based photoinitiator according to formula (XVI) or (XVII) wherein:
E is S;
each L is independently a direct bond or an oxyalkylene of formula (XXVIII), preferably at least one of L is an oxyalkylene of formula (XXVIII) and the other L is a direct bond or an oxyalkylene of formula (XXVIII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each Rₑ, R'ₑ, R_{f} and R'_{f} is independently as defined above for the PI moiety of formula (VI) or (VII), preferably Rₑ, R'ₑ, R_{f} and R'_{f} are independently H or -C(=O)R₂.

In a preferred embodiment, the compound of formula (I) may be an anthraquinone-based photoinitiator according to formula (XV) wherein:
E is -C(=O)-;
L is an alkylene of formula (XXVII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each R_{d} and R'_{d} is independently as defined above for the PI moiety of formula (V), preferably R_{d} and R'_{d} are independently H or -C(=O)R₂.

In a preferred embodiment, the compound of formula (I) may be an anthraquinone -based photoinitiator according to formula (XVI) or (XVII) wherein:
E is -C(=O)-;
each L is independently a direct bond or an oxyalkylene of formula (XXVIII), preferably at least one of L is an oxyalkylene of formula (XXVIII) and the other L is a direct bond or an oxyalkylene of formula (XXVIII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each Rₑ, R'ₑ, R_{f} and R'_{f} is independently as defined above for the PI moiety of formula (VI) or (VII), preferably Rₑ, R'ₑ, R_{f} and R'_{f} are independently H or -C(=O)R₂.

In a preferred embodiment, the compound of formula (I) may be a xanthone-based photoinitiator according to formula (XV) wherein:
E is O;
L is an alkylene of formula (XXVII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each R_{d} and R'_{d} is independently as defined above for the PI moiety of formula (V), preferably R_{d} and R'_{d} are independently H or -C(=O)R₂.

In a preferred embodiment, the compound of formula (I) may be a xanthone-based photoinitiator according to formula (XVI) or (XVII) wherein:
E is O;
each L is independently a direct bond or an oxyalkylene of formula (XXVIII), preferably at least one of L is an oxyalkylene of formula (XXVIII) and the other L is a direct bond or an oxyalkylene of formula (XXVIII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each Rₑ, R'ₑ, R_{f} and R'_{f} is independently as defined above for the PI moiety of formula (VI) or (VII), preferably Rₑ, R'ₑ, R_{f} and R'_{f} are independently H or -C(=O)R₂.

In a preferred embodiment, the compound of formula (I) may be an acridone-based photoinitiator according to formula (XV) wherein:
E is NRi;
L is an alkylene of formula (XXVII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each R_{d} and R'_{d} is independently as defined above for the PI moiety of formula (V), preferably R_{d} and R'_{d} are independently H or -C(=O)R₂.

In a preferred embodiment, the compound of formula (I) may be an acridone-based photoinitiator according to formula (XVI) or (XVII) wherein:
E is NRₗ;
each L is independently a direct bond or an oxyalkylene of formula (XXVIII), preferably at least one of L is an oxyalkylene of formula (XXVIII) and the other L is a direct bond or an oxyalkylene of formula (XXVIII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
each Rₑ, R'ₑ, R_{f} and R'_{f} is independently as defined above for the PI moiety of formula (VI) or (VII), preferably Rₑ, R'ₑ, R_{f} and R'_{f} are independently H or -C(=O)R₂.

In a preferred embodiment, the compound of formula (I) may be an acetophenone-based photoinitiator according to formula (XVIII) wherein:
L is an alkylene of formula (XXVII);
n is 1;
X is a direct bond;
Y is -Z'-(OM')_{m'};
Z and Z' are independently alkylene of formula (XXXIV);
M and M' are independently H or (meth)acryloyl;
m and m' are 1;
Rᵢ and R'ᵢ are independently an alkyl or Rᵢ and R'ᵢ form a ring;
Rⱼ is -OH or -NRₗR'ₗ;
Rₗ and R'ₗ are independently an alkyl or Rₗ and R'ₗ form a ring;
each R_{g} is independently as defined above for the PI moiety of formula (VIII), preferably each R_{g} is H.

In a preferred embodiment, the compound of formula (I) may be an acetophenone-based photoinitiator according to formula (XIX) wherein:
each L is independently an oxyalkylene of formula (XXVIII);
n is 1;
X is a direct bond;
Y is -Z'-(OM')_{m'};
Z and Z' are independently alkylene of formula (XXXIV);
M and M' are independently H or (meth)acryloyl;
m and m' are 1;
Rᵢ and R'ᵢ are independently an alkyl or Rᵢ and R'ᵢ form a ring;
Rⱼ is -OH or -NRₗR'ₗ;
Rₗ and R'ₗ are independently an alkyl or Rₗ and R'ₗ form a ring;
each Rₕ is independently as defined above for the PI moiety of formula (IX), preferably each Rₕ is H.

In a preferred embodiment, the compound of formula (I) may be an acetophenone-based photoinitiator according to formula (XX) wherein:
L is an oxyalkylene of formula (XXVIII);
n is 1;
X is a direct bond;
Y is -Z'-(OM')_{m'};
Z and Z' are independently alkylene of formula (XXXIV);
M and M' are independently H or (meth)acryloyl;
m and m' are 1;
Rᵢ and R'ᵢ are independently an alkyl;
each Rₕ is independently as defined above for the PI moiety of formula (IX), preferably each Rₕ is H.

In a preferred embodiment, the compound of formula (I) may be an acylphosphine oxide-based photoinitiator according to formula (XXI) wherein:
L is an alkylene of formula (XXVII);
n is 1;
X is a direct bond;
Y is -Z'-(OM')_{m'};
Z and Z' are independently an alkylene of formula (XXXIV);
M and M' are independently H or (meth)acryloyl;
m and m' are 1;
Rₖ and R'ₖ are aryl, alkyl, alkoxy;
each R_{g} is independently as defined above for the PI moiety of formula (VIII), preferably each R_{g} is independently H or alkyl.

In a preferred embodiment, the compound of formula (I) may be an acylphosphine oxide-based photoinitiator according to formula (XXII) or (XXIII) wherein:
L is a direct bond;
n is 1;
X is an alkylene of formula -(CRₛR'ₛ)_{g}-;
Y is -Z'-(OM')_{m'};
Z and Z' are independently an alkylene of formula (XXXIV);
M and M' are independently H or (meth)acryloyl;
m and m' are 1;
Rₖ and R'ₖ are aryl, alkyl, alkoxy;
each R_{g} is independently as defined above for the PI moiety of formula (VIII), preferably each R_{g} is independently H or alkyl;
each Rₕ is independently as defined above for the PI moiety of formula (IX), preferably each Rₕ is independently H or alkyl.

In a preferred embodiment, the compound of formula (I) may be an acylphosphine oxide-based photoinitiator according to formula (XXIV) wherein:
L is a direct bond;
n is 1;
X is an alkylene of formula -(CRₛR'ₛ)_{g}-;
Y is -Z'-(OM')_{m'};
Z and Z' are independently an alkylene of formula (XXXIV);
M and M' are independently H or (meth)acryloyl;
m and m' are 1;
Rₖ is aryl, alkyl, alkoxy;
each Rₕ is independently as defined above for the PI moiety of formula (IX), preferably each Rₕ is independently H or alkyl.

In a preferred embodiment, the compound of formula (I) may be an acylphosphine oxide-based photoinitiator according to formula (XXV) wherein:
Y is -Z'-(OM')_{m'};
Z and Z' are independently an alkylene of formula (XXXIV);
M and M' are independently H or (meth)acryloyl;
m and m' are 1;
Rₖ is aryl, alkyl, alkoxy;
each R_{g} is independently as defined above for the PI moiety of formula (VIII), preferably each R_{g} is independently H or alkyl.

In a preferred embodiment, the compound of formula (I) may be an acylphosphine oxide-based photoinitiator according to formula (XXV) wherein:
Y is H;
Z is an alkylene of formula (XXXVI);
M is H or (meth)acryloyl;
m is 3;
Rₖ is aryl, alkyl, alkoxy;
each R_{g} is independently as defined above for the PI moiety of formula (VIII), preferably each R_{g} is independently H or alkyl.

Particularly preferred examples of photoinitiators of formula (I) according to the invention include, but are not limited to, the following compounds: 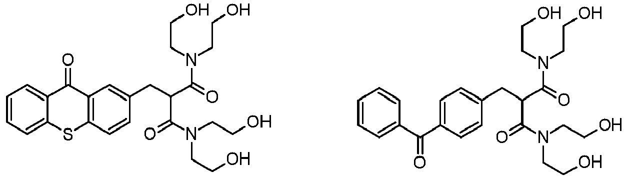 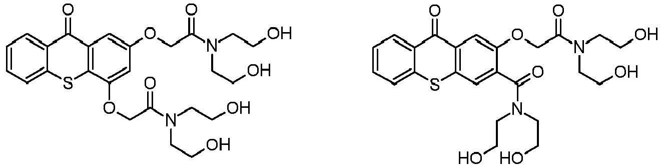 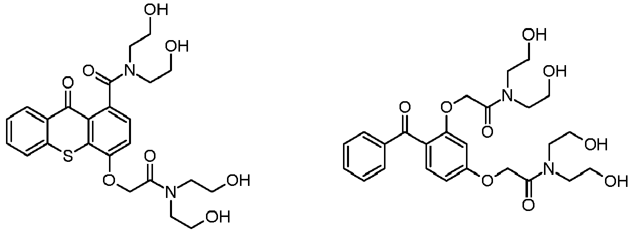 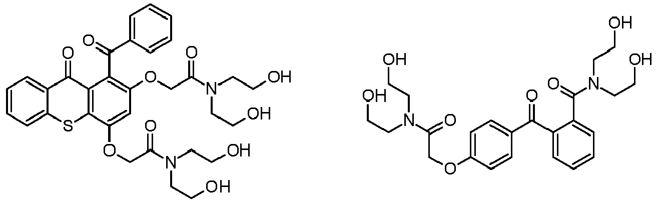 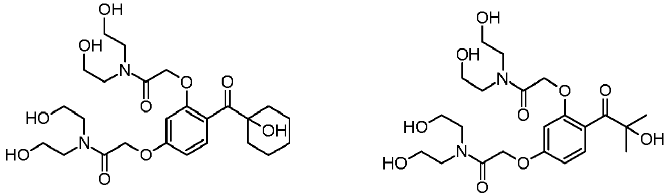 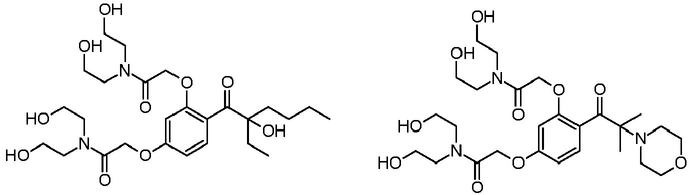 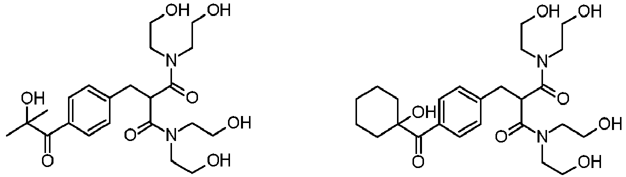 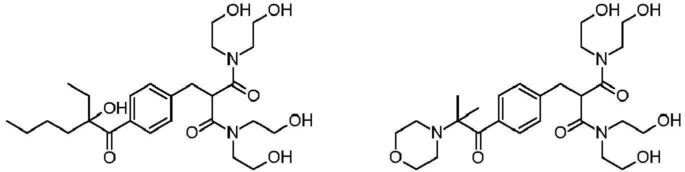 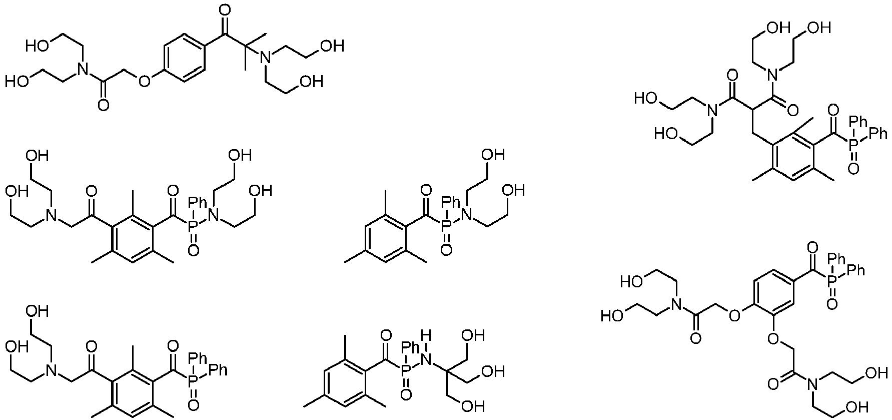 isomers thereof and combinations thereof.

The term "isomers thereof' refers to position isomers, in other words compounds that differ from one another by the position of the substituents on the aryl moiety (i.e. on the benzophenone, thioxanthone, or phenyl moiety).

### Method for preparing compounds of formula (I)

Compounds of formula (I) may be obtained using conventional reactions.

A compound of formula (I) bearing at least one -C(=O)-N(Y)-Z-(OH)ₘ moiety (i.e. a moiety containing an amide bond substituted with at least hydroxylated group) may be obtained by amidation of the corresponding ester or carboxylic acid with an amine bearing at least one hydroxylated group (i.e. HN(Y)-Z-(OH)ₘ).

A compound of formula (I) bearing at least one (meth)acryloyloxy group may be obtained by (meth)acrylation of the corresponding alcohol. Part or all of the hydroxy groups of a compound of formula (I) may be converted into (meth)acryloyloxy groups by direct esterification with (meth)acrylic acid in the presence of an acid catalyst or by transesterification with a (C1-C6)alkyl (meth)acrylate in the presence of a transesterification catalyst.

The invention thus relates to a process for preparing a compound of formula (I) wherein at least part of the M groups are (meth)acryloyl, the process comprising reacting a compound of formula (I) wherein all of the M groups are H with (meth)acrylic acid or a (C1-C6)alkyl (meth)acrylate in the presence of a catalyst. As used herein, the term "(C1-C6)alkyl (meth)acrylate" means a compound of formula A'-O-C(=O)-CHR=CH₂, wherein A' is a C1-C6 alkyl and R is H or Me. When (meth)acrylic acid is used, the catalyst may be an acid catalyst. When a (C1-C6)alkyl (meth)acrylate is used, the catalyst may be an transesterification catalyst, in particular a metal-based transesterification catalyst, more particularly a transesterification catalyst based on a metal selected from Zn, Zr, Ti or Sn, even more particularly Zn(acetylacetonate)₂, Zr(acetylacetonate)₄, Ti(isopropoxy)₄ or nBuSnOOH.

Compounds of formula (XII) comprising an amide bond (n is 1 and X is a direct bond) may be obtained with an acylation reaction followed by an amidation reaction with a hydroxylated amine. In particular, compounds of formula (XIIa) having a benzophenone moiety may be obtained by reacting an acyl halide (such as an acyl chloride) of formula (1) with a substituted phenyl of formula (2) in the presence of a Lewis acid (such as a metal halide, for example iron chloride or aluminum chloride) to form a diacid or diester of formula (3) which may be subsequently transformed in an amide of formula (XIIa) by reaction with a hydroxylated amine of formula (4) according to the following scheme: 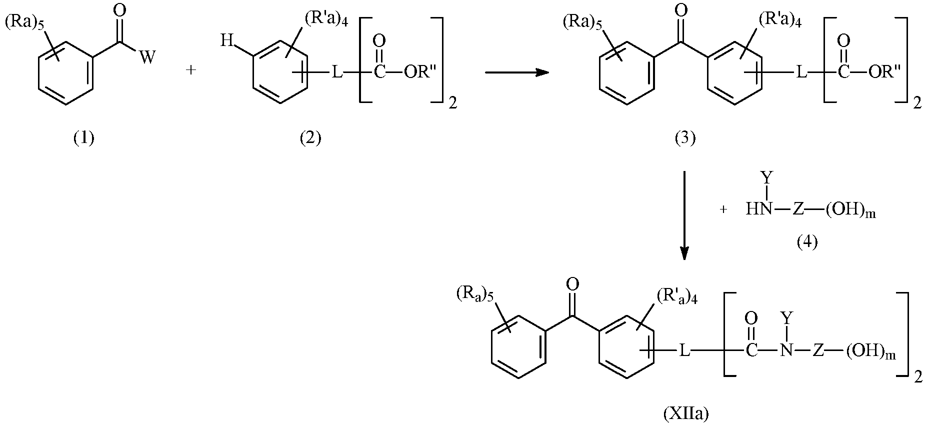 wherein
L, Rₐ, R'ₐ, Y, Z and m are as defined herein;
W is a halogen atom, in particular Cl; and
R" is H or alkyl, in particular methyl or ethyl.

Compounds of formula (XV) comprising an amide bond (n is 1 and X is a direct bond) may be obtained with an acylation reaction followed by an amidation reaction with a hydroxylated amine. In particular, compounds of formula (XVa) having a thioxanthone (E is S) or anthraquinone (E is C(=O)) moiety may be obtained by reacting an acyl halide (such as an acyl chloride) of formula (5) with a substituted phenyl of formula (6) in the presence of a Lewis acid (such as a metal halide, for example iron chloride or aluminum chloride) to form a diacid or diester of formula (7) which may be subsequently transformed in an amide of formula (XVa) by reaction with a hydroxylated amine of formula (4) according to the following scheme: 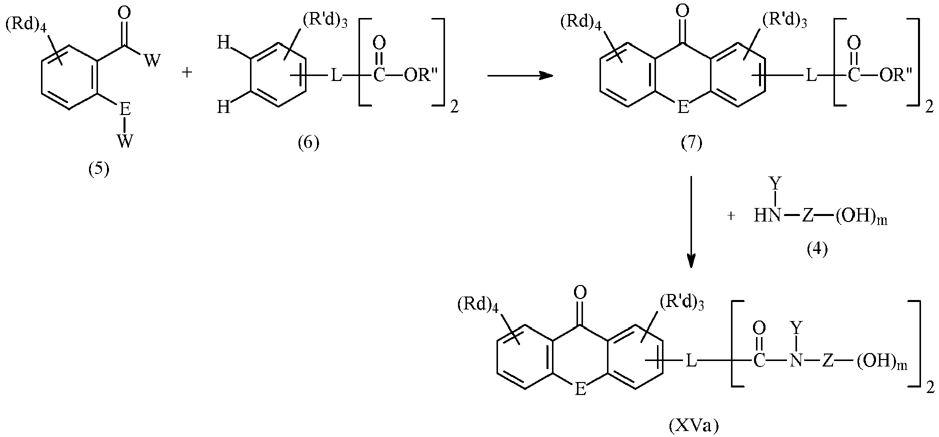 wherein
E is S or C(=O);
L, R_{d}, R'_{d}, Y, Z and m are as defined herein;
W is a halogen atom, in particular Cl; and
R" is H or alkyl, in particular methyl or ethyl.

Compounds of formula (XIIb) comprising an amide bond (n is 1 and X is a direct bond) may also be synthesized according to the schemes below by reacting malonate esters of formula (9) with haloalkyl- or acyl halide-substituted benzophenones of formula (8) wherein T is alkylene or C=O, in the presence of a suitable base (e.g. sodium or potassium hydroxide, sodium or potassium carbonate, calcium oxide). Direct cross-coupling of malonate esters of formula (9) with halo-substituted benzophenones of formula (8) wherein T is a direct bond may also be possible with the aid of a transition metal catalyst (e.g. a palladium catalyst). The resulting diester of formula (10) may be subsequently transformed in an amide of formula (XIIb) by reaction with a hydroxylated amine of formula (4). 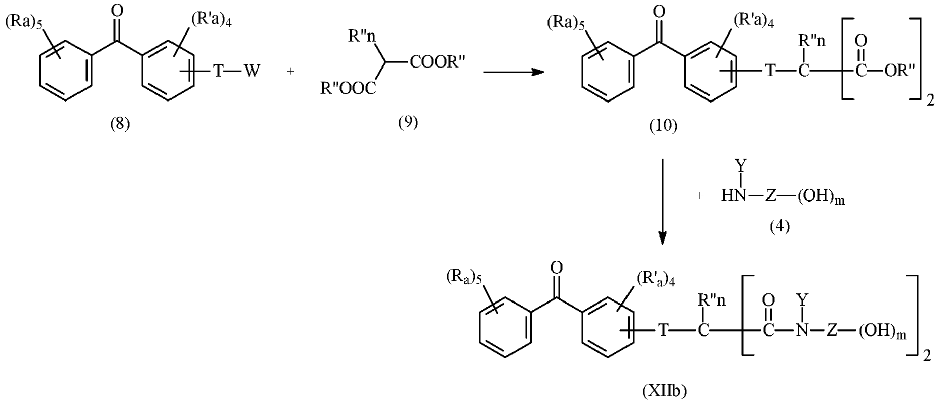 wherein
Rₐ, R'ₐ, R"ₙ, Y, Z and m are as defined herein;
T is a direct bond, alkylene or C=O;
W is a halogen atom, in particular Cl; and
R" is alkyl, in particular methyl or ethyl.

Compounds of formula (XVb) comprising an amide bond (n is 1 and X is a direct bond) may also be synthesized according to the schemes below by reacting malonate esters of formula (9) with haloalkyl- or acyl halide-substituted thioxanthones, anthraquinones, xanthones and acridones of formula (11) wherein T is alkylene or C=O, in the presence of a suitable base (e.g. sodium or potassium hydroxide, sodium or potassium carbonate, calcium oxide). Direct cross-coupling of malonate esters of formula (9) with halo-substituted thioxanthones, anthraquinones, xanthones or acridones of formula (11) wherein T is bond may also be possible with the aid of a transition metal catalyst (e.g. a palladium catalyst). The resulting diester of formula (12) may be subsequently transformed in an amide of formula (XVb) by reaction with a hydroxylated amine of formula (4). 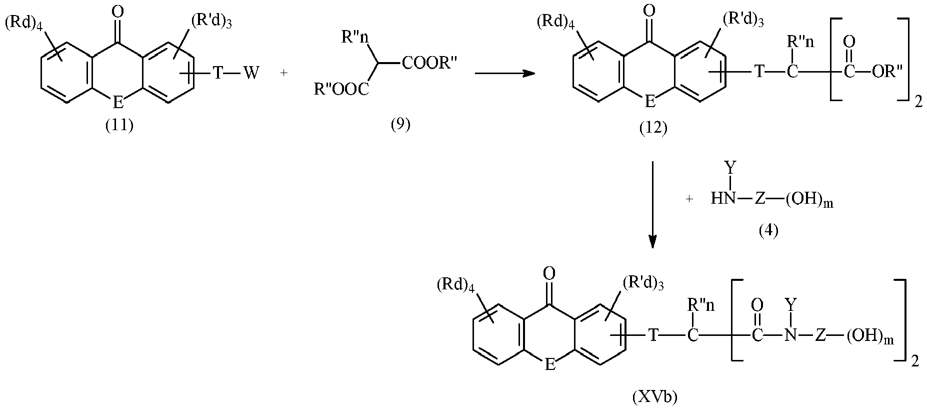 wherein
E is S, O, C(=O) or NRi;
Rₗ, R_{d}, R'_{d}, R"ₙ, Y, Z and m are as defined herein;
T is a direct bond, alkylene or C=O;
W is a halogen atom, in particular Cl; and
R" is alkyl, in particular methyl or ethyl.

Haloalkyl-substituted photoinitiators of formula (8) or (11) wherein T is alkylene may be prepared by the halogenation of alkyl-substituted photoinitiators, for instance, by using free-radical halogenation. For example, bromomethyl-substituted photoinitiators can be prepared by the radical bromination of methyl-substituted photoinitiators in the presence of a bromine source such as *N*-bromosuccinimide, and a radical initiator such as benzoyl peroxide.

Acyl halide-substituted photoinitiators of formula (8) or (11) wherein T is C(=O) may be prepared by the reaction of carboxylic acid-functionalized photoinitiators with suitable halogenating agents such as thionyl chloride.

Part or all of the OH groups in the compounds of formula (XIIa), (XIIb), (XVa) or (XVb) can then be converted into (meth)acryloyloxy groups by reaction with (meth)acrylic acid or a C1-C6 alkyl (meth)acrylate.

Compounds of formula (XIII), (XIV), (XVI) or (XVII) comprising an amide bond (n is 1 and X is a direct bond) may be obtained with an acylation reaction followed by an amidation reaction with a hydroxylated amine. A hydroxylated photo initiator of formula (13) having a benzophenone moiety (PI corresponds to formula (III) or (IV) as defined above), a thioxanthone moiety (PI corresponds to formula (VI) or (VII) as defined above with E is S), an anthraquinone moiety (PI corresponds to formula (VI) or (VII) as defined above with E is C(=O)), a xanthone moiety (PI corresponds to formula (VI) or (VII) as defined above with E is O) or an acridone moiety (PI corresponds to formula (VI) or (VII) as defined above with E is NRi) may be reacted with a halogenated ester of formula (14) wherein Q is alkylene in the presence of a suitable base (e.g. sodium or potassium hydroxide, sodium or potassium carbonate, calcium oxide) to form a diester of formula (15) which may be subsequently transformed in an amide of formula (XIIIa), (XIVa), (XVIa) or (XVIIa) by reaction with a hydroxylated amine of formula (4) according to the following scheme: 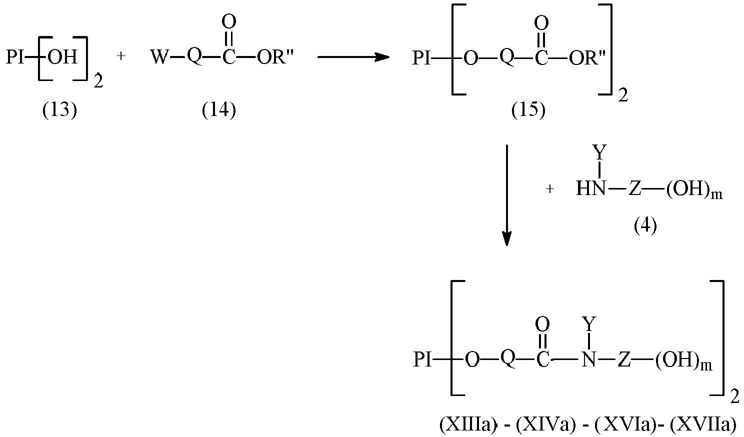 wherein
PI corresponds to formula (III), (IV), (VI) or (VII) as defined above;
Y, Z and m are as defined herein;
Q is an alkylene;
W is a halogen atom, in particular Cl; and
R" is alkyl, in particular methyl or ethyl.

Part or all of the OH groups in the compounds of formula (XIIIa), (XIVa), (XVIa) or (XVIIa) can then be converted into (meth)acryloyloxy groups by reaction with (meth)acrylic acid or a C1-C6 alkyl (meth)acrylate.

Compounds of formula (XVIII) comprising an amide bond (n is 1 and X is a direct bond) may be obtained with an acylation reaction followed by a substitution reaction and an amidation reaction with a hydroxylated amine. In particular, compounds of formula (XVIIIa) may be prepared according to the scheme below by the reaction of an aromatic malonate ester of formula (16) with an α-halo substituted acyl halide of formula (17) (such as α-bromoisobutyryl bromide), in the presence of a suitable Lewis acid catalyst (such as iron chloride or aluminium chloride). The intermediate α-halo ketone (18) thus produced can be reacted with an alkali metal hydroxide salt (19) when Rⱼ is -OH (such as sodium hydroxide) or an amine (20) when Rⱼ is -NRₗR'ₗ (such as morpholine) to give compounds of formula (21), which can be further reacted with a hydroxylated amine of formula (4) to give compounds of formula (XVIIIa). 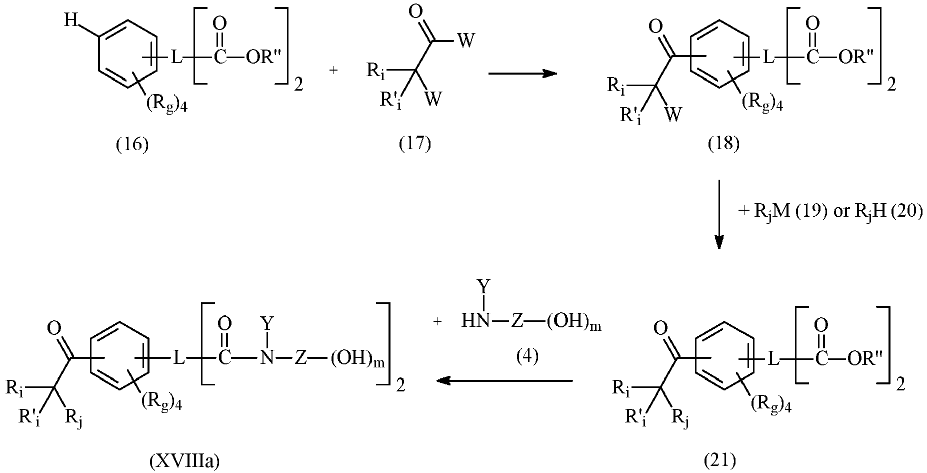 wherein
R_{g}, Rᵢ, R'ᵢ, L, Y, Z and m are as defined herein;
Rⱼ is -OH or -NRₗR'ₗ;
W is a halogen atom, in particular Br;
M is a metal ion, in particular Na or K;
R" is alkyl, in particular methyl or ethyl.

Compounds of formula (XX) comprising an amide bond (n is 1 and X is a direct bond) may be produced in a similar way as compounds of formula (XVIII) without the substitution reaction. In particular, compounds of formula (XXa) may be prepared according to the scheme below by the reaction of an aromatic ester of formula (16') with an α-halo substituted acyl halide of formula (17) (such as α-bromoisobutyryl bromide), in the presence of a suitable Lewis acid catalyst (such as iron chloride or aluminium chloride). The intermediate α-halo ketone (18') may be directly reacted with a hydroxylated amine of formula (4) to give compounds of formula (XXa). 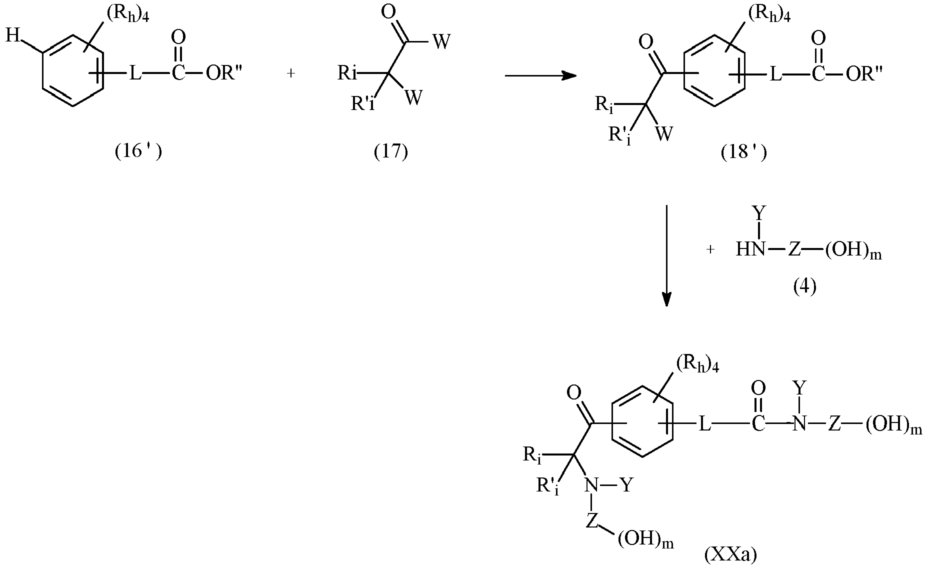 wherein
Rₕ, Rᵢ, R'ᵢ, L, Y, Z and m are as defined herein;
W is a halogen atom, in particular Br;
R" is alkyl, in particular methyl or ethyl.

Compounds of formula (XIX) comprising an amide bond (n is 1 and X is a direct bond) may be produced in a similar way as compounds of formula (XVIII), i.e. with an acylation reaction followed by a substitution reaction and an amidation reaction with a hydroxylated amine. In particular, compounds of formula (XIXa) may be prepared according to the scheme below by the reaction of an aromatic diester of formula (22) with an α-halo substituted acyl halide of formula (17) (such as α-bromoisobutyryl bromide), in the presence of a suitable Lewis acid catalyst such as iron chloride or aluminium chloride. The intermediate α-halo ketone (23) can be reacted with an alkali metal hydroxide salt (19) (such as sodium hydroxide) when Rⱼ is -OH or an amine (20) (such as morpholine) when Rⱼ is -NRₗR'ₗ to give compounds of formula (24), which can be further reacted with a hydroxylated amine of formula (4) to give compounds of formula (XIXa). 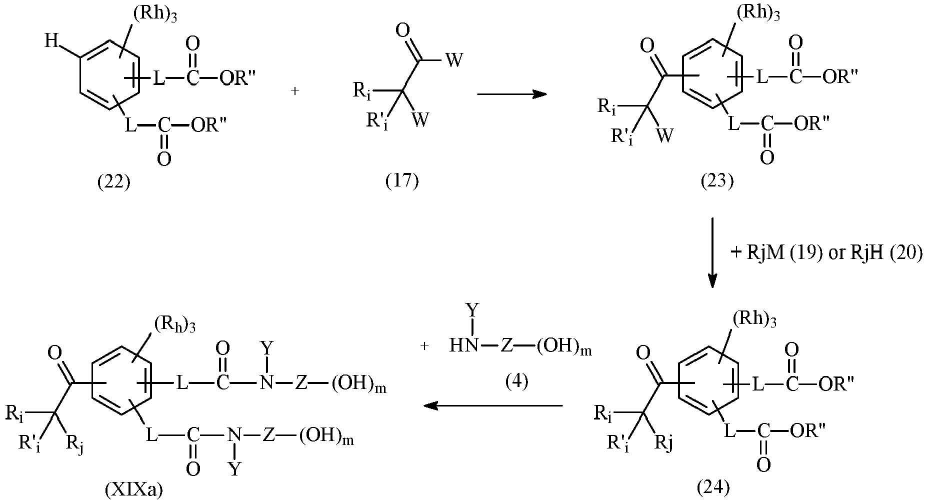 wherein
Rₕ, Rᵢ, R'ᵢ, L, Y, Z and m are as defined herein;
Rⱼ is -OH or -NRₗR'ₗ;
W is a halogen atom, in particular Br;
M is a metal ion, in particular Na or K;
R" is alkyl, in particular methyl or ethyl.

Part or all of the OH groups in the compounds of formula (XVIIIa), (XIXa) or (XXa) can then be converted into (meth)acryloyloxy groups by reaction with (meth)acrylic acid or a C1-C6 alkyl (meth)acrylate.

Compounds of formula (XXI) comprising an amide bond (n is 1 and X is a direct bond) may be produced by functionalization of a suitable aromatic acyl phosphine oxide. In particular, compounds of formula (XXIa) wherein L is an alkylene of formula (XXVII) may be obtained according to the scheme below by the reaction of an aromatic acyl phosphine oxide of formula (25) with an alkyl halide of formula (26) (such as bromomethyl methyl ether) in the presence of a suitable Lewis acid catalyst (such as zinc bromide), giving alkyl halide intermediate (27). Intermediate (27) can be further reacted with a metal salt of a malonate diester (28) to give diester intermediate (29), which can be subsequently reacted with a hydroxylated amine of formula (4) to gives compounds of formula (XXIa). 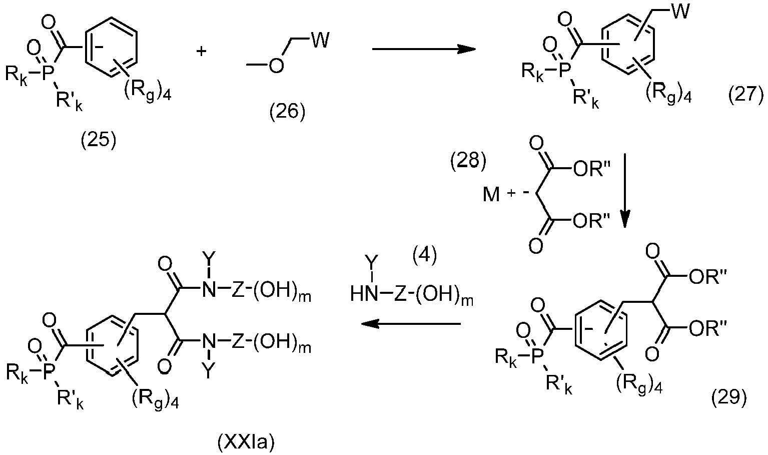 wherein
R_{g}, Rₖ, R'ₖ, Y, Z and m are as defined herein;
W is a halogen atom, in particular Br;
M is a metal ion, in particular Na or K;
R" is alkyl, in particular methyl or ethyl.

Compounds of formula (XXII) comprising a tertiary amine bond (L is direct bond, n is 1 and X is alkylene) may be produced by functionalization of a suitable aromatic acyl phosphine oxide. In particular, compounds of formula (XXIIa) may be obtained according to the scheme below by the reaction of an aromatic acyl phosphine oxide of formula (25) with an α-halo substituted acyl halide of formula (30) (such as chloroacetyl chloride) in the presence of a suitable Lewis acid catalyst, giving an alkyl halide intermediate of formula (31). Intermediate (31) can be further reacted with a hydroxylated amine of formula (4) to give a compound of formula (XXIIa). 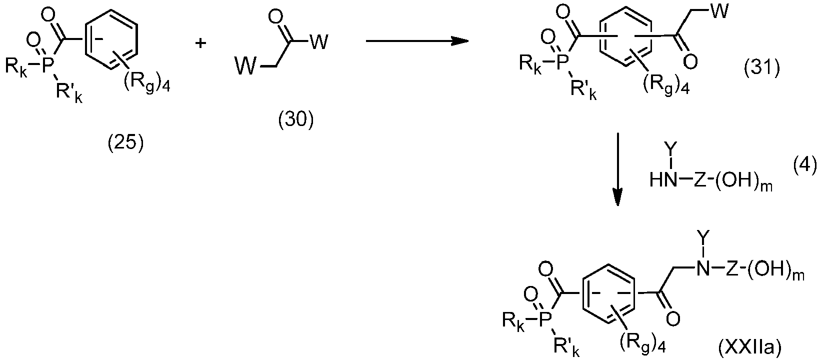 wherein
R_{g}, Rₖ, R'ₖ, Y, Z and m are as defined herein;
W is a halogen atom, in particular Cl.

Compounds of formula (XXIII) comprising an amide bond (n is 1 and X is a direct bond) may be produced by functionalization of a suitable aromatic aldehyde followed by an amidation reaction with a hydroxylated amine. In particular, compounds of formula (XXIIIa) may be obtained according to the scheme below by the reaction of an aldehyde of formula (32) with a phosphine oxide of formula (33). The alcohol intermediate (34) thus obtained can be oxidised to an acyl phosphine oxide using a suitable oxidising reagent (for example, hydrogen peroxide, in the presence of a catalyst such as vanadium(V) oxide). The diester intermediate (35) thus produced may be reacted with a hydroxylated amine of formula (4) to give a compound of formula (XXIIIa). 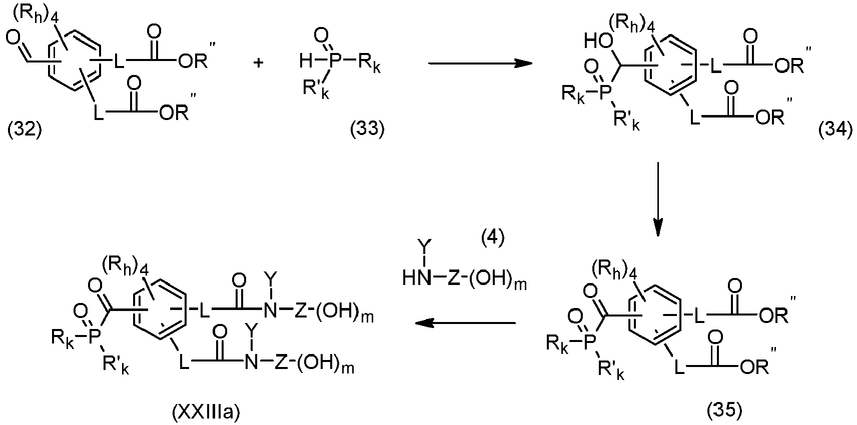 wherein
Rₕ, Rₖ, R'ₖ, L, Y, Z and m are as defined herein;
R" is alkyl, in particular methyl or ethyl.

Compounds of formula (XXIV) comprising a tertiary amine bond (L is direct bond, n is 1 and X is alkylene) may be produced by functionalization of a suitable aromatic acyl phosphine oxide. In particular, compounds of formula (XXIVa) may be prepared according to the scheme below by first converting a phosphinic acid of formula (36) to a phosphinic acid halide of formula (37) by reaction with a suitable halogenating reagent (such as thionyl chloride). The phosphinic acid halide (37) may be reacted with an α-halo substituted acyl halide of formula (30) (such as chloroacetyl chloride) in the presence of a suitable Lewis acid catalyst, giving an alkyl halide intermediate of formula (38). Intermediate (38) can be further reacted with a hydroxylated amine of formula (4) in the presence of a suitable base (e.g. an amine base such as triethylamine) to give compounds of formula (XXIVa). 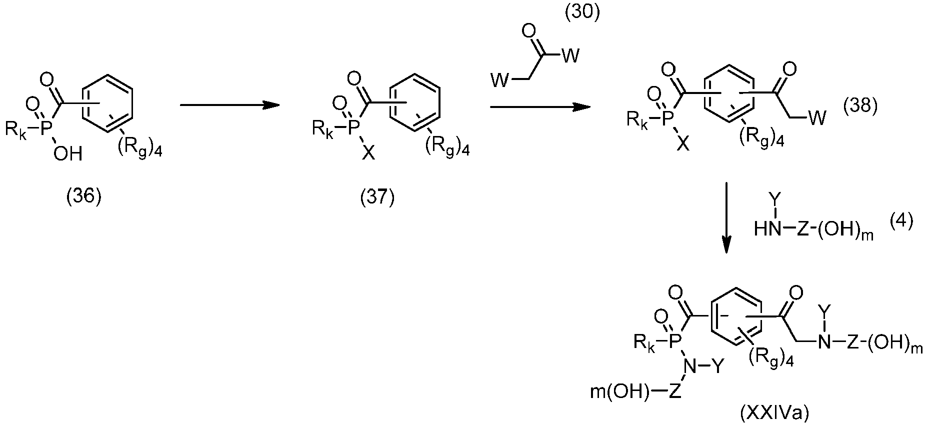 wherein
R_{g}, Rₖ, Y, Z and m are as defined herein;
W and X are independently halogen atoms, in particular Cl.

Compounds of formula (XXV) may be produced by functionalization of a suitable aromatic acyl phosphine oxide. In particular, a compound of formula (XXVa) may be prepared according to the scheme below by first converting a phosphinic acid of formula (36) to a phosphinic acid halide of formula (37) by reaction with a suitable halogenating agent (such as thionyl chloride). The phosphinic acid halide is further reacted with a hydroxylated amine of formula (4) in the presence of a suitable base (e.g. an amine base such as triethylamine) to give compounds of formula (XXVa). 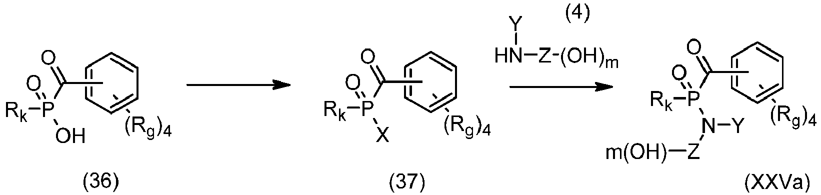 wherein
R_{g}, Rₖ, Y, Z and m are as defined herein;
X is a halogen atom, in particular Cl.

Part or all of the OH groups in the compounds of formula (XXIa), (XXIIa), (XXIIIa), (XXIVa) or (XXVa) can then be converted into (meth)acryloyloxy groups by reaction with (meth)acrylic acid or a C1-C6 alkyl (meth)acrylate.

### Photoinitiator composition

The photoinitiator composition of the invention comprises a mixture of at least two distinct photoinitiators or a mixture of at least one photoinitiator and at least one amine synergist which is distinct from the photoinitiator.

In a first embodiment, the photoinitiator composition may comprise at least one compound of formula (I) as defined above and at least one compound according to one of the following formula (XXXVII) or (XXXVIII) 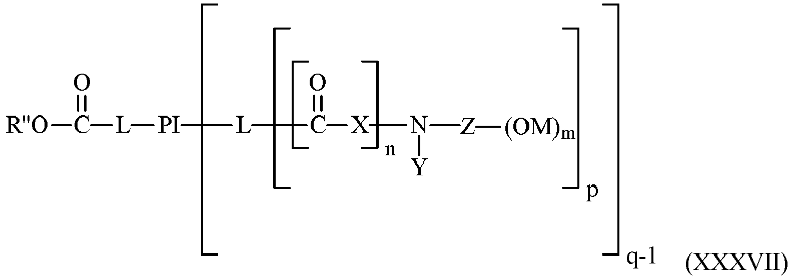 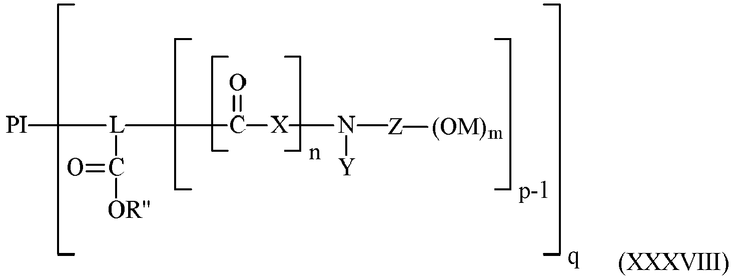 wherein PI, L, M, X, Y, Z, m, n, p and q are as defined above; and
R" is H or alkyl.

The compound of formula (XXXVII) or (XXXVIII) may correspond to an intermediate or side-product obtained during the synthesis of a compound of formula (I). For example, when the compound of formula (I) is obtained with a step of amidation of an ester or carboxylic acid as described above in the method for preparing a compound of formula (I), the compound of formula (XXXVII) or (XXXVIII) may correspond to a product that has partly reacted in the amidation reaction and contains unreacted ester or acid groups -COOR₇.

In a second embodiment, the photoinitiator composition of the invention may comprise a mixture of at least two, in particular at least three, distinct compounds of formula (I) as defined above.

The distinct compounds of formula (I) may be selected from the following compounds:
- a compound where all of the Y groups are -Z-(OM)ₘ,
- a compound where all of the Y groups are alkyl, and
- at least one compound where part of the Y groups are -Z-(OM)ₘ and part of the Y groups are alkyl;
and combinations thereof.

In particular, the photoinitiator composition of the invention may comprise:
- a compound of formula (I) where all of the Y groups are -Z-(OM)ₘ,
- a compound of formula (I) where all of the Y groups are alkyl, and
- at least one compound of formula (I) where part of the Y groups are -Z-(OM)ₘ and part of the Y groups are alkyl.

Said mixture may be obtained when the compound of formula (I) is prepared with a step of amidation of an ester or carboxylic acid, as described above in the method for preparing a compound of formula (I), using a mixture of distinct amines. For example, the amidation reaction may be carried out with an amine bearing two -Z-OH groups (such as diethanolamine) and an amine bearing one -Z-OH group and one alkyl group (such as methylethanolamine). When all of the ester or carboxylic acid groups of the starting compound react with an amine bearing two -Z-OH groups, then all of the Y groups of the resulting compound of formula (I) are -Z-OH. When all of the ester or carboxylic acid groups of the starting compound react with an amine bearing one -Z-OH group and one alkyl group, then all of the Y groups of the resulting compound of formula (I) are alkyl. When part of the ester or carboxylic acid groups of the starting compound react with an amine bearing two -Z-OH groups and the other part of the ester or carboxylic acid groups of the starting compound react with an amine bearing one -Z-OH group and one alkyl group, then part of the Y groups of the resulting compound of formula (I) are -Z-OH and the other part of the Y groups are alkyl.

In a third embodiment, the photoinitiator composition of the invention may comprise a compound of formula (I) as defined above and a photoinitiator other than a compound of formula (I).

The photoinitiator other than a compound of formula (I) may be a radical photoinitiator, in particular a radical photoinitiator having Norrish type I activity and/or Norrish type II activity.

In one embodiment, the photoinitiator other than a compound of formula (I) may be a radical photoinitiator having Norrish type I activity.

Non-limiting types of radical photoinitiators suitable for use in the curable compositions of the present invention include, for example, benzoins, benzoin ethers, acetophenones, α-hydroxy acetophenones, benzyl, benzyl ketals, anthraquinones, phosphine oxides, acylphosphine oxides, α-hydroxyketones, phenylglyoxylates, α-aminoketones, benzophenones, thioxanthones, xanthones, acridine derivatives, phenazene derivatives, quinoxaline derivatives, triazine compounds, benzoyl formates, aromatic oximes, metallocenes, acylsilyl or acylgermanyl compounds, camphorquinones, polymeric derivatives thereof, and mixtures thereof.

Examples of suitable radical photoinitiators include, but are not limited to, 2-methylanthraquinone, 2-ethylanthraquinone, 2-chloroanthraquinone, 2-benzylanthraquinone, 2-t-butylanthraquinone, 1,2-benzo-9,10-anthraquinone, benzyl, benzoins, benzoin ethers, benzoin, benzoin methyl ether, benzoin ethyl ether, benzoin isopropyl ether, alpha-methylbenzoin, alpha-phenylbenzoin, Michler's ketone, acetophenones such as 2,2-dialkoxybenzophenones and 1-hydroxyphenyl ketones, benzophenone, 4,4'-bis-(diethylamino) benzophenone, acetophenone, 2,2-diethyloxyacetophenone, diethyloxyacetophenone, 2-isopropylthioxanthone, thioxanthone, diethyl thioxanthone, 1,5-acetonaphthylene, benzil ketone, α-hydroxy keto, 2,4,6-trimethylbenzoyldiphenyl phosphine oxide, benzyl dimethyl ketal, 2,2-dimethoxy-1,2-diphenylethanone, 1-hydroxycylclohexyl phenyl ketone, 2-methyl-1-[4-(methylthio) phenyl]-2-morpholinopropanone-1, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, anthraquinone, anthraquinone-2-sulfonic acid, sodium salt monohydrate, (benzene) tricarbonylchromium, benzil, benzoin isobutyl ether, benzophenone/1-hydroxycyclohexyl phenyl ketone, 50/50 blend, 3,3',4,4'-benzophenonetetracarboxylic dianhydride, 4-benzoylbiphenyl, 2-benzyl-2-(dimethylamino)-4'-morpholinobutyrophenone, 4,4'-bis(diethylamino)benzophenone, 4,4'-bis(dimethylamino)benzophenone, camphorquinone, 2-chlorothioxanthen-9-one, dibenzosuberenone, 4,4'-dihydroxybenzophenone, 2,2-dimethoxy-2-phenylacetophenone, 4-(dimethylamino)benzophenone, 4,4'-dimethylbenzil, 2,5-dimethylbenzophenone, 3,4-dimethylbenzophenone, diphenyl(2,4,6-trimethylbenzoyl)phosphine oxide /2-hydroxy-2-methylpropiophenone, 50/50 blend, 4'-ethoxyacetophenone, 2,4,6-trimethylbenzoyldiphenylphophine oxide, phenyl bis(2,4,6-trimethyl benzoyl)phosphine oxide, ferrocene, 3'-hydroxyacetophenone, 4'-hydroxyacetophenone, 3-hydroxybenzophenone, 4-hydroxybenzophenone, 1-hydroxycyclohexyl phenyl ketone, 2-hydroxy-2-methylpropiophenone, 2-methylbenzophenone, 3-methylbenzophenone, methybenzoylformate, 2-methyl-4'-(methylthio)-2-morpholinopropiophenone, phenanthrenequinone, 4'-phenoxyacetophenone, (cumene)cyclopentadienyl iron(ii) hexafluorophosphate, 9,10-diethoxy and 9,10-dibutoxyanthracene, 2-ethyl-9,10-dimethoxyanthracene, thioxanthen-9-one and combinations thereof.

In particular, the photoinitiator other a compound of formula (I) may be a benzophenone such as SpeedCure^{®} BP (benzophenone), SpeedCure^{®} 7005 (polymeric benzophenone) or SpeedCure^{®} 7006 (polymeric benzophenone); a thioxanthone such as SpeedCure^{®} 7010 (polymeric thioxanthone), SpeedCure^{®} ITX (isopropyl thioxanthone) or SpeedCure^{®} CPTX (1-chloro-4-propoxythioxanthone); an α-hydroxy acetophenone; an acylphosphine oxide such as SpeedCure^{®} BPO (phenyl bis(2,4,6-trimethylbenzoyl)-phosphine oxide), SpeedCure^{®} TPO (2,4,6-trimethylbenzoyldiphenylphosphine oxide) or SpeedCure^{®} TPO-L (ethyl (2,4,6-trimethylbenzoyl)phenyl phosphinate). Preferably, the photoinitiator other than a compound of formula (I) is Speedcure^{®} CPTX.

In one embodiment, the photoinitiator other than a compound of formula (I) may be a benzoylformate, such as SpeedCure MBF (methylbenzoylformate). In another embodiment, the photoinitiator other a compound of formula (I) may be an acylphosphine oxide, such as SpeedCure^{®} TPO-L (ethyl (2,4,6-trimethylbenzoyl)phenyl phosphinate). In a particularly preferred embodiment, the photoinitiator other a compound of formula (I) may be an α-hydroxy acetophenone, such as SpeedCure 73 (2-hydroxy-2-methyl-1-phenylpropanone).

In another embodiment, the photoinitiator other than a compound of formula (I) may be a cationic photoinitiator. A cationic photoinitiator is advantageously present when the composition comprises a cationically polymerizable compound as defined below.

Suitable cationic photoinitiators include any type of photoinitiator that, upon exposure to radiation such as actinic radiation, forms cations (e.g., Bronsted or Lewis acids) that initiate the reaction of the monomeric and (if present) oligomeric cationically polymerizing organic substances in the curable composition. For example, a cationic photoinitiator may be comprised of a cationic portion and an anionic portion. The cationic portion of the photoinitiator molecule can be responsible for the absorption of UV radiation while the anionic portion of the molecule becomes a strong acid after UV absorption.

In particular, the photoinitiator other than a compound of formula (I) may be a cationic photoinitiator selected from onium salts with anions of weak nucleophilicity, such as halonium salts, iodonium salts (e.g., diaryliodonium salts such as bis(4-t-butylphenyl) iodonium perfluoro-1-butane sulfonate) or sulfonium salts (e.g., triarylsulfonium salts such as triarylsulfonium hexafluoroantimonate salts); sulfoxonium salts; diazonium salts; metallocene salts; and mixtures thereof.

The molar ratio between a compound of formula (I) and a photoinitiator other than a compound of formula (I) may be varied as may be appropriate depending on the photoinitiator(s) selected, the amounts and types of polymerizable species that are intended to be photopolymerized, the radiation source and the radiation conditions used, among other factors. Typically, however, the molar ratio between a compound of formula (I) and a photoinitiator other than a compound of formula (I) may be from 10/90 to 90/10, or from 20/80 to 80/20, or from 30/70 to 70/30, or from 40/60 to 60/40 or from 45/55 to 55/45. In a preferred embodiment, the molar ratio between a compound of formula (I) and a photoinitiator other than a compound of formula (I) may be from 50/50 to 90/10, or from 60/40 to 80/20, or from 70/30 to 80/20, or about 75/25.

The weight ratio between a compound of formula (I) and a photoinitiator other than a compound of formula (I) may be from 10/90 to 90/10, or from 20/80 to 80/20, or from 30/70 to 70/30, or from 40/60 to 60/40 or from 45/55 to 55/45. In a preferred embodiment, the weight ratio between a compound of formula (I) and a photoinitiator other than a compound of formula (I) may be from 50/50 to 90/10, or from 60/40 to 80/20, or from 70/30 to 80/20, or about 75/25.

In a fourth embodiment, the photoinitiator composition may comprise a compound of formula (I) as defined above and an amine synergist other than a compound of formula (I), preferably an amine synergist bearing a tertiary amine group.

Amine synergists may be introduced in the photoinitiator composition of the invention in order to act synergistically with Norrish Type II photoinitiators and/or to reduce oxygen inhibition. Amine synergists are typically tertiary amines. When used in conjunction with Norrish Type II photoinitiators, the tertiary amine provides an active hydrogen donor site for the excited triple state of the photoinitiator, thus producing a reactive alkyl-amino radical which can subsequently initiate polymerization. Tertiary amines are also able to convert unreactive peroxy species, formed by reaction between oxygen and free radicals, to reactive alkyl-amino radicals, thus reducing the effects of oxygen on curing.

Examples of suitable amine synergists include low-molecular weight tertiary amines (i.e. having a molecular weight of less than 200 g/mol) such as triethanol amine or N-methyldiethanol amine. Other types of amine synergists are aminobenzoates. Examples of aminobenzoates include ethyl 4-(dimethylamino)benzoate (EDB), pentyl 4-(dimethylamino)benzoate, 2-ethylhexyl 4-(dimethylamino)benzoate and 2-butoxyethyl 4-(dimethylamino)benzoate (BEDB).

The weight ratio between a compound of formula (I) and an amine synergist other than a compound of formula (I) may be from 10/90 to 90/10, or from 20/80 to 80/20, or from 30/70 to 70/30, or from 40/60 to 60/40 or from 45/55 to 55/45. In a preferred embodiment, the weight ratio between a compound of formula (I) and an amine synergist other than a compound of formula (I) may be from 10/90 to 60/40, or from 15/85 to 50/50, or from 20/80 to 40/60, or about 30/70.

### Photopolymerization process

The compound of formula (I) as defined above or the photoinitiator compositions as defined above may be used in a photopolymerization process, i.e. a process for photopolymerizing (e.g. curing) one or more ethylenically unsaturated compounds.

The process for photopolymerizing one or more ethylenically unsaturated compounds comprises contacting one or more ethylenically unsaturated compounds with a compound of formula (I) as defined above or a photoinitiator composition as defined above, and irradiating the mixture, in particular with visible, near-UV and/or UV light. When the compound of formula (I) comprises a thioxanthone moiety, the mixture may be irradiated with a LED light source, in particular a LED light source having an emission band in the range of 365-420 nm.

The ethylenically unsaturated compound(s) may be as defined below.

### Curable composition

### Composition A comprising an ethylenically unsaturated compound

The invention further relates to a curable composition comprising a compound of formula (I) as defined above or a photoinitiator composition as defined above, referred to as component
a), and further comprising an ethylenically unsaturated compound, referred to as component
b). This embodiment is referred to as Composition A.

Composition A may comprise from 0.05% to 30%, from 0.1% to 20%, from 0.2 to 15%, from 0.5 to 10% or from 1 to 5%, by weight of compound of formula (I) based on the total weight of the ethylenically unsaturated compound.

Composition A may comprise from 0.05% to 30%, from 0.1% to 20%, from 0.2 to 15%, from 0.5 to 10% or from 1 to 5%, by weight of photoinitiator other than a compound of formula (I) based on the total weight of the ethylenically unsaturated compound.

Composition A may comprise:
- from 0.05 to 30%, from 0.1 to 20%, from 0.2 to 15%, from 0.5 to 10% or from 1 to 5% of component a);
- from 70 to 99,95%, from 80 to 99.9%, from 85 to 99.8%, from 90 to 99.5% or from 95 to 99% of component b);
the % being % by weight based on the total weight of components a) and b).

Composition A comprises an ethylenically unsaturated compound. Composition A may comprise a mixture of ethylenically unsaturated compounds.

As used herein, the term "ethylenically unsaturated compound" means a compound that comprises a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate (including cyanoacrylate), methacrylate, acrylamide, methacrylamide, styrene, maleate, fumarate, itaconate, allyl, propenyl, vinyl and combinations thereof, preferably selected from acrylate, methacrylate and vinyl, more preferably selected from acrylate and methacrylate. The carbon-carbon double bonds of a phenyl ring are not considered as polymerizable carbon-carbon double bonds.

In one embodiment, the ethylenically unsaturated compound comprises a (meth)acrylate-functionalized compound, in particular a (meth)acrylate-functionalized compound selected from a (meth)acrylate-functionalized monomer, a (meth)acrylate-functionalized oligomer, an amine-modified acrylate and mixtures thereof.

The total amount of ethylenically unsaturated compound (including (meth)acrylate functionalized monomer, (meth)acrylate functionalized oligomer and amine-modified acrylate) in Composition A may be from 40 to 99.5%, in particular 50 to 95%, more particularly 60 to 90%, by weight based on the weight of the composition. In particular, Composition A may comprise 40 to 80%, or 40 to 75% or 40 to 70% or 40 to 65% or 40 to 60% by weight of ethylenically unsaturated compound based on the weight of the composition. Alternatively, Composition A may comprise 60 to 99.5%, or 65 to 99.5% or 70 to 99.5% or 75 to 99.5% or 80 to 99.5% by weight of ethylenically unsaturated compound based on the weight of the composition.

As used herein, the term "(meth)acrylate-functionalized compound" means a compound comprising a (meth)acrylate group, in particular an acrylate group. The term "(meth)acrylate-functionalized monomer" means a monomer comprising a (meth)acrylate group, in particular an acrylate group. The term "(meth)acrylate-functionalized oligomer" means an oligomer comprising a (meth)acrylate group, in particular an acrylate group. The term "(meth)acrylate group" encompasses acrylate groups (-O-CO-CH=CH₂) and methacrylate groups (-O-CO-C(CH₃)=CH₂).

In one embodiment, the ethylenically unsaturated compound comprises a (meth)acrylate-functionalized monomer. The ethylenically unsaturated compound may comprise a mixture of (meth)acrylate-functionalized monomers.

The (meth)acrylate-functionalized monomer may have a molecular weight of less than 600 g/mol, in particular from 100 to 550 g/mol, more particularly 200 to 500 g/mol.

The (meth)acrylate-functionalized monomer may have 1 to 6 (meth)acrylate groups, in particular 1 to 3 (meth)acrylate groups.

The (meth)acrylate-functionalized monomer may comprise a mixture of (meth)acrylate-functionalized monomers having different functionalities. For example the (meth)acrylate-functionalized monomer may comprise a mixture of a (meth)acrylate-functionalized monomer containing a single acrylate or methacrylate group per molecule (referred to herein as "mono(meth)acrylate-functionalized compounds") and a (meth)acrylate-functionalized monomer containing 2 or more, preferably 2 or 3, acrylate and/or methacrylate groups per molecule.

In one embodiment, the (meth)acrylate functionalized monomer comprises a mono(meth)acrylate-functionalized monomer. The mono(meth)acrylate-functionalized monomer may advantageously function as a reactive diluent and reduce the viscosity of the composition.

Examples of suitable mono(meth)acrylate-functionalized monomers include, but are not limited to, mono-(meth)acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and the like.

The following compounds are specific examples of mono(meth)acrylate-functionalized monomers suitable for use in Composition A: methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth)acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; alkoxylated tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; alkoxylated phenol (meth)acrylates; alkoxylated nonylphenol (meth)acrylates; cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethyl-butyl urethane (meth)acrylates; 3-(2-hydroxyalkyl)oxazolidinone (meth)acrylates; and combinations thereof.

In one embodiment, the (meth)acrylate functionalized monomer may comprise a (meth)acrylate-functionalized monomer containing two or more (meth)acrylate groups per molecule.

Examples of suitable (meth)acrylate-functionalized monomers containing two or more (meth)acrylate groups per molecule include acrylate and methacrylate esters of polyhydric alcohols (organic compounds containing two or more, e.g., 2 to 6, hydroxyl groups per molecule). Specific examples of suitable polyhydric alcohols include C₂₋₂₀ alkylene glycols (glycols having a C₂₋₁₀ alkylene group may be preferred, in which the carbon chain may be branched; e.g., ethylene glycol, trimethylene glycol, 1,2-propylene glycol, 1,2-butanediol, 1,3-butanediol, 2,3-butanediol, tetramethylene glycol (1,4-butanediol), 1,5-pentanediol, 1,6-hexanediol, 1,8-octanediol, 1,9-nonanediol, 1,12-dodecanediol, cyclohexane-1,4-dimethanol, bisphenols, and hydrogenated bisphenols, as well as alkoxylated (e.g., ethoxylated and/or propoxylated) derivatives thereof), diethylene glycol, glycerin, alkoxylated glycerin, triethylene glycol, dipropylene glycol, tripropylene glycol, trimethylolpropane, alkoxylated trimethylolpropane, ditrimethylolpropane, alkoxylated ditrimethylolpropane, pentaerythritol, alkoxylated pentaerythritol, dipentaerythritol, alkoxylated dipentaerythritol, cyclohexanediol, alkoxylated cyclohexanediol, cyclohexanedimethanol, alkoxylated cyclohexanedimethanol, norbornene dimethanol, alkoxylated norbornene dimethanol, norbornane dimethanol, alkoxylated norbornane dimethanol, polyols containing an aromatic ring, cyclohexane-1,4-dimethanol ethylene oxide adducts, bis-phenol ethylene oxide adducts, hydrogenated bisphenol ethylene oxide adducts, bisphenol propylene oxide adducts, hydrogenated bisphenol propylene oxide adducts, cyclohexane-1,4-dimethanol propylene oxide adducts, sugar alcohols and alkoxylated sugar alcohols. Such polyhydric alcohols may be fully or partially esterified (with (meth)acrylic acid, (meth)acrylic anhydride, (meth)acryloyl chloride or the like), provided they contain at least two (meth)acrylate functional groups per molecule.

Exemplary (meth)acrylate-functionalized monomers containing two or more (meth)acryloyloxy groups per molecule may include bisphenol A di(meth)acrylate; hydrogenated bisphenol A di(meth)acrylate; ethylene glycol di(meth)acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth)acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-nonanediol di(meth)acrylate; 1,12-dodecanediol di(meth)acrylate; neopentyl glycol di(meth)acrylate; 2-methyl-2,4-pentanediol di(meth)acrylate; poly butadiene di(meth)acrylate; cyclohexane-1,4-dimethanol di(meth)acrylate; tricyclodecane dimethanol di(meth)acrylate; metallic di(meth)acrylates; modified metallic di(meth)acrylates; glyceryl di(meth)acrylate; glyceryl tri(meth)acrylate; trimethylolethane tri(meth)acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate, sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; as well as the alkoxylated (e.g., ethoxylated and/or propoxylated) derivatives thereof; and combinations thereof.

Composition A may comprise 0 to 99.5%, in particular 5 to 90%, more particularly 10 to 80%, even more particularly 15 to 75%, more particularly still 20 to 70% by weight of (meth)acrylate-functionalized monomer based on the total weight of the composition. In particular, Composition A may comprise 5 to 50% or 10 to 50% or 15 to 50% or 20 to 50% or 25 to 50% or 30 to 50%, by weight of (meth)acrylate-functionalized monomer based on the total weight of the composition. Alternatively, the Composition A may comprise 50 to 99.5% or 55 to 99.5% or 60 to 99.5% or 65 to 99.5% or 70 to 99.5%, by weight of (meth)acrylate-functionalized monomer based on the total weight of the composition.

In one embodiment, the ethylenically unsaturated compound comprises a (meth)acrylate-functionalized oligomer. The ethylenically unsaturated compound may comprise a mixture of (meth)acrylate-functionalized oligomers.

The (meth)acrylate-functionalized oligomer may be selected in order to enhance the flexibility, strength and/or modulus, among other attributes, of a cured polymer prepared using Composition A.

The (meth)acrylate functionalized oligomer may have 1 to 18 (meth)acrylate groups, in particular 2 to 6 (meth)acrylate groups, more particularly 2 to 6 acrylate groups.

The (meth)acrylate functionalized oligomer may have a number average molecular weight equal or more than 600 g/mol, in particular 800 to 15,000 g/mol, more particularly 1,000 to 5,000 g/mol.

In particular, the (meth)acrylate-functionalized oligomers may be selected from the group consisting of (meth)acrylate-functionalized urethane oligomers (sometimes also referred to as "urethane (meth)acrylate oligomers," "polyurethane (meth)acrylate oligomers" or "carbamate (meth)acrylate oligomers"), (meth)acrylate-functionalized epoxy oligomers (sometimes also referred to as "epoxy (meth)acrylate oligomers"), (meth)acrylate-functionalized polyether oligomers (sometimes also referred to as "polyether (meth)acrylate oligomers"), (meth)acrylate-functionalized polydiene oligomers (sometimes also referred to as "polydiene (meth)acrylate oligomers"), (meth)acrylate-functionalized polycarbonate oligomers (sometimes also referred to as "polycarbonate (meth)acrylate oligomers"), and (meth)acrylate-functionalized polyester oligomers (sometimes also referred to as "polyester (meth)acrylate oligomers") and mixtures thereof.

Preferably, the (meth)acrylate-functionalized oligomer comprises a (meth)acrylate-functionalized urethane oligomer, more preferably an acrylate-functionalized urethane oligomer.

Advantageously, the (meth)acrylate-functionalized oligomer comprises a (meth)acrylate-functionalized urethane oligomer having two (meth)acrylate groups, more preferably an acrylate-functionalized urethane oligomer having two acrylate groups.

Exemplary polyester (meth)acrylate oligomers include the reaction products of acrylic or methacrylic acid or mixtures or synthetic equivalents thereof with hydroxyl group-terminated polyester polyols. The reaction process may be conducted such that all or essentially all of the hydroxyl groups of the polyester polyol have been (meth)acrylated, particularly in cases where the polyester polyol is difunctional. The polyester polyols can be made by poly condensation reactions of polyhydroxyl functional components (in particular, diols) and polycarboxylic acid functional compounds (in particular, dicarboxylic acids and anhydrides). The polyhydroxyl functional and polycarboxylic acid functional components can each have linear, branched, cycloaliphatic or aromatic structures and can be used individually or as mixtures.

Examples of suitable epoxy (meth)acrylates include the reaction products of acrylic or methacrylic acid or mixtures thereof with an epoxy resin (polyglycidyl ether or ester). The epoxy resin may, in particular, by selected from bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethyl-3',4'-epoxycyclohexanecarboxylate, 2-(3,4-epoxycyclohexyl-5,5-spiro-3,4-epoxy)cyclohexane-1,4-dioxane, bis(3,4-epoxycyclohexylmethyl)adipate, vinylcyclohexene oxide, 4-vinylepoxycyclohexane, bis(3,4-epoxy-6-methylcyclohexylmethyl)adipate,3,4-epoxy-6-methylcyclohexy 1-3',4'-epoxy-6'-methylcyclohexanecarboxylate, methylenebis(3,4-epoxycyclohexane), dicyclopentadiene diepoxide, di(3,4-epoxycyclohexylmethyl) ether of ethylene glycol, ethylenebis(3, 4-epoxycyclohexanecarboxylate), 1,4-butanediol diglycidyl ether, 1,6-hexanediol diglycidyl ether, glycerol triglycidyl ether, trimethylolpropane triglycidyl ether, polyethylene glycol diglycidyl ether, polypropylene glycol diglycidyl ether, polyglycidyl ethers of a polyether polyol obtained by the addition of one or more alkylene oxides to an aliphatic polyhydric alcohol such as ethylene glycol, propylene glycol, and glycerol, diglycidyl esters of aliphatic long-chain dibasic acids, monoglycidyl ethers of aliphatic higher alcohols, monoglycidyl ethers of phenol, cresol, butyl phenol, or polyether alcohols obtained by the addition of alkylene oxide to these compounds, glycidyl esters of higher fatty acids, epoxidized soybean oil, epoxybutylstearic acid, epoxyoctylstearic acid, epoxidized linseed oil, epoxidized polybutadiene, and the like.

Suitable polyether (meth)acrylate oligomers include, but are not limited to, the condensation reaction products of acrylic or methacrylic acid or synthetic equivalents or mixtures thereof with polyetherols which are polyether polyols (such as polyethylene glycol, polypropylene glycol or polytetramethylene glycol). Suitable polyetherols can be linear or branched substances containing ether bonds and terminal hydroxyl groups. Polyetherols can be prepared by ring opening polymerization of cyclic ethers such as tetrahydrofuran or alkylene oxides (e.g., ethylene oxide and/or propylene oxide) with a starter molecule. Suitable starter molecules include water, polyhydroxyl functional materials, polyester polyols and amines.

Polyurethane (meth)acrylate oligomers (sometimes also referred to as "urethane (meth)acrylate oligomers") suitable for use in Composition A include urethanes based on aliphatic, cycloaliphatic and/or aromatic polyester polyols and polyether polyols and aliphatic, cycloalipahtic and/or aromatic polyester diisocyanates and polyether diisocyanates capped with (meth)acrylate end-groups. Suitable polyurethane (meth)acrylate oligomers include, for example, aliphatic polyester-based urethane di- and tetra-acrylate oligomers, aliphatic polyether-based urethane di- and tetra-acrylate oligomers, as well as aliphatic polyester/polyether-based urethane di- and tetra-acrylate oligomers.

The polyurethane (meth)acrylate oligomers may be prepared by reacting aliphatic, cycloaliphatic and/or aromatic polyisocyanates (e.g., diisocyanate, triisocyanate) with OH group terminated polyester polyols, polyether polyols, polycarbonate polyols, polycaprolactone polyols, polyorganosiloxane polyols (e.g., polydimethylsiloxane polyols), or polydiene polyols (e.g., polybutadiene polyols), or combinations thereof to form isocyanate-functionalized oligomers which are then reacted with hydroxyl-functionalized (meth)acrylates such as hydroxyethyl acrylate or hydroxyethyl methacrylate to provide terminal (meth)acrylate groups. For example, the polyurethane (meth)acrylate oligomers may contain two, three, four or more (meth)acrylate functional groups per molecule. Other orders of addition may also be practiced to prepare the polyurethane (meth)acrylate, as is known in the art. For example, the hydroxyl-functionalized (meth)acrylate may be first reacted with a polyisocyanate to obtain an isocyanate-functionalized (meth)acrylate, which may then be reacted with an OH group terminated polyester polyol, polyether polyol, polycarbonate polyol, polycaprolactone polyol, polydimethylsiloxane polyol, polybutadiene polyol, or a combination thereof. In yet another embodiment, a polyisocyanate may be first reacted with a polyol, including any of the aforementioned types of polyols, to obtain an isocyanate-functionalized polyol, which is thereafter reacted with a hydroxyl-functionalized (meth)acrylate to yield a polyurethane (meth)acrylate. Alternatively, all the components may be combined and reacted at the same time.

Suitable acrylic (meth)acrylate oligomers (sometimes also referred to in the art as "acrylic oligomers") include oligomers which may be described as substances having an oligomeric acrylic backbone which is functionalized with one or (meth)acrylate groups (which may be at a terminus of the oligomer or pendant to the acrylic backbone). The acrylic backbone may be a homopolymer, random copolymer or block copolymer comprised of repeating units of acrylic monomers. The acrylic monomers may be any monomeric (meth)acrylate such as C1-C6 alkyl (meth)acrylates as well as functionalized (meth)acrylates such as (meth)acrylates bearing hydroxyl, carboxylic acid and/or epoxy groups. Acrylic (meth)acrylate oligomers may be prepared using any procedures known in the art, such as by oligomerizing monomers, at least a portion of which are functionalized with hydroxyl, carboxylic acid and/or epoxy groups (e.g., hydroxyalkyl(meth)acrylates, (meth)acrylic acid, glycidyl (meth)acrylate) to obtain a functionalized oligomer intermediate, which is then reacted with one or more (meth)acrylate-containing reactants to introduce the desired (meth)acrylate functional groups.

Composition A may comprise 0 to 99.5%, in particular 5 to 90%, more particularly 10 to 80%, even more particularly 15 to 75%, more particularly still 20 to 70% by weight of (meth)acrylate-functionalized oligomer based on the total weight of the composition. In particular, Composition A may comprise 5 to 50% or 10 to 50% or 15 to 50% or 20 to 50% or 25 to 50% or 30 to 50%, by weight of (meth)acrylate-functionalized oligomer based on the total weight of the composition. Alternatively, Composition A may comprise 50 to 99.5% or 55 to 99.5% or 60 to 99.5% or 65 to 99.5% or 70 to 99.5%, by weight of (meth)acrylate-functionalized oligomer based on the total weight of the composition.

In one embodiment, the ethylenically unsaturated compound comprises an amine-modified acrylate. The ethylenically unsaturated compound may comprise a mixture of amine-modified acrylates.

An amine-modified acrylate is obtained by reacting an acrylate-functionalized compound with an amine-containing compound (aza-Michael addition). The amine-modified acrylate comprises at least one remaining acrylate group (i.e. an acrylate group that has not reacted with the amine-containing compound during the aza-Michael addition) and/or at least one (meth)acrylate group (which may not be reactive towards primary or secondary amines).

The acrylate-functionalized compound may be an acrylate-functionalized monomer and/or acrylate-functionalized oligomer as defined above.

The amine-containing compound comprises a primary or secondary amino group and optionally a tertiary amino group. The amine-containing compound may comprise more than one primary and/or secondary amino groups. The amine-containing compound may be selected from monoethanolamine (2-aminoethanol), 2-ethylhexylamine, octylamine, cyclohexylamine, sec-butylamine, isopropylamine, diethylamine, diethanolamine, dipropylamine, dibutylamine, 2-(methylamino)ethano-1,2-methoxyethylamine, bis(2-hydroxypropyl)amine, diisopropylamine, dipentylamine, dihexylamine, bis(2-ethylhexyl)amine, 1,2,3,4-tetrahydroisoquinoline, N-benzylmethylamine, morpholine, piperidine, dioctylamine, and di-cocoamine, dimethylaminopropylamine, dimethylaminopropylaminopropylamine, 1,4-bis(3-aminopropyl)piperazine, 1,3-bis(aminomethyl)cyclohexane, 1,4-bis(3-aminopropyl)piperazine, aniline and an optionally substituted benzocaine (ethyl-4-aminobenzoate).

Examples of commercially available amine-modified acrylates include CN3705, CN3715, CN3755, CN381 and CN386, all available from Arkema. Polymeric or multi-amino versions are also suitable.

Composition A may comprise from 0% to 25%, in particular 2.5% to 20%, more particularly 5 to 15%, by weight of amine-modified acrylate based on the total weight of the composition.

In a first embodiment, the ethylenically unsaturated compound b) consists essentially of one or more (meth)acrylate-functionalized monomers as defined above and optionally one or more (meth)acrylate-functionalized oligomers as defined above.

In a second embodiment, the ethylenically unsaturated compound b) consists essentially of one or more (meth)acrylate-functionalized monomers as defined above and optionally one or more amine-modified acrylates as defined above.

In a third embodiment, the ethylenically unsaturated compound b) consists essentially of a mixture of one or more (meth)acrylate-functionalized monomers as defined above and one or more (meth)acrylate-functionalized oligomers as defined above.

In a fourth embodiment, the ethylenically unsaturated compound b) consists essentially of one or more (meth)acrylate-functionalized oligomers as defined above and optionally one or more (meth)acrylate-functionalized monomers as defined above.

In a fifth embodiment, the ethylenically unsaturated compound b) consists essentially of one or more (meth)acrylate-functionalized oligomers as defined above and optionally one or more amine-modified acrylates as defined above.

In a sixth embodiment, the ethylenically unsaturated compound b) consists essentially of a mixture of one or more (meth)acrylate-functionalized monomers as defined above and one or more (meth)acrylate-functionalized oligomers as defined above and one or more amine-modified acrylates as defined above.

### Composition B comprising an unsaturated polymer dispersion or emulsion

The invention further relates to a curable composition comprising a compound of formula (I) as defined above or a photoinitiator composition as defined above, referred to as component a), and further comprising an unsaturated polymer dispersion or emulsion, referred to as component c). This embodiment is referred to as Composition B.

Composition B may comprise from 0.05% to 30%, from 0.1% to 20%, from 0.2 to 15%, from 0.5 to 10% or from 1 to 5%, by weight of compound of formula (I) based on the total weight of the composition.

Composition B may comprise from 0.05% to 30%, from 0.1% to 20%, from 0.2 to 15%, from 0.5 to 10% or from 1 to 5%, by weight of photoinitiator other than a compound of formula (I) based on the total weight of the ethylenically unsaturated polymer.

Composition B may comprise:
- from 0.05 to 30%, from 0.1 to 20%, from 0.2 to 15%, from 0.5 to 10% or from 1 to 5% of component a);
- from 70 to 99,95%, from 80 to 99.9%, from 85 to 99.8%, from 90 to 99.5% or from 95 to 99% of component c);
the % being % by weight based on the total weight of components a) and c).

As used herein the term "unsaturated polymer dispersion or emulsion" means a dispersion or an emulsion comprising an ethylenically unsaturated polymer.

The unsaturated polymer dispersion or emulsion may be an aqueous polymer dispersion or emulsion. In one embodiment, the unsaturated polymer dispersion is a polyurethane dispersion (PUD), an acrylic dispersion and mixtures thereof, in particular a PUD. In one embodiment, the unsaturated polymer emulsion is an unsaturated polyester emulsion. In one embodiment, the unsaturated polymer emulsion is an emulsion of a (meth)acrylate-functionalized oligomer as defined above, in particular an emulsion of a (meth)acrylate-functionalized oligomer selected from a (meth)acrylate-functionalized urethane oligomer, a (meth)acrylate-functionalized epoxy oligomer, a (meth)acrylate-functionalized polyether oligomer, a (meth)acrylate-functionalized polydiene oligomer, a (meth)acrylate-functionalized polycarbonate oligomer, a (meth)acrylate-functionalized polyester oligomer and mixtures thereof.

As used herein, the term "polyurethane dispersion" means a dispersion comprising a polyurethane polymer or a polyurethane copolymer such as a polyurethane-acrylate copolymer.

Polyurethane dispersions are typically produced by first forming a polyurethane pre-polymer, which comprises terminal groups, such as isocyanate (NCO) groups, which can undergo subsequent chain extension reactions. The polyurethane pre-polymer may be diluted with an organic solvent or a reactive diluent. The dilution advantageously reduces the viscosity of the prepolymer to facilitate its subsequent dispersion in water. The pre-polymer can undergo a chain-extension reaction prior to or after dispersion in water to increase the length of the polymer chain and/or add additional functionality to the polyurethane.

In one embodiment, the polyurethane dispersion comprises a polyurethane polymer and optionally a reactive diluent. The polyurethane polymer may bear ethylenically unsaturated groups. The polyurethane polymer may be formed by chain extending at least one isocyanate-terminated ethylenically unsaturated polyurethane pre-polymer (P), with a chain extender (E) bearing at least 2 isocyanate-reactive groups.

Pre-polymer (P) may be formed by reacting:
(i) one or more isocyanate reactive components containing at least one ethylenic unsaturation, preferably chosen from active hydrogen-containing (meth)acrylates ;
(ii) one or more polyisocyanates, preferably diisocyanates ;
(iii) one or more isocyanate reactive components containing ionic groups, potentially ionic groups or hydrophilic ether groups, preferably ionic groups derived from acidic groups, in particular with said component (iii) bearing two isocyanate reactive groups, more particularly bearing two OH groups ; and
(iv) optionally, one or more isocyanate reactive components other than component (i) or component (iii), preferably bearing two isocyanate reactive groups, in particular OH, such that the mole ratios of components (i), (ii), (iii) and (iv) result in a polyurethane pre-polymer comprising terminal isocyanate groups.

Component (i) may be a polyol component comprising (i-1) a monoalcohol bearing at least 1, preferably from 1 to 5 (meth)acrylate groups and optionally (i-2) a diol bearing at least 1, preferably from 1 to 4 (meth)acrylate groups. Component (i) containing ethylenic unsaturation may be chosen from polyester (meth)acrylates, epoxy (meth)acrylates, polyether (meth)acrylates, urethane (meth)acrylates, hydroxy-bearing (meth)acrylates (such as, for example partial (meth)acrylic esters of alkylene polyols which may be alkoxylated or partial (meth)acrylic esters of polyols resulting from di-polyol ethers of said alkylene polyols) and combinations thereof.

Component (iii) may be a diol bearing an acidic group and salts thereof. The acidic group may be chosen from a carboxylic acid (-COOH) group, a carboxylate (-COO⁻) group, a phosphonic acid (-P(=O)(OH)₂) group, a phosphonate (-P(=O)(OR)₂) group, a sulfonic acid (-S(=O)₂OH) group, a sulfonate (-S(=O)₂OR) group, a phosphate (-O-P(=O)(OR)₂) group, wherein each R is independently a counterion, a hydrogen atom, or an optionally substituted hydrocarbyl.

Component (iv) may be a monomeric or oligomeric monoalcohol or polyol, preferably a diol. More particularly, component (iv) is oligomeric such as polyether or polyester diol.

Chain extender (E) may be selected from polyols and/or polyamines, more preferably from diols and/or diamines, even more preferably from diamines.

The reactive diluent (D) may be a compound having one or more ethylenically unsaturated groups that may be used as a diluent in the preparation of the polyurethane pre-polymer and to dilute the polyurethane pre-polymer during the formation of the polyurethane dispersion. It may react by free radical reaction with the acrylate groups on dispersed polyurethane during the actinic radiation curing step. The reactive diluent, when added to the polyurethane pre-polymer, can be used to control the viscosity of the polyurethane pre-polymer. In particular, the reactive diluent (D) may be mono(meth)acrylate-functionalized monomer as described above for composition A. Reactive diluent (D) may represent from about 10% to about 90%, preferably from 10 to 50% by weight relative to the solids content of the dispersion.

In another embodiment, the polyurethane dispersion may be a dispersion comprising a polyurethane-acrylate copolymer (also referred to as a PUA dispersion). The polyurethane-acrylate copolymer may be obtained by reacting the isocyanate-terminated ethylenically unsaturated polyurethane pre-polymer (P) as defined above with (meth)acrylate monomers, such as esters of (meth)acrylic acid with mono alcohols having 1 to 20, preferably 4 to 12 carbon atoms.

As used herein, the term "acrylic dispersion" means a dispersion comprising an acrylic polymer or an acrylic copolymer. The acrylic polymer or copolymer comprises residual ethylenically unsaturations.

As used herein, the term "unsaturated polyester emulsion" means an emulsion comprising an unsaturated polyester.

As used herein, the term "emulsion of a (meth)acrylate-functionalized oligomer" means an emulsion comprising a (meth)acrylate-functionalized oligomer.

The emulsion of a (meth)acrylate-functionalized oligomer may be obtained by emulsifying one or more (meth)acrylate-functionalized oligomers in water with a surfactant, in particular a polymeric amphiphilic surfactant (protective colloid).

### Common features between Compositions A and B

Composition A and Composition B as described above may further comprise one or more compounds selected from:
- an amine synergist;
- a cationically polymerizable compound;
- an additive;
- a solvent.

### Amine synergist

The polymerizable composition of the present invention may comprise an amine synergist. The polymerizable composition may comprise a mixture of amine synergists.

The amine synergist may be as defined above for the photoinitiator composition.

### Cationically polymerizable compound

The composition of the invention may further comprise a cationically polymerizable compound. The composition of the invention may comprise a mixture of cationically polymerizable compounds.

When the composition comprises a cationically polymerizable compound, the composition may be a hybrid free-radical/cationic composition, i.e. a composition that is cure by free radical polymerization and cationic polymerization.

The term "cationically polymerizable compound" means a compound comprising a polymerizing functional group which polymerizes via a cationic mechanism, for example a heterocyclic group or a carbon-carbon double bond substituted with an electrodonating group. In a cationic polymerization mechanism, a cationic initiator transfers charge to the cationically polymerizable compound which then becomes reactive and leads to chain growth by reaction with another cationically polymerizable compound.

The cationically polymerizable compound may be selected from epoxy-functionalized compounds, oxetanes, oxolanes, cyclic acetals, cyclic lactones, thiiranes, thiethanes, spiro orthoesters, ethylenically unsaturated compounds other than (meth)acrylates, derivatives thereof and mixtures thereof.

In a preferred embodiment, the cationically polymerizable compound may be selected from epoxy-functionalized compounds, oxetanes and mixtures thereof, in particular aromatic epoxy-functionalized compounds, cycloaliphatic epoxy-functionalized compounds, oxetanes and mixtures thereof.

Suitable epoxy-functionalized compounds capable of being cationically polymerized are glycidyl ethers, in particular mono-, di-, tri- and polyglycidyl ether compounds, and alicyclic ether compounds including those comprising residue of carboxylic acids such as, for example, alkylcarboxylic acid residual groups, alkylcycloalkylcarboxylic acid residual groups and dialkyl dicarboxylic acid residual groups. For example, the epoxy-functionalized compounds may be bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethyl-3',4'-epoxycyclohexanecarboxylate, 2-(3,4-epoxycyclohexyl-5,5-spiro-3,4-epoxy)cyclohexane-1,4-dioxane, bis(3,4-epoxycyclohexylmethyl)adipate, vinylcyclohexene oxide, 4-vinylepoxycyclohexane, bis(3,4-epoxy-6-methylcyclohexylmethyl)adipate, 3,4-epoxy-6-methylcyclohexyl-3',4'-epoxy-6'-methylcyclohexanecarboxylate, methylenebis(3,4-epoxycyclohexane), dicyclopentadiene diepoxide, di(3,4-epoxycyclohexylmethyl) ether of ethylene glycol, ethylenebis(3, 4-epoxycyclohexanecarboxylate), epoxyhexahydrodioctylphthalate, epoxyhexahydro-di-2-ethylhexyl phthalate, 1,4-butanediol diglycidyl ether, 1,6-hexanediol diglycidyl ether, glycerol triglycidyl ether, trimethylolpropane triglycidyl ether, polyethylene glycol diglycidyl ether, polypropylene glycol diglycidyl ether, polyglycidyl ethers of polyether polyol obtained by the addition of one or more alkylene oxides to aliphatic polyhydric alcohols such as ethylene glycol, propylene glycol, and glycerol, diglycidyl esters of aliphatic long-chain dibasic acids, monoglycidyl ethers of aliphatic higher alcohols, monoglycidyl ethers of phenol, cresol, butyl phenol, or polyether alcohols obtained by the addition of alkylene oxide to these compounds, glycidyl esters of higher fatty acids, epoxidized soybean oil, epoxybutylstearic acid, epoxyoctylstearic acid, epoxidized linseed oil, epoxidized polybutadiene, and the like.

Suitable oxetanes capable of being cationically polymerized include trimethylene oxide, 3,3-dimethyloxetane, 3,3-dichloromethyloxetane, 3-ethyl-3-phenoxymethyloxetane, and bis(3-ethyl-3-methyloxy)butane, 3-ethyl-3-oxetanemethanol.

Suitable oxolanes capable of being cationically polymerized include tetrahydrofuran and 2,3-dimethyltetrahydrofuran.

Suitable cyclic acetals capable of being cationically polymerized include trioxane, 1,3-dioxolane, and 1,3,6-trioxanecyclooctane.

Suitable cyclic lactones capable of being cationically polymerized include β-propiolactone and ε-caprolactone.

Suitable thiiranes capable of being cationically polymerized include ethylene sulfide, 1,2-propylene sulfide, and thioepichlorohydrin.

Suitable thiethanes capable of being cationically polymerized include 3,3-dimethylthiethane.

Suitable spiro orthoesters capable of being cationically polymerized are compounds obtained by the reaction of an epoxy compound and a lactone.

Suitable ethylenically unsaturated compounds other than (meth)acrylates capable of being cationically polymerized include vinyl ethers such as ethylene glycol divinyl ether, triethylene glycol divinyl ether and trimethylolpropane trivinyl ether; aliphatic vinyl monomers such as vinylcyclohexane; olefins such isobutylene; dienes such as butadiene; vinyl alkyl ethers; vinyl aromatic monomers such as styrene and alkylstyrenes; unsaturated polymers such as polybutadiene; derivatives of the above organic substances; and the like, at least some of which may also be polymerizable by free radical mechanisms.

When the composition of the invention comprises a cationically polymerizable compound, it may further comprise a polyol. Suitable polyols are as defined above.

The curable composition of the invention may comprise 0 to 50%, in particular 5 to 40%, more particularly 10 to 30%, by weight of cationically polymerizable compound based on the total weight of the curable composition.

### Additives

The curable composition of the present invention may comprise an additive. The curable composition may comprise a mixture of additives.

In particular, the additive may be selected from sensitizers, stabilizers (antioxidants, light blockers/absorbers, polymerization inhibitors), foam inhibitors, flow or leveling agents, colorants, pigments, dispersants (wetting agents, surfactants), slip additives, fillers, chain transfer agents, thixotropic agents, matting agents, impact modifiers, waxes, mixtures thereof, and any other additive conventionally used in the coating, sealant, adhesive, molding, 3D printing or ink arts.

The curable composition may comprise a sensitizer.

Sensitizers may be introduced in the curable composition of the present invention in order to extend the sensitivity of the photoinitiator to longer wavelengths. For example, the sensitizer may absorb light at longer or shorter wavelengths than the photoinitiator and be capable of transferring the energy to the photoinitiator and revert to its ground state.

Examples of suitable sensitizers include anthracenes and carbazoles.

The concentration of sensitizer in the curable composition will vary depending on the photoinitiator that is used. Typically, however, the curable composition is formulated to comprise from 0% to 5%, in particular 0.1% to 3%, more particularly 0.5 to 2%, by weight of sensitizer based on the total weight of the curable composition.

The curable composition may comprise a stabilizer.

Stabilizers may be introduced in the curable composition of the present invention in order to provide adequate storage stability, processing stability, and shelf life. Advantageously, one or more such stabilizers are present at each stage of the method used to prepare the curable composition, to protect against unwanted reactions during processing of the ethylenically unsaturated components of the curable composition. As used herein, the term "stabilizer" means a compound or substance which retards or prevents reaction or curing of actinically-curable functional groups present in a composition in the absence of actinic radiation. However, it will be advantageous to select an amount and type of stabilizer such that the composition remains capable of being cured when exposed to actinic radiation (that is, the stabilizer does not prevent radiation curing of the composition). Typically, effective stabilizers for purposes of the present invention will be classified as free radical stabilizers (i.e., stabilizers which function by inhibiting free radical reactions).

Any of the stabilizers known in the art related to (meth)acrylate-functionalized compounds may be utilized in the present invention. Quinones represent a particularly preferred type of stabilizer which can be employed in the context of the present invention. As used herein, the term "quinone" includes both quinones and hydroquinones as well as ethers thereof such as monoalkyl, monoaryl, monoaralkyl and bis(hydroxyalkyl) ethers of hydroquinones. Hydroquinone monomethyl ether is an example of a suitable stabilizer which can be utilized. Other stabilizers known in the art such as hydroquinone (HQ), 4-tert-butylcatechol (TBC), 3,5-di-tertiobutyl-4-hydroxytoluene (BHT), phenothiazine (PTZ), pyrogallol, phosphite compounds, triphenyl antimony and tin(II) salts can also be used.

The concentration of stabilizer in the curable composition will vary depending upon the particular stabilizer or combination of stabilizers selected for use and also on the degree of stabilization desired and the susceptibility of components in the curable compositions towards degradation in the absence of stabilizer. Typically, however, the curable composition is formulated to comprise from 5 to 5000 ppm stabilizer. According to certain embodiments of the invention, the reaction mixture during each stage of the method employed to make the curable composition contains at least some stabilizer, e.g., at least 10 ppm stabilizer.

The polymerizable composition may comprise a colorant. A colorant may be a dye, a pigment and mixtures thereof. The term "dye", as used herein means a colorant having a solubility of 10 mg/L or more in the medium in which it is introduced at 25°C. The term "pigment" is defined in DIN 55943, as a colorant that is practically insoluble in the application medium under the pertaining ambient conditions, hence having a solubility of less than 10 mg/L therein at 25°C. The term "C.I." is used as an abbreviation for Colour Index.

The colorant may be a pigment. Organic and/or inorganic pigments may be used. If the colorant is not a self-dispersible pigment, the inkjet inks preferably also contain a dispersant, more preferably a polymeric dispersant. The pigment may be black, cyan, magenta, yellow, red, orange, violet, blue, green, brown and mixtures thereof. Pigments may be chosen from those disclosed by HERBST, Willy, et al. Industrial Organic Pigments, Production, Properties, Applications. 3rd edition. Wiley - VCH, 2004. ISBN 3527305769.

The polymerizable composition of the invention may comprise a dispersant. The dispersant may be used to disperse an insoluble material such as a pigment or filler in the polymerizable composition.

The dispersant may be a polymeric dispersant, a surfactant and mixtures thereof.

Typical polymeric dispersants are copolymers of two, three, four, five or even more monomers. The properties of polymeric dispersants depend on both the nature of the monomers and their distribution in the polymer. Copolymeric dispersants preferably have the following polymer compositions:
- random copolymer (e.g. ABBAABAB);
- alternating copolymer (e.g. ABABABAB);
- gradient copolymer (e.g. AAABAABBABBB) ;
- block copolymers (e.g. AAAAABBBBBB);
- graft copolymers (polymeric backbone with polymeric side chains attached to the backbone); and mixed forms of these copolymers.

The polymeric dispersant may have a number average molecular weight Mn between 500 and 30000, more preferably between 1500 and 10000.

Commercial examples of polymeric dispersants include:
- DISPERBYK^{®} dispersants available from BYK CHEMIE GMBH;
- SOLSPERSE^{®} dispersants available from LUBRIZOL;
- TEGO^{®} DISPERSE dispersants from EVONIK;
- DISPEX^{®}, EFKA^{®} and JONCRYL^{®} dispersants from BASF;
- DISPONER^{®} dispersants from ELEMENTIS.

### Solvent

The curable composition of the invention may be solvent-based or water-based. As used herein, the term "solvent" means a non-reactive organic solvent, i.e. a solvent comprising carbon and hydrogen atom that does not react when exposed to the actinic radiation used to cure the curable compositions described herein.

Advantageously, the curable compositions of the present invention may be formulated to be solvent-free, i.e., free of any non-reactive volatile substances (substances having a boiling point at atmospheric pressure of 150°C or less). For example, the curable compositions of the present invention may contain little or no non-reactive solvent, e.g., less than 10% or less than 5% or less than 1% or even 0% by weight of non-reactive solvent, based on the total weight of the curable composition. As used herein, the term non-reactive solvent means a solvent that does not react when exposed to the actinic radiation used to cure the curable compositions described herein.

In one embodiment, the curable composition of the present invention may be water-based. In particular the curable composition of the invention may be a water-based polyurethane

### Formulations

The curable composition of the invention may be formulated as a one component or one part system. That is, the curable composition may be cured directly, i.e. it is not combined with another component or second part prior to being cured.

In a first embodiment, the curable composition of the invention may comprise:
a) a compound of formula (I) or a photoinitiator composition as defined above;
b) an ethylenically unsaturated compound or an unsaturated polymer dispersion or emulsion;
c) optionally an amine synergist;
d) optionally a cationically polymerizable compound;
e) optionally an additive;
f) optionally a solvent.

Preferably, the composition of the first embodiment does not comprise any component other than components a)-f).

In preferred embodiments of the invention, the curable composition is a liquid at 25°C. In various embodiments of the invention, the curable compositions described herein are formulated to have a viscosity of less than 10,000 mPa.s, or less than 5,000 mPa.s, or less than 1,000 mPa.s, or less than 500 mPa.s, or less than 250 mPa.s, or even less than 100 mPa.s as measured at 25°C using a Brookfield viscometer, model DV-II, using a 27 spindle (with the spindle speed varying typically between 20 and 200 rpm, depending on viscosity). In advantageous embodiments of the invention, the viscosity of the curable composition is from 10 to 10,000 mPa.s, or from 10 to 5,000 mPa.s, or from 10 to 1,000 mPa.s, or from 10 to 500 mPa.s, or from 10 to 250 mPa.s, or from 10 to 100 mPa.s at 25°C.

The curable compositions described herein may be compositions that are to be subjected to curing by means of free radical polymerization. In particular embodiments, the curable compositions may be photocured (i.e., cured by exposure to actinic radiation such as light, in particular visible or UV light). When the compound of formula (I) is a thioxanthone, the composition may be cured by a LED light source.

The curable composition of the invention may be an ink composition, an overprint varnish composition, a coating composition, an adhesive composition, a sealant composition, a molding composition, a dental composition, a cosmetic composition or a 3D-printing composition, in particular an ink composition

End use applications for the curable compositions include, but are not limited to, inks, coatings, adhesives, additive manufacturing resins (such as 3D printing resins), molding resins, sealants, composites, antistatic layers, electronic applications, recyclable materials, smart materials capable of detecting and responding to stimuli, packaging materials, personal care articles, cosmetics, articles for use in agriculture, water or food processing, or animal husbandry, and biomedical materials. The curable compositions of the invention thus find utility in the production of biocompatible articles. Such articles may, for example, exhibit high biocompatibility, low cytotoxicity and/or low extractables.

The composition according to the invention may in particular be used to obtain a cured product and a 3D printed article according to the following processes.

### Process for the preparation of a cured product and a 3D-printed article

The process for the preparation of a cured product according to the invention comprises curing the composition of the invention. In particular, the composition may be cured by exposing the composition to radiation. More particularly, the composition may be cured by exposing the composition to UV, near-UV and/or visible radiation. When the compound of formula (I) is a thioxanthone, the composition may be cured by exposing the composition to a LED light source, in particular a LED light source having an emission band in the range of 365-420 nm.

Curing may be accelerated or facilitated by supplying energy to the curable composition, such as by heating the curable composition. Thus, the cured product may be deemed as the reaction product of the curable composition, formed by curing. A curable composition may be partially cured by exposure to actinic radiation, with further curing being achieved by heating the partially cured article. For example, a product formed from the curable composition (e.g., a 3D printed article) may be heated at a temperature of from 40°C to 120°C for a period of time of from 5 minutes to 12 hours.

Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used.

The substrate on which the curable composition is applied and cured may be any kind of substrate. Suitable substrates are detailed below. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable composition may be cast into a suitable mold and then cured).

The cured product obtained with the process of the invention may be an ink, an overprint varnish, a coating, an adhesive, a sealant, a molded article, a dental material, a cosmetic product or a 3D-printed article.

The 3D-printed article may, in particular, be obtained with a process for the preparation of a 3D-printed article that comprises printing a 3D article with the composition of the invention. In particular, the process may comprise printing a 3D article layer by layer or continuously.

A plurality of layers of a curable composition in accordance with the present invention may be applied to a substrate surface; the plurality of layers may be simultaneously cured (by exposure to a single dose of radiation, for example) or each layer may be successively cured before application of an additional layer of the curable composition.

The curable compositions which are described herein can be used as resins in three-dimensional printing applications. Three-dimensional (3D) printing (also referred to as additive manufacturing) is a process in which a 3D digital model is manufactured by the accretion of construction material. The 3D printed object is created by utilizing the computer-aided design (CAD) data of an object through sequential construction of two dimensional (2D) layers or slices that correspond to cross-sections of 3D objects. Stereolithography (SL) is one type of additive manufacturing where a liquid resin is hardened by selective exposure to a radiation to form each 2D layer. The radiation can be in the form of electromagnetic waves or an electron beam. The most commonly applied energy source is ultraviolet, visible or infrared radiation.

Sterolithography and other photocurable 3D printing methods typically apply low intensity light sources to radiate each layer of a photocurable resin to form the desired article. As a result, photocurable resin polymerization kinetics and the green strength of the printed article are important criteria if a particular photocurable resin will sufficiently polymerize (cure) when irradiated and have sufficient green strength to retain its integrity through the 3D printing process and post-processing.

The curable compositions of the invention are especially useful as 3D printing resin formulations, that is, compositions intended for use in manufacturing three-dimensional articles using 3D printing techniques. Such three-dimensional articles may be freestanding/self-supporting and may consist essentially of or consist of a composition in accordance with the present invention that has been cured. The three-dimensional article may also be a composite, comprising at least one component consisting essentially of or consisting of a cured composition as previously mentioned as well as at least one additional component comprised of one or more materials other than such a cured composition (for example, a metal component or a thermoplastic component or inorganic filler or fibrous reinforcement). The curable compositions of the present invention are particularly useful in digital light printing (DLP), although other types of three-dimensional (3D) printing methods may also be practiced using the inventive curable compositions (e.g., SLA, inkjet, multi-jet printing, piezoelectric printing, actinically-cured extrusion, and gel deposition printing). The curable compositions of the present invention may be used in a three-dimensional printing operation together with another material which functions as a scaffold or support for the article formed from the curable composition of the present invention.

Thus, the curable compositions of the present invention are useful in the practice of various types of three-dimensional fabrication or printing techniques, including methods in which construction of a three-dimensional object is performed in a step-wise or layer-by-layer manner. In such methods, layer formation may be performed by solidification (curing) of the curable composition under the action of exposure to radiation, such as visible, UV or other actinic irradiation. For example, new layers may be formed at the top surface of the growing object or at the bottom surface of the growing object. The curable compositions of the present invention may also be advantageously employed in methods for the production of three-dimensional objects by additive manufacturing wherein the method is carried out continuously. For example, the object may be produced from a liquid interface. Suitable methods of this type are sometimes referred to in the art as "continuous liquid interface (or interphase) product (or printing)" ("CLIP") methods. Such methods are described, for example, in WO 2014/126830; WO 2014/126834; WO 2014/126837; and Tumbleston et al., "Continuous Liquid Interface Production of 3D Objects," Science Vol. 347, Issue 6228, pp. 1349-1352 (March 20, 2015), the entire disclosure of which is incorporated herein by reference in its entirety for all purposes.

The curable composition may be supplied by ejecting it from a printhead rather than supplying it from a vat. This type of process is commonly referred to as inkjet or multijet 3D printing. One or more UV curing sources mounted just behind the inkjet printhead cures the curable composition immediately after it is applied to the build surface substrate or to previously applied layers. Two or more printheads can be used in the process which allows application of different compositions to different areas of each layer. For example, compositions of different colors or different physical properties can be simultaneously applied to create 3D printed parts of varying composition. In a common usage, support materials - which are later removed during post-processing - are deposited at the same time as the compositions used to create the desired 3D printed part. The printheads can operate at temperatures from about 25°C up to about 100°C. Viscosities of the curable compositions are less than 30 mPa.s at the operating temperature of the printhead.

The process for the preparation of a 3D-printed article may comprise the steps of:
a) providing (e.g., coating) a first layer of a curable composition in accordance with the present invention onto a surface;
b) curing the first layer, at least partially, to provide a cured first layer;
c) providing (e.g., coating) a second layer of the curable composition onto the cured first layer;
d) curing the second layer, at least partially, to provide a cured second layer adhered to the cured first layer; and
e) repeating steps c) and d) a desired number of times to build up the three-dimensional article.

After the 3D article has been printed, it may be subjected to one or more post-processing steps. The post-processing steps can be selected from one or more of the following steps removal of any printed support structures, washing with water and/or organic solvents to remove residual resins, and post-curing using thermal treatment and/or actinic radiation either simultaneously or sequentially. The post-processing steps may be used to transform the freshly printed article into a finished, functional article ready to be used in its intended application.

### Process of inkjet printing

The process of inkjet printing according to the invention comprised jetting the polymerizable composition of the invention onto a substrate.

The substrate on which the polymerizable composition is jetted may be any kind of substrate as defined above.

The polymerizable composition may be jetted by one or more print heads ejecting small droplets in a controlled manner through nozzles onto a substrate moving relative to the print head(s).

The print head may be a piezoelectric head or a continuous type print head.

The inkjet printing process may be carried out in a single pass or with a multi-pass printing mode.

The inkjet printing process may further comprise a UV-curing step. In inkjet printing, the UV curing device may be arranged in combination with the print head of the inkjet printer, travelling therewith so that the liquid UV curable inkjet ink is exposed to curing radiation very shortly after been jetted.

In a particularly preferred embodiment, the UV curing step is performed using UV LED light sources.

For facilitating curing, the inkjet printer may include one or more oxygen depletion units. The oxygen depletion units place a blanket of nitrogen or other relatively inert gas (e.g. CO₂), with adjustable position and adjustable inert gas concentration, in order to reduce the oxygen concentration in the curing environment.

### Substrate

The substrate on which the binder or the polymerizable composition of the invention is applied may be any kind of substrate.

The substrate may be a ceramic, metallic, mineral, cellulosic, animal-based or polymeric substrate. The substrate may also be a part of a human body, such as a tooth or a nail.

The substrate may be porous or substantially non-porous. The substrates may be transparent, translucent or opaque.

Examples of ceramic substrates include alumina-based ceramics and zirconia-based ceramics.

Examples of metallic substrates include titanium, gold, silver, copper, brass, steel and bronze.

Examples of mineral substates include glass, asbestos and basalt.

Examples of cellulosic substrates include plain paper or resin coated paper (e.g. polyethylene or polypropylene coated paper). There is no real limitation on the type of paper and it includes newsprint paper, magazine paper, office paper, wallpaper but also paper of higher grammage, usually referred to as boards, such as white lined chipboard, corrugated board and packaging board. Further examples of cellulosic substrates include bamboo, cotton, flax, hemp, jute, lyocell, modal, rayon, raffia, ramie and sisal.

Examples of cellulosic substrates include wool, fur, silk and leather.

Examples of polymeric substrates include polyethylene, polypropylene, polycarbonate, polyvinyl chloride, polyethylene terephthalate, polyethylene naphthalate, polylactide, polyamide, polyimide, polyacrylonitrile, polyurethane, acrylonitrile butadiene styrene.

There is no restriction on the shape of the substrate. It can be a sheet, a film, a non-woven or woven fiber mat or a three dimensional object.

In particular, the substrate may be selected from a food and beverage packaging, a pharmaceutical packaging, a textile, a nail, a tooth, a medical device, a food and beverage processing equipment, a water pipe.

### Uses

The compound of formula (I) as defined above or the photoinitiator composition as defined above may be used as a photoinitiating system in a radiation curable composition, in particular in a UV or LED-curable composition.

As used herein, "UV curable composition" means a composition that is at least partially cured by exposure to UV light emitted by a mercury light source, in particular a mercury-vapor lamp, and "LED-curable composition" means a composition that is at least partially cured by exposure to UV light emitted by a LED light source, in particular a LED light source having an emission band in the range of 365-420 nm.

Other curing methods may be combined with UV light, such as heating,

The compound of formula (I) as defined above or the photoinitiator composition as defined above may be used in a photopolymerization reaction. The photopolymerization reaction may be used to photopolymerize (e.g. cure) one or more ethylenically unsaturated compounds as defined above.

The compound of formula (I) as defined above or the photoinitiator composition as defined above may be used in a curable composition as defined above. In particular, the curable composition may be an ink composition, an overprint varnish composition, a coating composition, an adhesive composition, a sealant composition, a molding composition, a dental composition, a cosmetic composition or a 3D-printing composition.

The curable composition of the invention may be used to obtain a cured product. In particular, the cured product may be an ink, an overprint varnish, a coating, an adhesive, a sealant, a molded article, a cosmetic product or a 3D-printed article.

When the curable composition comprises a compound of formula (I) wherein at least part of the M groups are (meth)acryloyl, the cured product obtained therefrom may exhibit a reduced amount of extractables since at least part of the compound of formula (I) is chemically bound to the cured polymeric matrix.

The invention thus also relates to the use of a compound of formula (I) wherein at least part of the M groups are (meth)acryloyl, to obtain a cured product having a reduced amount of extractables. The reduction in the amount of extractables may be assessed in comparison with a cured product obtained with a conventional photoinitiator (i.e. a non-polymerizable photoinitiator or a polymeric photoinitiator).

The extractables may be any component that migrates from the cured product. In particular, the extractables may be a photoinitiator or a residue thereof.

Migration in inks may occur in different ways:
Penetration Migration - through the substrate to the reverse-side of the print;
Set-off Migration - from the printed side of a substrate to the reverse side of the substrate while stacked or stored on a roll;
Vapor-phase migration - evaporation of volatile compounds when heated;
Condensation Extraction - condensation of critical compounds when cooked or sterilized.

The amount of extractables may be determined quantitatively using a suitable analytical method such as liquid chromatography mass spectroscopy (LC-MS). For example, the curable composition can be applied in 12 µm thickness film on a glass substrate, and crosslinked using a UV Hg lamp. Resulting cured films are removed from the glass plate, weighed and soaked in solvent such as acetonitrile or dichloromethane. The liquid fraction is finally evaporated and the residue, corresponding to the extractables part, is weighed, allowing to determine the amount of product that is uncured (not trapped within the photocured network).

Analytical methods such as nuclear magnetic resonance (NMR), liquid chromatography mass spectroscopy (LC-MS) or gas chromatography mass spectroscopy (GC-MS) may then be used to identify the nature of the extractables and refine their corresponding content.

In particular, the cured product may have less than 5%, less than 2%, less than 1%, less than 0.5%, less than 0.25% or less than 0,1%, by weight of extractables based on the weight of the cured product.

### Aspects of the invention

The invention may be as described in the following aspects:
Aspect 1. A compound according to the following formula (I): 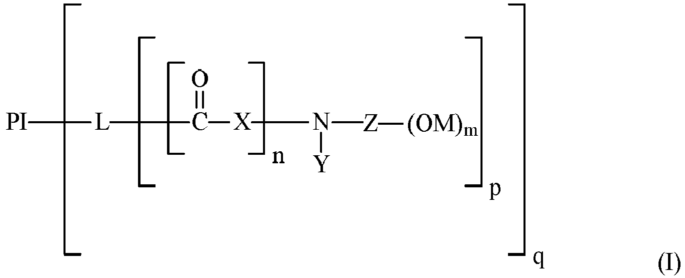 wherein
   PI is a photoinitiator moiety;
   each L is independently selected from a direct bond or a linker comprising 1 to 20 carbon atoms;
   each X is independently selected from a direct bond, a -P(=O)(R')- bond and a linker comprising 1 to 6, in particular 1 to 4, more particularly 1 to 3, carbon atoms; R' is aryl, alkyl or alkoxy;
   each Y is independently H, alkyl or -Z'-(OM')_{m'};
   each Z and Z' is independently a linker comprising 1 to 6, in particular 2 to 5, more particularly 2 to 4, carbon atoms;
   each M and M' is independently H or (meth)acryloyl;
   each m and m' is independently 1, 2 or 3, in particular 1;
   each n is independently 0 or 1, in particular 1;
   each p is independently 1, 2 or 3; in particular 1 or 2;
   q is 1, 2 or 3, in particular 1 or 2;
   with the proviso that the following conditions are met:
      - the compound of formula (I) comprises a -P(=O)(R')- bond or at least two-N(Y)-Z-(OM)ₘ moieties where Y is other than H;
      - when L is a direct bond, it is directly connected to a -C(=O)-X- moiety;
      - the -L-[C(=O)-X]ₙ- moieties and the -Z- moieties are devoid of ester bonds.
Aspect 2. The compound according to Aspect 1, wherein PI is a photoinitiator moiety comprising an optionally substituted aryl selected from benzophenone, thioxanthone, anthraquinone, xanthone, acridone and phenyl;
   with the proviso that when PI is an optionally substituted phenyl, then at least one -C(=O)-group is directly bonded to said phenyl;
   in particular PI corresponds to one of the following formulae (II) to (IX) 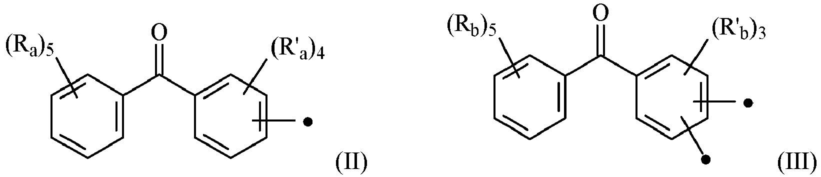 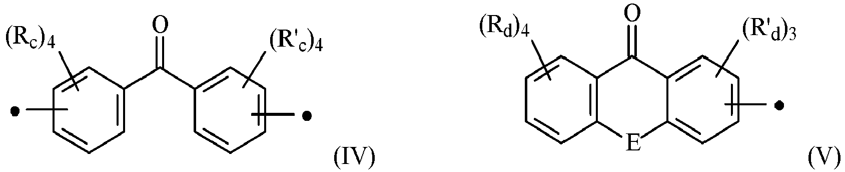 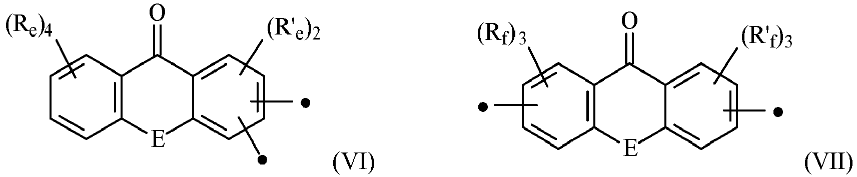 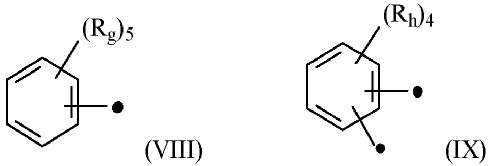 wherein
   E is S, O, C(=O) or NRi, in particular S;
   Ri is H, an optionally substituted alkyl, an optionally substituted aryl;
   each symbol ● represents a point of attachment to a linker L;
   each Rₐ, R'ₐ, R_{b}, R'_{b}, R_{c}, R'_{c}, R_{d}, R'_{d}, Rₑ, R'ₑ, R_{f}, R'_{f}, R_{g} and Rₕ is independently selected from H, F, Cl, Br, I, -OR₂, -SR₂, -N(R₂)₂, -NO₂, -CN, -C(=O)R₂, -O-C(=O)R₂, -C(=O)OR₂, - C(=O)N(R₂)₂, -NR₂-C(=O)-R₂, -SO₂-N(R₂)₂, -C(=O)-P(=O)R₃R₄, or an optionally substituted group selected from alkyl, heteroatom-containing alkyl, cycloalkyl, heterocycloalkyl, alkenyl, alkynyl, aryl, aralkyl, alkaryl and heteroaryl;
   two adjacent Rₐ, R'ₐ, R_{b}, R'_{b}, R_{c}, R'_{c}, R_{d}, R'_{d}, Rₑ, R'ₑ, R_{f}, R'_{f}, R_{g} or Rₕ groups may form, with the carbon atoms to which they are attached, a 5 to 8 membered ring;
   each R₂ is independently H or an optionally substituted group selected from alkyl, cycloalkyl, heterocycloalkyl, alkenyl, alkynyl, aryl, aralkyl, alkaryl, heteroaryl and heteroatom-containing alkyl;
   R₃ and R₄ are independently an optionally substituted group selected from aryl, alkyl, alkoxy or amino;
   with the proviso that when PI corresponds to formula (VIII) or (IX), at least one -C(=O)-group is directly bonded to the phenyl moiety of formula (VIII) or (IX) at a symbol ● and/or at a substituent R_{g} or R_{h.}
Aspect 3. Compound according to Aspect 2, wherein PI corresponds to formula (VIII) or (IX) and the compound comprises a moiety having the following formula (X) or (XI) directly bonded to the phenyl group of PI: 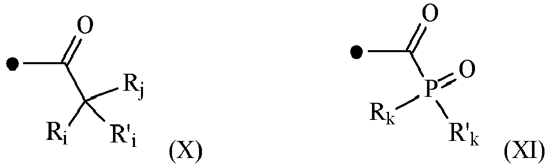 wherein
   Rᵢ and R'ᵢ are alkyl or Rᵢ and R'ᵢ may, together with the carbon atom to which they are attached, form a 5 to 8 membered ring
   Rⱼ is -OH or -NRₗR'ₗ;
   Rₖ and R'ₖ are independently selected from aryl, alkyl, alkoxy and -N(Y)-Z-(OM)ₘ;
   Ri and R'i are independently selected from alkyl and -Z-(OM)ₘ or R₁ and R'₁ may, together with the nitrogen atom to which they are attached, form a 5 to 8 membered ring;
   the symbol ● represents a point of attachment to the PI moiety.
Aspect 4. The compound according to any one of Aspects 1 to 3, wherein the compound is according to one of formulae (XII) to (XXV) 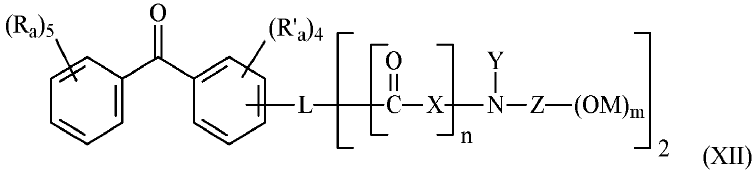 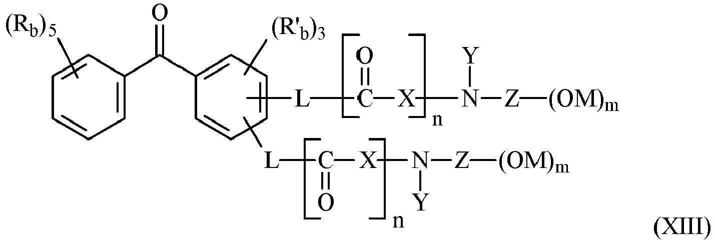 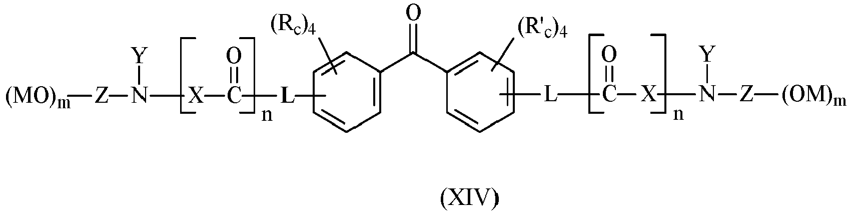 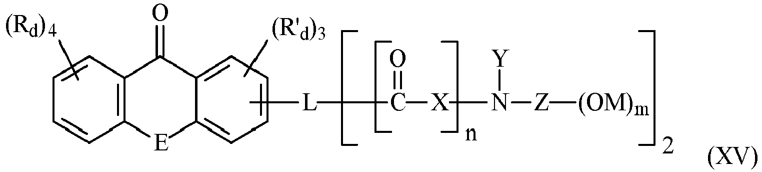 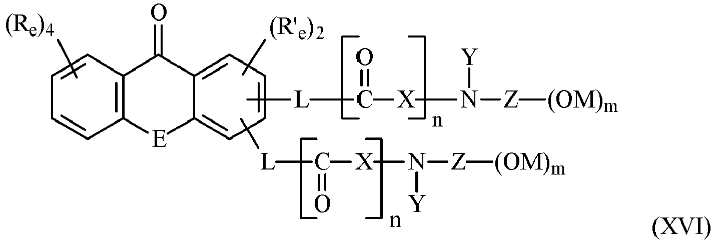 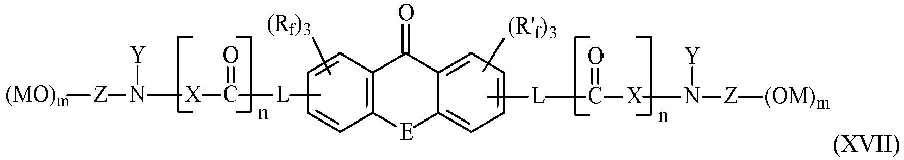 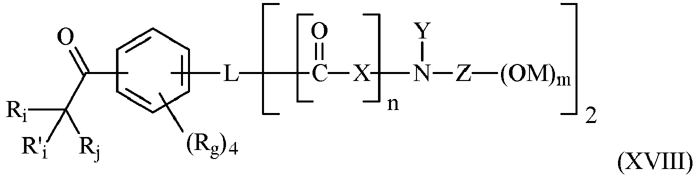 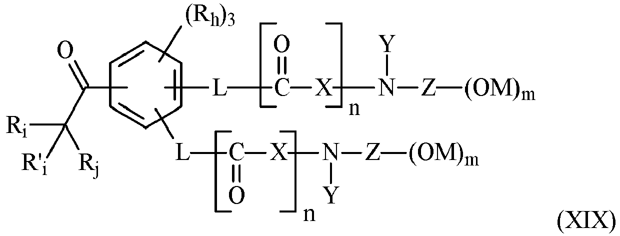 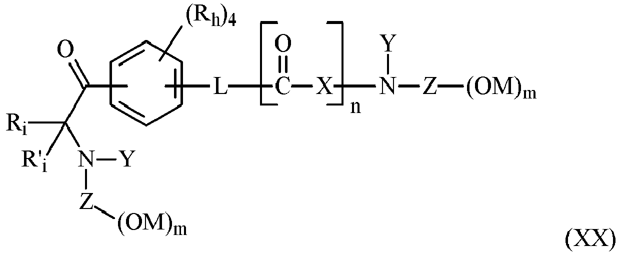 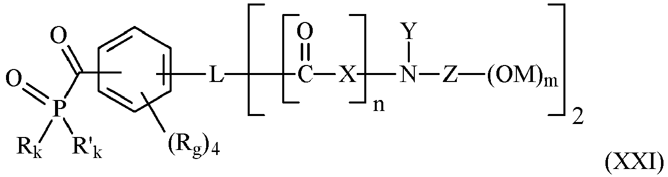 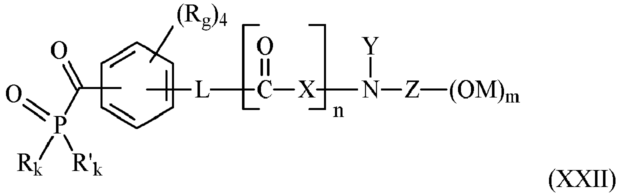 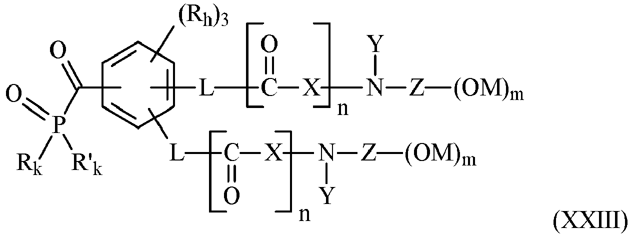 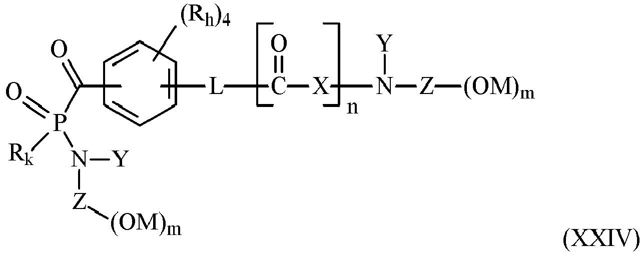 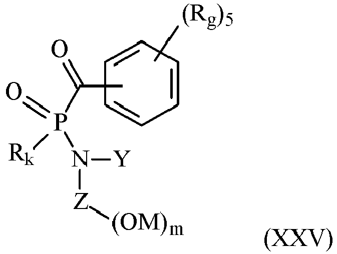 wherein
   L, M, X, Y, Z, m and n are as defined in Aspect 1;
   Rₐ, R'ₐ, R_{b}, R'_{b}, R_{c}, R'_{c}, R_{d}, R'_{d}, Rₑ, R'ₑ, R_{f}, R'_{f}, R_{g} and Rₕ are as defined in Aspect 2;
   Rᵢ and R'ᵢ are as defined in Aspect 3;
   Rⱼ is -OH or -NRₗR'ₗ;
   Rₖ and R'ₖ are independently selected from aryl, alkyl and alkoxy;
   Rₗ and R'ₗ are independently selected from alkyl or Rₗ and R'ₗ may, together with the nitrogen atom to which they are attached, form a 5 to 8 membered ring.
Aspect 5. The compound according to any one of Aspects 1 to 4, wherein each L is independently selected from direct bond, C1-C6 alkylene, C1-C6 oxyalkylene, C1-C6 alkenylene, C1-C6 thioalkylene, C1-C6 ketoalkylene, C1-C6 ketoalkenylene, C7-C13 ketoarylene, C6-C13 ketocycloalkylene;
   more particularly each L is independently selected from a direct bond, an alkylene of formula (XXVI) or (XXVII), an oxyalkylene of formula (XXVIII), a thioalkylene of formula (XXIX), a ketoalkylene of formula (XXX), a ketoalkenylene of formula (XXXI), a ketoarylene of formula (XXXII), a ketocycloalkylene of formula (XXXIII) 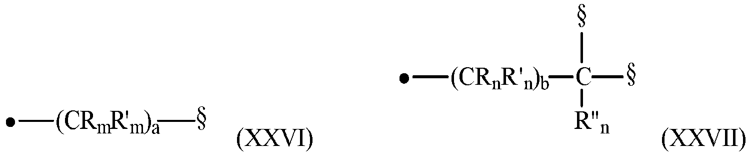 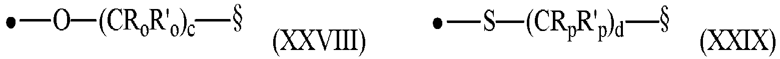 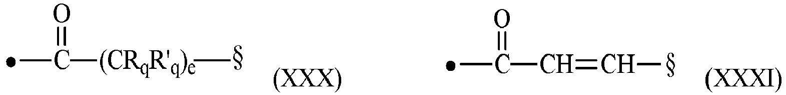 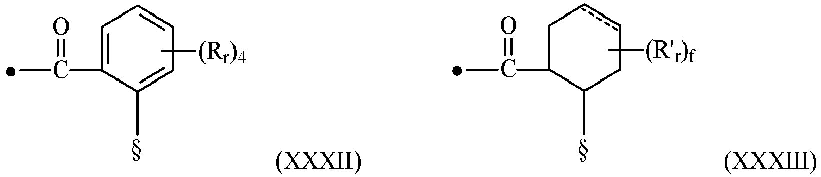 wherein
   each Rₘ, R'ₘ, Rₙ, R'ₙ, R"ₙ, Rₒ, R'ₒ, Rₚ, R'ₚ, R_{q} and R'_{q} is independently H or alkyl, in particular H;
   each Rr and R'r is independently H, halogen, alkyl, cycloalkyl, heterocycloalkyl, alkoxy, aryloxy, thioalkyl, thioaryl, alcenyl, alcynyl, aryl, aralkyl, alkaryl, heteroaryl, -C(=O)R₂, - NR₃R₄, alkylamino, alkylthiol, hydroxyalkyl, haloalkyl, -NO₂, -CN, -C(=O)OR₃, - C(=O)NR₃R₄, -OH and -SH;
   two adjacent Rᵣ groups or two adjacent R'ᵣ groups may form, with the carbon atoms to which they are attached, a 5 to 8 membered ring;
   a is 1, 2, 3, 4, 5 or 6, in particular 1 or 2;
   b is 1, 2, 3, 4 or 5, in particular 1;
   c is 1, 2, 3, 4, 5 or 6, in particular 1 or 2;
   d is 1, 2, 3, 4, 5 or 6, in particular 1 or 2;
   e is 1, 2, 3, 4 or 5, in particular 1;
   f is 6 or 8;
   the partially hashed bond 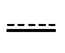 represents either a single C-C bond or a double C=C bond; the symbol ● represents a point of attachment to the PI moiety;
   the symbol § represents a point of attachment to a -C(=O)-X-N(Y)-Z-(OM)ₘ moiety.
Aspect 6. The compound according to any one of Aspects 1 to 5, wherein each X is independently selected from direct bond, alkylene and -P(=O)(R')-;
   in particular each X is independently selected from a direct bond, an alkylene of formula - (CRₛR'ₛ)_{g}- and -P(=O)(R')- where R' is phenyl;
   wherein each Rₛ and R'ₛ is independently H or alkyl, in particular alkyl;
      g is 1, 2, 3, 4, 5 or 6, in particular 1.
Aspect 7. The compound according to any one of Aspects 1 to 6, wherein each Y is independently alkyl or -Z'-(OM')_{m'}, in particular methyl, ethyl or -Z'-(OM')_{m'}, more particularly -Z'-(OM')_{m'}.
Aspect 8. The compound according to any one of Aspects 1 to 7, wherein all of the Y groups are -Z'-(OM')_{m'}.
Aspect 9. The compound according to any one of Aspects 1 to 7, wherein all of the Y groups are alkyl.
Aspect 10. The compound according to any one of Aspects 1 to 7, wherein part of the Y groups are -Z'-(OM')_{m'} and part of the Y groups are alkyl.
Aspect 11. The compound according to any one of Aspects 1 to 10, wherein each Z and Z' is independently an alkylene; in particular each Z and Z' is independently an alkylene of formula (XXXIV), (XXXV) or (XXXVI), even more particularly an alkylene of formula (XXXIV) 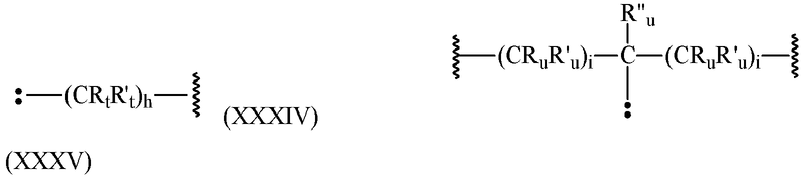 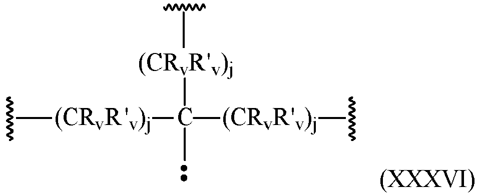
   wherein each Rₜ, R'ₜ, Rᵤ, R'ᵤ, Rᵥ, R'ᵥ is independently H or alkyl, in particular H; R"ᵤ is H or alkyl, in particular alkyl;
   h is 1, 2, 3, 4, 5 or 6, in particular 2;
   each i is independently 1, 2 or 3, in particular 1;
   each j is independently 1, 2 or 3, in particular 1;
   the symbol 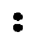 represents a point of attachment to the nitrogen atom;
   each symbol 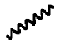 represents a point of attachment to an -OM or -OM' group.
Aspect 12. The compound according to any one of Aspects 1 to 11, wherein the compound has a total number of -OM and -OM' groups of at least 2, in particular at least 3, more particularly at least 4.
Aspect 13. The compound according to any one of Aspects 1 to 12, wherein all of the M and M' groups are H.
Aspect 14. The compound according to any one of Aspects 1 to 12, wherein all of the M and M' groups are (meth)acryloyl.
Aspect 15. The compound according to any one of Aspects 1 to 12, wherein part of the M and M' groups are H and part of the M and M' groups are (meth)acryloyl.
Aspect 16. A photoinitiator composition comprising at least one compound of formula (I) according to any one of Aspects 1 to 15 and at least one compound according to one of the following formula (XXXVII) or (XXXVIII) 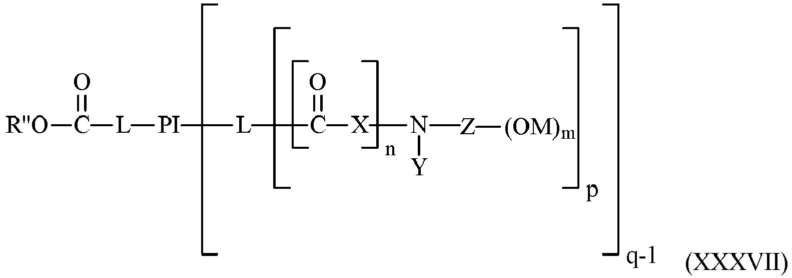 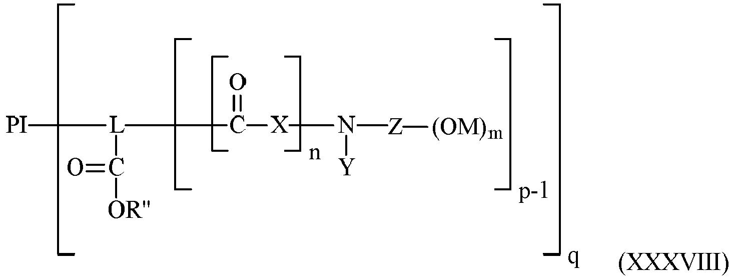 wherein PI, L, M, X, Y, Z, m, n, p and q are as defined in Aspects 1 to 15; and R" is H or alkyl.
Aspect 17. A photoinitiator composition comprising a mixture of at least two, in particular at least three, distinct compounds of formula (I) according to any one of Aspects 1 to 15. Aspect 18. The photoinitiator composition according to Aspect 17, wherein the distinct compounds are selected from a compound where all of the Y groups are -Z-(OM)ₘ, a compound where all of the Y groups are alkyl, and a compound where part of the Y groups are -Z-(OM)ₘ and part of the Y groups are alkyl, and combinations thereof; in particular the mixture comprises a compound where all of the Y groups are -Z-(OM)ₘ, a compound where all of the Y groups are alkyl, and a compound where part of the Y groups are -Z-(OM)ₘ and part of the Y groups are alkyl.
Aspect 19. A photoinitiator composition comprising a compound of formula (I) according to any one of Aspects 1 to 15 and an amine synergist other than a compound of formula (I), preferably an amine synergist bearing a tertiary amine group.
Aspect 20. A process for preparing a compound of formula (I) according to any one of Aspects 1 to 15, wherein at least part of the M groups are (meth)acryloyl, the process comprising reacting a compound of formula (I) wherein all of the M groups are H with (meth)acrylic acid or a (C1-C6)alkyl (meth)acrylate in the presence of a catalyst.
Aspect 21. The process according to Aspect 20, wherein a (C1-C6)alkyl (meth)acrylate is used and the catalyst is a transesterification catalyst.
Aspect 22. A process for photopolymerising one or more ethylenically unsaturated compounds, the process comprising contacting one or more ethylenically unsaturated compounds with a compound of formula (I) according to any one of Aspects 1 to 15 or a photoinitiator composition according to any one of Aspects 16 to 19, and irradiating the mixture, in particular with visible, near-UV and/or UV light.
Aspect 23. A curable composition comprising:
   a) a compound of formula (I) according to any one of Aspects 1 to 15 or a photoinitiator composition according to any one of Aspects 16 to 19; and
   b) an ethylenically unsaturated compound.
Aspect 24. A curable composition comprising:
   a) a compound of formula (I) according to any one of Aspects 1 to 15 or a photoinitiator composition according to any one of Aspects 16 to 19; and
   c) an unsaturated polymer dispersion or emulsion.
Aspect 25. The curable composition according to Aspect 24, wherein the unsaturated polymer dispersion or emulsion is a polyurethane dispersion, an acrylic dispersion, an unsaturated polyester emulsion or an emulsion of a (meth)acrylate-functionalized oligomer and mixtures thereof, in particular the unsaturated polymer dispersion or emulsion is a polyurethane dispersion.
Aspect 26. The curable composition according to any one of Aspect 23 to 25, wherein the composition is water-based.
Aspect 27. The curable composition according to any one of Aspects 23 to 26, wherein the composition is an ink composition, an overprint varnish composition, a coating composition, an adhesive composition, a sealant composition, a molding composition, a dental composition, a cosmetic composition or a 3D-printing composition.
Aspect 28. A process for the preparation of a cured product, the process comprising curing the curable composition according to any one of Aspects 23 to 27, in particular by exposing the curable composition to radiation such as UV, near-UV and/or visible radiation.
Aspect 29. A process of inkjet printing comprising jetting the curable composition according to any one of Aspects 23 to 27 onto a substrate.
Aspect 30. A substrate on which the curable composition according to any one of Aspects 23 to 27 has been applied and cured, in particular the substrate is a food and beverage packaging, a pharmaceutical packaging, a textile, a nail, a tooth, a medical device, a food and beverage processing equipment, a water pipe.
Aspect 31. A use of a compound of formula (I) according to any one of Aspects 1 to 15, or of the photoinitiator composition of any one of Aspects 16 to 19, in a photopolymerization reaction.
Aspect 32. A use of a compound of formula (I) according to any one of Aspects 1 to 15 or a photoinitiator composition according to any one of Aspects 16 to 19, as a photoinitiator or a photoinitiating system in a radiation curable composition, in particular in a UV or LED-curable composition.
Aspect 33. A use of the compound of formula (I) according to any one of Aspects 1 to 15 wherein at least part of the M groups are (meth)acryloyl, to reduce the amount of extractibles from a cured layer of a curable composition.

Within this specification, embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein.

In some embodiments, the invention herein can be construed as excluding any element or process step that does not materially affect the basic and novel characteristics of the invention. Additionally, in some embodiments, the invention can be construed as excluding any element or process step not specified herein.

Although the invention is illustrated and described herein with reference to specific embodiments, the invention is not intended to be limited to the details shown. Rather, various modifications may be made in the details within the scope and range of equivalents of the claims and without departing from the invention.

### Examples

### Materials

All materials used in the examples are readily available from standard commercial sources such as Sigma-Aldrich Company Ltd. and Tokyo Chemical Industry Ltd. unless otherwise specified. 2-(Chlorothio)benzoyl chloride was prepared according to the procedure given in US 4,101,558 (The Sherwin-Williams Company). Ucecoat 2805 is a UV-curable polyurethane dispersion in water available from Allnex. SpeedCure 2959 is a hydroxyacetophenone photoinitiator available from Arkema.

**Synthesis of Intermediate 1a:** diethyl 2,2'-[(4-benzoyl-1,3-phenylene)bis(oxy)]diacetate

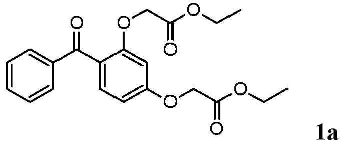

To a stirred solution of 2,4-dihydroxybenzophenone (85.0 g, 0.397 mol) in 2-butanone (500 mL) was added potassium iodide (6.6 g, 0.0397 mol), potassium carbonate (345.5 g, 2.5 mol) and ethyl chloroacetate (102 g, 0.833 mol). The reaction mixture was heated to reflux for 5 hr before cooling to room temperature and pouring onto water. The organic phase was separated and washed with equal volumes of water until a clear, colourless aqueous phase was obtained. The organic phase was separated, dried (MgSO4) and concentrated under reduced pressure to give **Intermediate 1a** as a pale yellow oil, which solidified with time. (145 g, 95 %). ¹H NMR (400 MHz, CDCl₃); 7.83 (dd, *J* = 8.5 and 1.5 Hz, 2H), 7.53 (m, 1H), 7.43 (m, 3H), 6.55 (dd, *J* = 8.5 and 2.0 Hz, 1H), 6.48 (d, *J* = 2.0 Hz, 1H), 4.66 (s, 2H), 4.40 (s, 2H), 4.30 (q, *J* = 7.0 Hz, 2H), 4.17 (q, *J* = 7.0 Hz, 2H), 1.32 (t, *J* = 7.0 Hz, 3H), 1.23 (t, *J* = 7.0 Hz, 3H). vₘₐₓ/cm⁻¹ 2984, 2938, 1746, 1651, 1607, 1581.

### Synthesis of benzophenone polyol 1 according to the invention

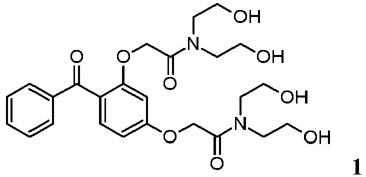

A mixture of **Intermediate 1a** (467 g, 1.21 mol) and diethanolamine (256 g, 2.44 mol) were heated, with stirring, to 100-105 °C. Ethanol was removed by distillation at atmospheric pressure. The mixture was heated for 15 h before allowing to cool to ∼80 °C. The product was concentrated under reduced pressure to remove remaining ethanol. The product was allowed to cool to room temperature. The desired **polyol 1** was obtained as a pale yellow solid (610 g, 100%). ¹H NMR (400 MHz, D₂O); 7.42 (d*, J* = 8.5 Hz, 2H), 7.36 (t, *J* = 7.5 Hz, 1H), 7.20 (t, *J* = 7.5 Hz, 2H), 7.08 (d, *J* = 8.5 Hz, 1H), 6.40 (m 2H), 4.78 (s, 2H), 4.59 (s, 2H), 3.67-3.55 (m, 4H), 3.50 (t, *J* = 5.5 Hz, 2H), 3.45 (t, *J* = 6.0 Hz, 2H), 3.39-3.33 (m, 4H), 3.26 (t, *J=* 6.0 Hz, 2H), 3.20 (t, *J* = 5.5 Hz, 2H). vₘₐₓ/cm⁻¹ 3352 (br), 2935, 1640, 1601, 1578.

### Synthesis of Intermediate 2a: diethyl [(9-oxo-9H-thioxanthenyl)methyl]propanedioate (as a mixture of isomers)

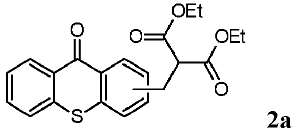

A solution of diethyl benzylmalonate (220 g, 880 mmol) and 2-(chlorothio)benzoyl chloride (200 g, 966 mol) in dichloromethane (1 L) was added over 3 h to a stirred, ice-cooled suspension of anhydrous iron(III) chloride (428 g, 2.64 mol) in dichloromethane (1.5 L) and the resulting deep red solution was allowed to warm to room temperature and stirred for a further 16 h. The mixture was poured into ice water (∼2 L) and the biphasic mixture was stirred vigorously for 0.5 h. The mixture was filtered and the layers were separated. The organic layer was collected and concentrated under reduced pressure (at 30-35 °C). To the crude residue was added toluene (1.5 h), and the resulting solution was washed with 5% hydrochloric acid (2 × 500 mL), water (500 mL), saturated sodium bicarbonate solution (2 × 500 mL) and brine (300 mL). The organic phase was dried (MgSO₄) and concentrated under reduced pressure to give the desired thioxanthone **Intermediate 2a** as a yellow/orange liquid (305 g, 90%, a mixture of isomers; the 2-isomer is the major isomer). ¹H NMR (400 MHz, CDCl₃); 8.63-8.45 (m, 2H), 7.65-7.40 (m, 5H), 4.24-4.12 (m, 4H), 3.75 (t, *J=* 8.0 Hz, 1H), 3.36-3.33 (m, 2H), 1.27-1.18 (m, 6H). vₘₐₓ/cm⁻¹ 2981, 1727, 1636, 1591.

### Synthesis of thioxanthone polvol 2 according to the invention

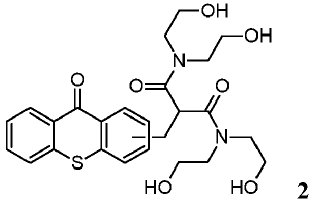

A mixture of **Intermediate 2a** (50.0 g, 130 mmol) and diethanolamine (26.0 g, 247 mmol) was heated at 110-115 °C for 5 h and ethanol was removed by distillation. The resulting mixture was dried thoroughly under reduced pressure to give the crude product (59.0 g). 7 g of this crude material was purified by column chromatography (eluting with 5:1 dichloromethane-methanol) to give the pure **polyol 2** as a pale yellow solid (2.7 g, 39%, mixture of isomers; the 2-isomer is the major isomer). ¹H NMR (400 MHz, *d*₆-DMSO); 8.49 (dd, *J* = 1.0 and 8.0 Hz, 1H), 8.39 (s, 1H), 7.85-7.60 (m, 5H), 4.92 (t, *J* = 5.0 Hz, 2H), 4.68 (t, *J* = 5.0 Hz, 2H), 4.47 (t, *J* = 6.5 Hz, 1H), 3.35-3.41 (m, 18H). vₘₐₓ/cm⁻¹ 3365 (br), 2937, 1618, 1588.

### UV-Curing experiments:

Formulations containing 2 g of the water-borne resin Ucecoat 2805 (Allnex), 0.04 g of a photoinitiator (2 phr = 2 parts by weight per 100 parts by weight of resin), and 0.10 g triethanolamine (as amine synergist, 5 phr) were prepared by mixing the materials together and stirring at room temperature for -30 mins. The PIs and amine co-initiators were fully dissolved in the resin within this time. The formulations were then cured under a Hg lamp (30% intensity) in 24-µm films using a belt speed of 20 m/min. The number of passes under the Hg lamp required to achieve full-cure (as determined by the thumb-twist test, in which pressure is applied with a thumb in a twisting motion until no marks are left on the cured film surface), is given below.

| | Photoinititiator | Co-initiator (phr) | No. of passes to full cure |
|---|---|---|---|
| Formulation 1 | benzophenone **polyol 1** (2 phr) | triethanolamine (5 phr) | 2 |
| Formulation 2 | thioxanthone **polyol 2** (2 phr) | triethanolamine (5 phr) | 2 |
| Formulation 3 | SpeedCure 2959 (2 phr) | triethanolamine (5 phr) | 2 |
| Formulation 4 | | triethanolamine (5 phr) | 10 |

The cure speed of the two polyols according to the invention in a water-borne resin system was comparable to the state-of-the-art SpeedCure 2959; cure speed was significantly enhanced compared to the formulation with no photoinitiator present.

## Claims

1. A compound according to the following formula (I): 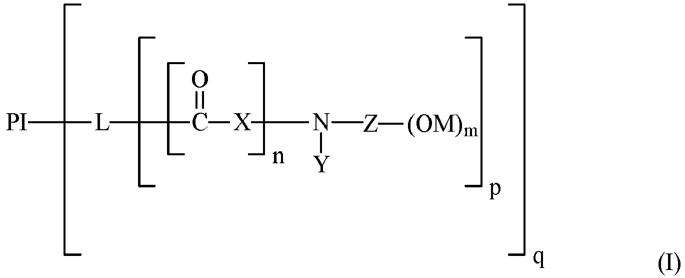 wherein
PI is a photoinitiator moiety;
each L is independently selected from a direct bond or a linker comprising 1 to 20 carbon atoms;
each X is independently selected from a direct bond, a -P(=O)(R')- bond and a linker comprising 1 to 6, in particular 1 to 4, more particularly 1 to 3, carbon atoms; R' is aryl, alkyl or alkoxy;
each Y is independently H, alkyl or -Z'-(OM')_{m'};
each Z and Z' is independently a linker comprising 1 to 6, in particular 2 to 5, more particularly 2 to 4, carbon atoms;
each M and M' is independently H or (meth)acryloyl;
each m and m' is independently 1, 2 or 3, in particular 1;
each n is independently 0 or 1, in particular 1;
each p is independently 1, 2 or 3; in particular 1 or 2;
q is 1, 2 or 3, in particular 1 or 2;
with the proviso that the following conditions are met:
• the compound of formula (I) comprises a -P(=O)(R')- bond or at least two -N(Y)-Z-(OM)ₘ moieties where Y is other than H;
• when L is a direct bond, it is directly connected to a -C(=O)-X- moiety;
• the -L-[C(=O)-X]ₙ- moieties and the -Z- moieties are devoid of ester bonds.

2. The compound according to claim 1, wherein PI is a photoinitiator moiety comprising an optionally substituted aryl selected from benzophenone, thioxanthone, anthraquinone, xanthone, acridone and phenyl;
with the proviso that when PI is an optionally substituted phenyl, then at least one -C(=O)-group is directly bonded to said phenyl;
in particular PI corresponds to one of the following formulae (II) to (IX) 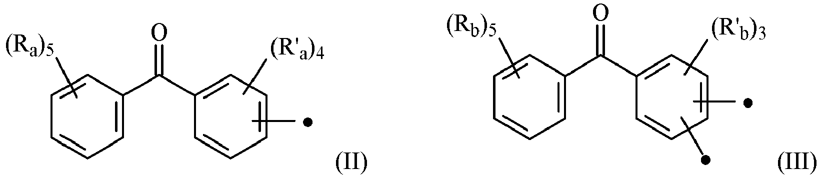 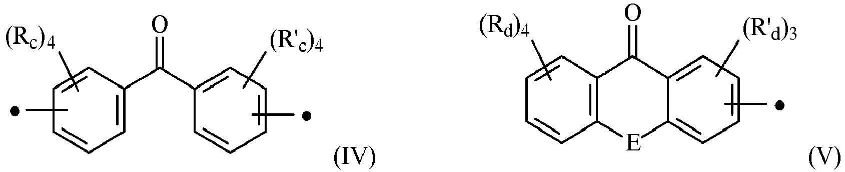 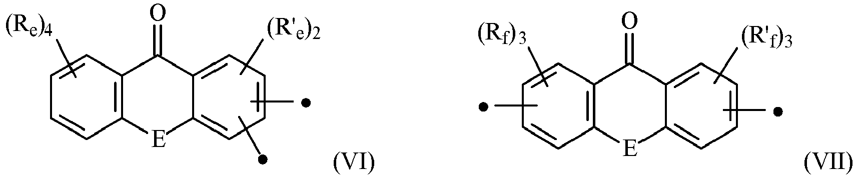 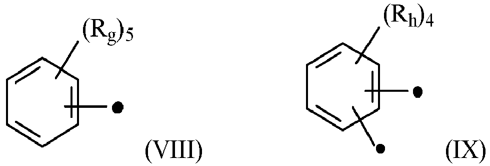 wherein
E is S, O, C(=O) or NR₁, in particular S;
Ri is H, an optionally substituted alkyl, an optionally substituted aryl;
each symbol ● represents a point of attachment to a linker L;
each Rₐ, R'ₐ, R_{b}, R'_{b}, R_{c}, R'_{c}, R_{d}, R'_{d}, Re, R'ₑ, R_{f}, R'_{f}, R_{g} and Rₕ is independently selected from H, F, Cl, Br, I, -OR₂, -SR₂, -N(R₂)₂, -NO₂, -CN, -C(=O)R₂, -O-C(=O)R₂, -C(=O)OR₂, - C(=O)N(R₂)₂, -NR₂-C(=O)-R₂, -SO₂-N(R₂)₂, -C(=O)-P(=O)R₃R₄, or an optionally substituted group selected from alkyl, heteroatom-containing alkyl, cycloalkyl, heterocycloalkyl, alkenyl, alkynyl, aryl, aralkyl, alkaryl and heteroaryl;
two adjacent Rₐ, R'ₐ, R_{b}, R'_{b}, R_{c}, R'_{c}, R_{d}, R'_{d}, Rₑ, R'ₑ, R_{f}, R'_{f}, R_{g} or Rₕ groups may form, with the carbon atoms to which they are attached, a 5 to 8 membered ring;
each R₂ is independently H or an optionally substituted group selected from alkyl, cycloalkyl, heterocycloalkyl, alkenyl, alkynyl, aryl, aralkyl, alkaryl, heteroaryl and heteroatom-containing alkyl;
R₃ and R₄ are independently an optionally substituted group selected from aryl, alkyl, alkoxy or amino;
with the proviso that when PI corresponds to formula (VIII) or (IX), at least one -C(=O)-group is directly bonded to the phenyl moiety of formula (VIII) or (IX) at a symbol ● and/or at a substituent R_{g} or Rₕ.

3. Compound according to claim 2, wherein PI corresponds to formula (VIII) or (IX) and the compound comprises a moiety having the following formula (X) or (XI) directly bonded to the phenyl group of PI: 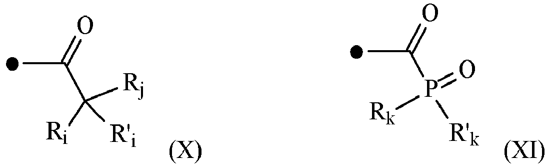 wherein
Rᵢ and R'ᵢ are alkyl or Rᵢ and R'ᵢ may, together with the carbon atom to which they are attached, form a 5 to 8 membered ring
Rⱼ is -OH or -NRₗR'ₗ;
Rₖ and R'ₖ are independently selected from aryl, alkyl, alkoxy and -N(Y)-Z-(OM)ₘ;
Rₗ and R'ₗ are independently selected from alkyl and -Z-(OM)ₘ or Rₗ and R'ₗ may, together with the nitrogen atom to which they are attached, form a 5 to 8 membered ring;
the symbol ● represents a point of attachment to the PI moiety.

4. The compound according to any one of claims 1 to 3, wherein the compound is according to one of formulae (XII) to (XXV) 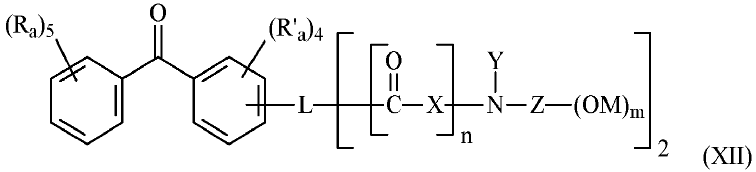 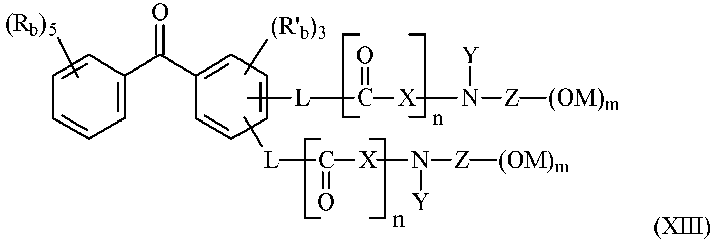 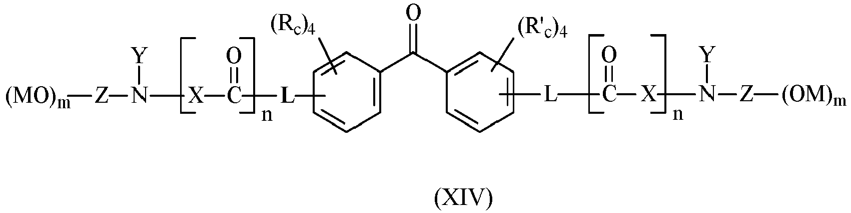 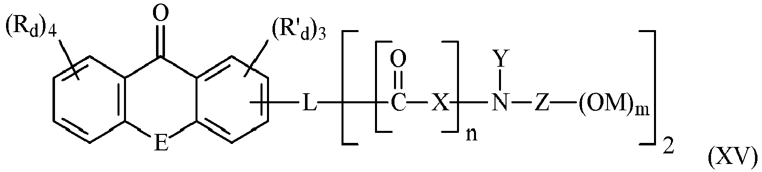 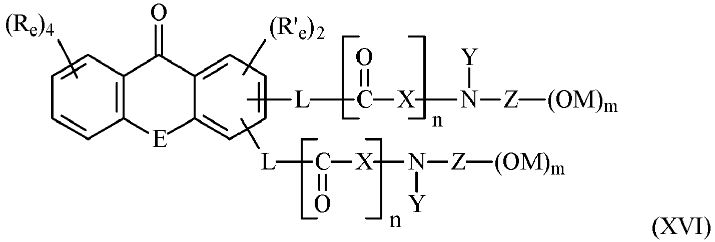 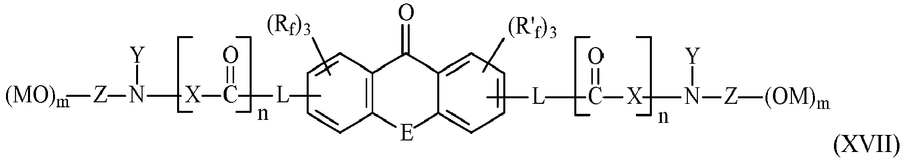 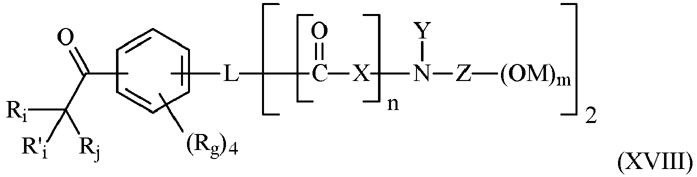 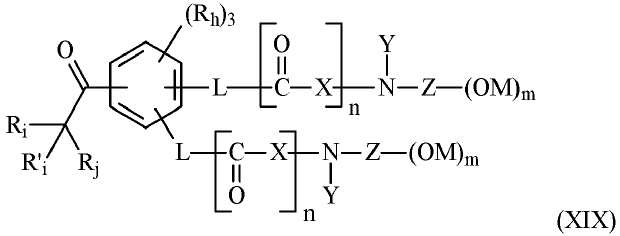 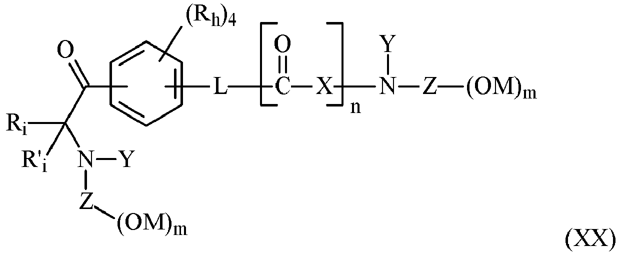 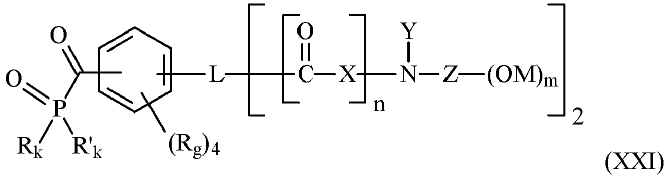 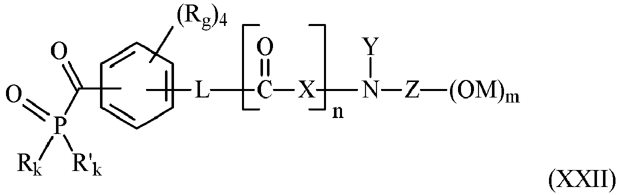 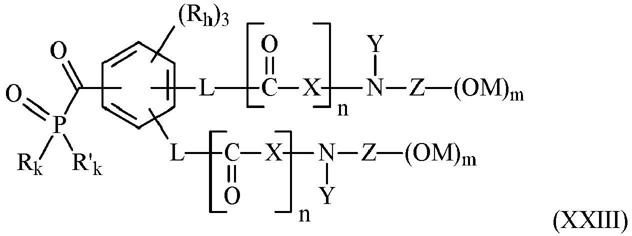 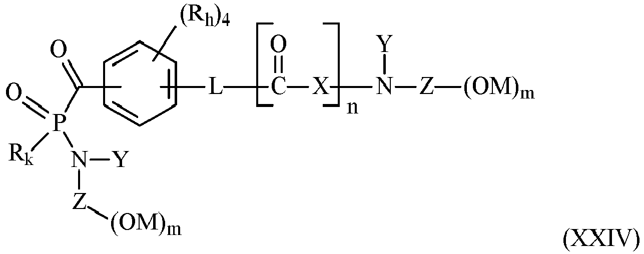 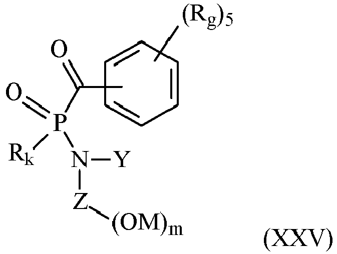 wherein
L, M, X, Y, Z, m and n are as defined in claim 1;
Rₐ, R'ₐ, R_{b}, R'_{b}, R_{c}, R'_{c}, R_{d}, R'_{d}, Rₑ, R'ₑ, R_{f}, R'_{f}, R_{g} and Rₕ are as defined in claim 2; Rᵢ and R'ᵢ are as defined in claim 3;
Rⱼ is -OH or -NRₗR'ₗ;
Rₖ and R'ₖ are independently selected from aryl, alkyl and alkoxy;
Rₗ and R'ₗ are independently selected from alkyl or Rₗ and R'ₗ may, together with the nitrogen atom to which they are attached, form a 5 to 8 membered ring.

5. The compound according to any one of claims 1 to 4, wherein each L is independently selected from direct bond, C1-C6 alkylene, C1-C6 oxyalkylene, C1-C6 alkenylene, C1-C6 thioalkylene, C1-C6 ketoalkylene, C1-C6 ketoalkenylene, C7-C13 ketoarylene, C6-C13 ketocycloalkylene;
more particularly each L is independently selected from a direct bond, an alkylene of formula (XXVI) or (XXVII), an oxyalkylene of formula (XXVIII), a thioalkylene of formula (XXIX), a ketoalkylene of formula (XXX), a ketoalkenylene of formula (XXXI), a ketoarylene of formula (XXXII), a ketocycloalkylene of formula (XXXIII) 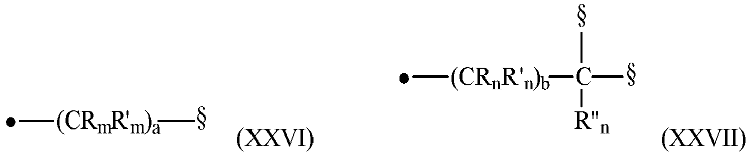 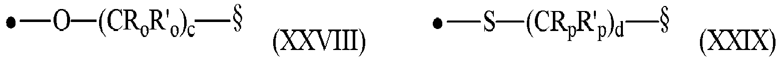 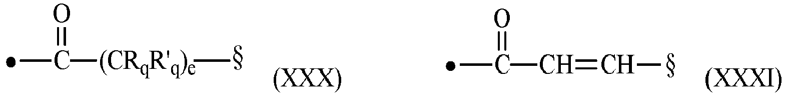 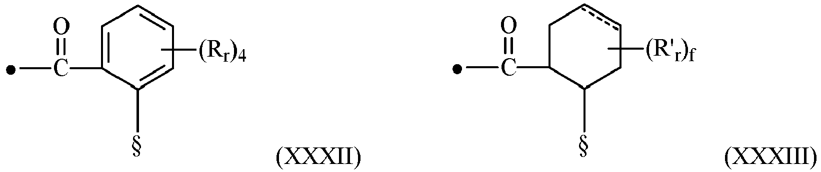 wherein
each Rₘ, R'ₘ, Rₙ, R'ₙ, R"ₙ, Rₒ, R'ₒ, Rₚ, R'ₚ, R_{q} and R'_{q} is independently H or alkyl, in particular H;
each Rᵣ and R'ᵣ is independently H, halogen, alkyl, cycloalkyl, heterocycloalkyl, alkoxy, aryloxy, thioalkyl, thioaryl, alcenyl, alcynyl, aryl, aralkyl, alkaryl, heteroaryl, -C(=O)R₂, - NR₃R₄, alkylamino, alkylthiol, hydroxyalkyl, haloalkyl, -NO₂, -CN, -C(=O)OR₃, - C(=O)NR₃R₄, -OH and -SH;
two adjacent Rᵣ groups or two adjacent R'ᵣ groups may form, with the carbon atoms to which they are attached, a 5 to 8 membered ring;
a is 1, 2, 3, 4, 5 or 6, in particular 1 or 2;
b is 1, 2, 3, 4 or 5, in particular 1;
c is 1, 2, 3, 4, 5 or 6, in particular 1 or 2;
d is 1, 2, 3, 4, 5 or 6, in particular 1 or 2;
e is 1, 2, 3, 4 or 5, in particular 1;
f is 6 or 8; the partially hashed bond 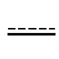 represents either a single C-C bond or a double C=C bond; the symbol 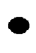 represents a point of attachment to the PI moiety; the symbol § represents a point of attachment to a -C(=O)-X-N(Y)-Z-(OM)ₘ moiety.

6. The compound according to any one of claims 1 to 5, wherein each X is independently selected from direct bond, alkylene and -P(=O)(R')-;
in particular each X is independently selected from a direct bond, an alkylene of formula-(CRₛR'ₛ)_{g}- and -P(=O)(R')- where R' is phenyl;
wherein each Rₛ and R'ₛ is independently H or alkyl, in particular alkyl;
g is 1, 2, 3, 4, 5 or 6, in particular 1.

7. The compound according to any one of claims 1 to 6, wherein each Y is independently alkyl or -Z'-(OM')_{m'}, in particular methyl, ethyl or -Z'-(OM')_{m'}, more particularly -Z'-(OM')_{m'}.

8. The compound according to any one of claims 1 to 7, wherein each Z and Z' is independently an alkylene; in particular each Z and Z' is independently an alkylene of formula (XXXIV), (XXXV) or (XXXVI), even more particularly an alkylene of formula (XXXIV) 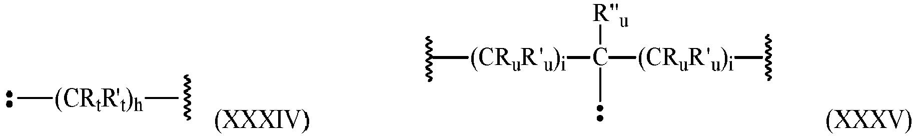 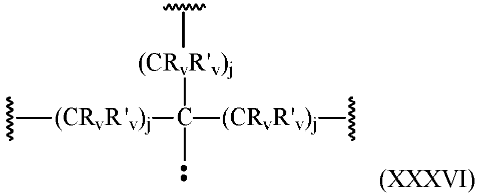
wherein each Rₜ, R'ₜ, Rᵤ, R'ᵤ, Rᵥ, R'ᵥ is independently H or alkyl, in particular H;
R"ᵤ is H or alkyl, in particular alkyl;
h is 1, 2, 3, 4, 5 or 6, in particular 2;
each i is independently 1, 2 or 3, in particular 1;
each j is independently 1, 2 or 3, in particular 1;
the symbol 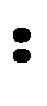 represents a point of attachment to the nitrogen atom;
each symbol 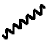 represents a point of attachment to an -OM or -OM' group.

9. The compound according to any one of claims 1 to 8, wherein the compound has a total number of -OM and -OM' groups of at least 2, in particular at least 3, more particularly at least 4.

10. A photoinitiator composition comprising at least one compound of formula (I) according to any one of claims 1 to 9 and at least one compound according to one of the following formula (XXXVII) or (XXXVIII) 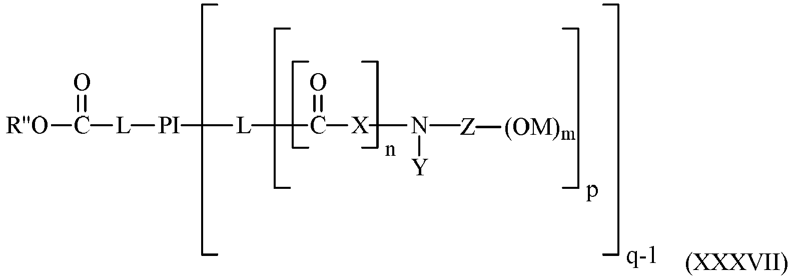 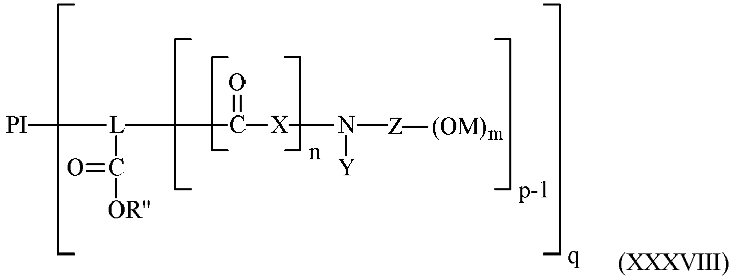 wherein PI, L, M, X, Y, Z, m, n, p and q are as defined in claims 1 to 9; and
R" is H or alkyl.

11. A photoinitiator composition comprising a mixture of at least two, in particular at least three, distinct compounds of formula (I) according to any one of claims 1 to 9.

12. A photoinitiator composition comprising a compound of formula (I) according to any one of claims 1 to 9 and an amine synergist other than a compound of formula (I), preferably an amine synergist bearing a tertiary amine group.

13. A process for preparing a compound of formula (I) according to any one of claims 1 to 9, wherein at least part of the M groups are (meth)acryloyl, the process comprising reacting a compound of formula (I) wherein all of the M groups are H with (meth)acrylic acid or a (C1-C6)alkyl (meth)acrylate in the presence of a catalyst.

14. A process for photopolymerising one or more ethylenically unsaturated compounds, the process comprising contacting one or more ethylenically unsaturated compounds with a compound of formula (I) according to any one of claims 1 to 9 or a photoinitiator composition according to any one of claims 10 to 12, and irradiating the mixture, in particular with visible, near-UV and/or UV light.

15. A curable composition comprising:
a) a compound of formula (I) according to any one of claims 1 to 9 or a photoinitiator composition according to any one of claims 10 to 12; and
b) an ethylenically unsaturated compound.

16. A curable composition comprising:
a) a compound of formula (I) according to any one of claims 1 to 9 or a photoinitiator composition according to any one of claims 10 to 12; and
c) an unsaturated polymer dispersion or emulsion.

17. The curable composition according to claim 16, wherein the unsaturated polymer dispersion or emulsion is a polyurethane dispersion, an acrylic dispersion, an unsaturated polyester emulsion or an emulsion of a (meth)acrylate-functionalized oligomer and mixtures thereof, in particular the unsaturated polymer dispersion or emulsion is a polyurethane dispersion.

18. A process for the preparation of a cured product, the process comprising curing the curable composition according to any one of claims 15 to 17, in particular by exposing the curable composition to radiation such as UV, near-UV and/or visible radiation.

19. A process of inkjet printing comprising jetting the curable composition according to any one of claims 15 to 17 onto a substrate.

20. A substrate on which the curable composition according to any one of claims 15 to 17 has been applied and cured, in particular the substrate is a food and beverage packaging, a pharmaceutical packaging, a textile, a nail, a tooth, a medical device, a food and beverage processing equipment, a water pipe.
